# EUROPEAN PATENT APPLICATION

(11) **EP 4 851 715 A2**
(43) Date of publication of application: **09.12.2026**
(21) Application number: 26205112.1
(22) Date of filing: 18.08.2025
(51) Int. Cl.: G03F 7/09

(54) **COMPOSITION FOR FORMING ORGANIC FILM, METHOD FOR FORMING ORGANIC FILM, PATTERNING PROCESS, AND COMPOUND**

(30) Priority: 20.08.2024 JP 2024139272
(62) Document number(s) of the earlier application(s) in accordance with Art. 76 EPC: 25196431.8 / 4 707 927
(71) Applicant: SHIN-ETSU CHEMICAL CO., LTD., Tokyo 100-0005 (JP)
(72) Inventor: Kori, Daisuke, Niigata (JP); Yamamoto, Yasuyuki, Niigata (JP)
(74) Representative: Schicker, Silvia

(57) **Abstract**

Provided is a composition for forming an organic film that is excellent in terms of film formability (in-plane uniformity) and embedding characteristics on a substrate (wafer), suppresses a hump during an EBR process, and has an excellent process tolerance when used as an organic lower layer film for a multi-layer resist. A composition for forming an organic film containing (A) a resin or compound for forming an organic film, (B) a compound represented by the general formula (1), and (C) a solvent, 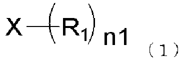 wherein R₁ is a terminal group represented by the general formula (2), X is an n1-valent organic group having 2 to 50 carbon atoms, and n1 represents an integer of 2 to 8, 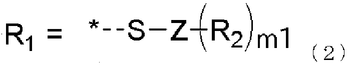 wherein R₂ is a monovalent organic group, wherein the monovalent organic group contains at least any of structures having fluorine atoms represented by the general formula (3), m1 is 1 or 2, wherein when m1 is 1, Z represents a single bond or a divalent organic group optionally containing a heteroatom, and when m1 is 2, Z represents a trivalent organic group optionally containing a heteroatom,

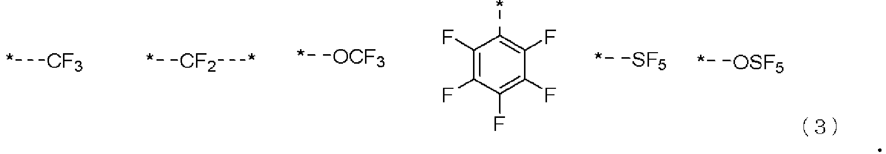

## Description

### TECHNICAL FIELD

The present invention relates to: a composition for forming an organic film, a method for forming an organic film, a patterning process, and a compound.

### BACKGROUND ART

Along with the high integration and speed up of LSI, miniaturization of pattern rules is rapidly progressing. This is because 5G high-speed communication and artificial intelligence (AI) have become widespread and high-performance devices for processing these are in demand. As a cutting-edge miniaturization technique, mass production of devices with 5 nm nodes by 13.5 nm-wavelength extreme ultraviolet (EUV) lithography is being performed. Furthermore, studies have been underway regarding the use of EUV lithography in next-generation 3 nm node or next-next-generation 2 nm node devices as well.

When the thinning of resist patterns progresses as described above, in a single-layer resist method that is used as a typical resist patterning process, the patterning process becomes difficult, and as fine patterning processes, a multi-layer resist method in which films having different dry etching characteristics are laminated together to form a pattern with a high aspect ratio on a stepped substrate and a pattern is formed is known to be excellent, and a three-layer resist method in which a photoresist layer made of an organic photosensitive polymer for use in the single-layer resist method, a middle layer made of a silicon-based polymer or a silicon-based CVD film, and a lower layer made of an organic polymer are combined together (Patent Documents 1 and 2) has been developed and put into practical use.

In this three-layer resist method, an organic film made of novolac or the like is uniformly formed as a resist lower layer film on a substrate to be processed, a silicon-containing film is formed as a resist middle layer film thereon, and a normal organic photoresist film is formed as a resist upper layer film thereon. The organic resist upper layer film has a more favorable etching selectivity than the silicon-containing resist middle layer film with respect to dry etching with a fluorine-based gas plasma, and a resist pattern is thus transferred to the silicon-containing resist middle layer film using dry etching with a fluorine-based gas plasma. According to this method, it is possible to transfer a pattern to the silicon-containing film using even a resist composition with which formation of a pattern having a film thickness large enough to directly process a substrate to be processed is difficult or a resist composition having dry etching resistance not strong enough to process a substrate, and subsequently, it is possible to obtain a pattern of a novolac film having dry etching resistance strong enough for processing by transferring the pattern using dry etching with an oxygen-based gas plasma.

Many techniques have been already known (for example, Patent Document 3) for such an organic lower layer film as described above, but a necessity of excellent embedding characteristics in addition to dry etching resistance has been increasing along with the recent miniaturization progress. There is a necessity of an organic lower layer film material that enables uniform film formation on an underlayer substrate to be processed even when the substrate to be processed or the material have a complicated shape, and has embedding characteristics high enough to embed a necessary pattern with no gaps.

Such an organic lower layer film as described above is formed using a coater/developer capable of a treatment, such as a spin coating process, an EBR process, or a baking process in the production of a semiconductor substrate or the like. The EBR (edge bead removal) process refers to a process in which, after a coating film is formed on a substrate (wafer) by spin coating, the coating film is removed with a removal liquid at the end portion of the substrate for the purpose of preventing contamination on the substrate transport arm of the coater/developer. As the removal liquid for use in the EBR process, there is a mixed liquid of propylene glycol monomethyl ether acetate and propylene glycol monomethyl ether (30 mass%:70 mass%), which is in wide use in the EBR process of a resist film or a resist lower layer film (a silicon-containing middle layer film or an organic lower layer film).

Due to the influence of a removal agent in the EBR process, the organic lower layer film may have a thick film thickness (hump) in the outer peripheral portion. In the dry etching process during the above-described substrate processing, the hump may cause a defect, and there is thus a demand for an organic lower layer film in which a hump is suppressed.

Besides the organic lower layer film, a resist material for use in photolithography using the above-described organic photosensitive polymer is also made to form a film by, similar to the organic lower layer film, applying a solution by a method, such as spin coating, and evaporating the solvent by baking. Similar to the organic lower layer film, the film thickness needs to be uniform and flat after the baking, and the requirement for uniformity and flatness is becoming stricter every year.

In recent years, there has been a demand for a thick resist film in the 3D-NAND memory use, and a higher flatness is in demand. As the film thickness increases, flatness in the film becomes more difficult to achieve. On the other hand, thickness reduction is progressing along with the progress of miniaturization, and in this case, there is an increasing risk of the generation of a pinhole defect or the like.

Examples of film materials for which an organic substance is used that are used as processing materials of semiconductors have been described above, but it also becomes a significant industrial advantage to obtain a material that forms a film having in-plane uniformity in film thickness or having no pinholes from a film-forming material for which no organic substances are used.

Recently, the influence of perfluoroalkyl compounds (PFAS) on health has been pointed out, and there has been a movement to impose restrictions on the production and sales of the PFAS compounds in REACH in Europe. The perfluoroalkyl compounds have diverse uses and are being used in a wide range of uses, such as a water repellent, a surface treatment agent, an emulsifier, a fire extinguishing agent, and a coating agent, due to the characteristics of being repellent to water or oil, being highly resistant to heat and chemicals, and absorbing no light, which are all attributed to the structure, and development of an alternative material not having the PFAS structure has thus become a matter of urgent necessity.

As one example of a material for which the above-described perfluoroalkyl compound is used, a surfactant having a fluoroalkyl group or a silicone chain has a strong effect of decreasing surface tension, and in particular, a fluoroalkyl group-based surfactant for which a risk of the generation of a silicon-derived particle after the dry ashing of a resist film is low is in wide use (Patent Documents 3 and 4). In addition, a fluorine-based surfactant is applied not only to a resist material but also to a top coat that is formed in the upper layer of a resist or an antireflection film in a lower layer that is formed in the lower layer of a resist (Patent Document 5).

From the viewpoint of tightening regulations in the future, there is a need to use a material that is not subject to PFAS regulations. For example, as the above-described surfactant, a surfactant having a trifluoromethoxy group or a pentafluorosulfanyl group and the use thereof have been proposed (Patent Document 6). In addition, in the resist material field, a resist material for which a photoacid generator is used or the like has been proposed (Patent Document 7).

### CITATION LIST

### PATENT LITERATURE

| | |
|---|---|
| Patent Document 1: | JP 4355943 B |
| Patent Document 2: | JP 2004-205685 A |
| Patent Document 3: | JP H06-186735 A |
| Patent Document 4: | JP H06-214380 A |
| Patent Document 5: | JP 2010-139822 A |
| Patent Document 6: | JP 2008-526792 W |
| Patent Document 7: | WO 2023/223624 A1 |

### SUMMARY OF INVENTION

### TECHNICAL PROBLEM

In view of the circumstances, the present invention aims to provide: a composition for forming an organic film that is excellent in terms of film formability (in-plane uniformity) and embedding characteristics on a substrate (wafer), suppresses a hump during an EBR process, and has an excellent process tolerance when used as an organic lower layer film for a multi-layer resist; a method for forming an organic film using this composition; and a patterning process.

### SOLUTION TO PROBLEM

To achieve the object, the present invention provides a composition for forming an organic film containing (A) a resin or compound for forming an organic film, (B) a compound represented by the following general formula (1), and (C) a solvent. 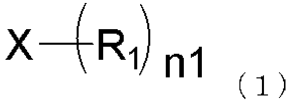
wherein R₁ is a terminal group represented by the following general formula (2), X is an n1-valent organic group having 2 to 50 carbon atoms, and n1 represents an integer of 2 to 8: 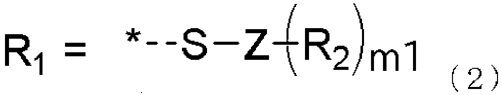
wherein R₂ is a monovalent organic group, wherein the monovalent organic group contains at least any of structures having fluorine atoms represented by the following general formula (3), m1 is 1 or 2, wherein when m1 is 1, Z represents a single bond or a divalent organic group optionally containing a heteroatom, and when m1 is 2, Z represents a trivalent organic group optionally containing a heteroatom, and * represents an attachment point, 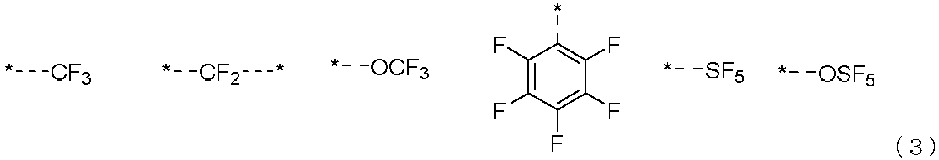
wherein * represents an attachment point.

Such a composition for forming an organic film can form an organic film that is excellent in terms of in-plane uniformity and embedding characteristics and suppresses the formation of a hump attributed to the influence of a removal agent in an EBR process. In addition, the (B) fluorine-containing compound has a partial structure having fluorine atoms introduced into a terminal group of the compound. Therefore, when used in an organic lower layer film, the (B) fluorine-containing compound evaporates from the organic film surface and is removed from the inside of the film by baking for the formation of a cured film, due to the action of the terminal group introduced via S. Thus, the composition for forming an organic film can form an organic film excellent in coatability of a silicon-containing middle layer film, and is excellent in process tolerance.

In addition, the terminal group represented by the general formula (2) in the (B) compound is preferably any one or more of the following general formulae (4) to (8): 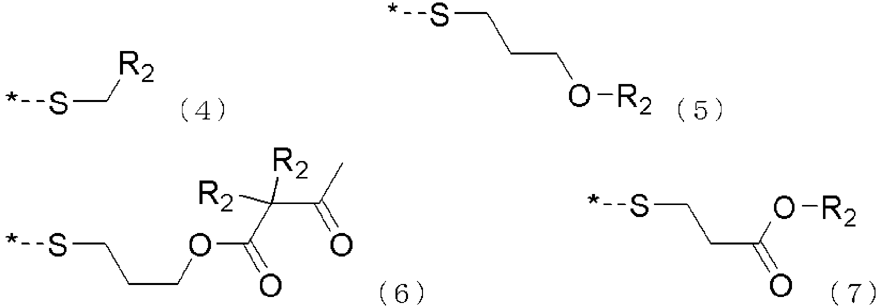 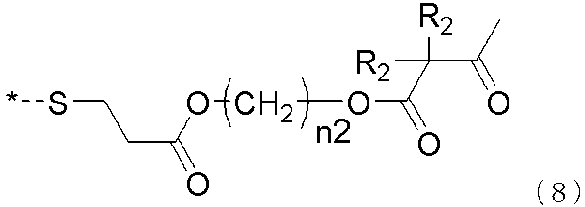 wherein R₂ is as defined above, and n2 represents an integer of 2 to 6.

In the composition for forming an organic film containing the compound having such a structure, since the interaction of the fluorine structure can be adjusted by appropriately selecting the introduction form of the substituent R₂ in a repeating unit, it becomes possible to adjust the function of a surfactant in accordance with the types of the compound or resin and the solvent that are used in the composition for forming an organic film.

In addition, R₂ in the (B) compound is preferably any of groups represented by the following general formula (9):

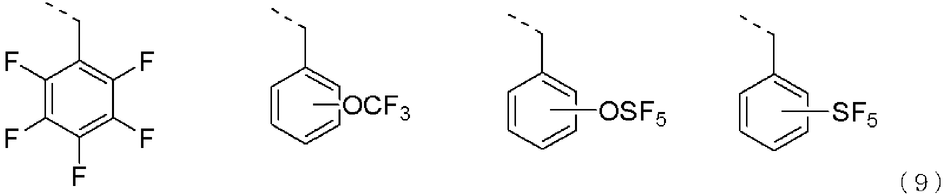

As described above, management of chemical substances relating to PFAS regulations has been strengthened, and the above aromatic groups substituted with fluorine atoms, a pentasulfanyl group, or a pentasulfanyl group do not belong to the PFAS classification in REACH. In addition, aromatic groups substituted with a difluoromethoxy group or fluorine atoms do not belong to the PFAS classification in OECD. Therefore, the composition for forming an organic film for which the compound of the present invention is used as a surfactant not only imparts excellent film formability but can also be expected to serve as an environmentally conscious material which is environmentally friendly.

In addition, the (B) compound preferably has a weight-average molecular weight of 300 to 4000.

Within such a range of weight-average molecular weight, it becomes possible to form an organic film having excellent film formability and embedding characteristics. In addition, it is possible to control the contact angle on the film surface after the formation of the film to be within an appropriate range, and it becomes possible to form an organic lower layer film suitable for use in multi-layer resist processes.

In addition, the ratio Mw/Mn of the weight-average molecular weight Mw to the number-average molecular weight Mn of the (B) compound in terms of polystyrene by gel permeation chromatography is preferably 1.00 ≤ Mw/Mn ≤ 1.20.

Within such an Mw/Mn (molecular weight distribution) range, it is possible to control the contact angle on the film surface after the formation of the film to be within an appropriate range, and it becomes possible to form an organic lower layer film suitable for use in multi-layer resist processes.

In addition, the content of the (B) compound is preferably 0.01 parts by mass to 5 parts by mass based on 100 parts by mass of the (A) resin or compound for forming an organic film.

In the composition for forming an organic film containing the above-described content of the (B) compound, the in-plane uniformity of a formed organic film is superior, which is preferable.

In addition, the present invention provides a method for forming an organic film for use in a process of manufacturing a semiconductor device, in which spin coating of the composition for forming an organic film is performed on a substrate to be processed, and a heat treatment is performed on the substrate coated with the composition for forming an organic film at a temperature of 100°C or higher and 600°C or lower for a range of 10 to 600 seconds, thereby forming a cured film.

The composition for forming an organic film of the present invention is particularly useful in the case of embedding a pattern with a complex shape on a substrate to be processed by spin coating, forming an organic film having excellent in-plane uniformity, and removing the organic film at an end portion while a hump is suppressed in the EBR process.

In addition, the present invention provides a patterning process, in which an organic film is formed on a body to be processed using the composition for forming an organic film, a resist middle layer film is formed on the organic film using a resist middle layer film material containing a silicon atom, a resist upper layer film is formed on the resist middle layer film using a resist upper layer film material composed of a photoresist composition, a circuit pattern is formed in the resist upper layer film, the pattern is transferred to the resist middle layer film by etching using the resist upper layer film in which the circuit pattern has been formed as a mask, the pattern is transferred to the organic film by etching using the resist middle layer film to which the pattern has been transferred as a mask, and the pattern is further transferred to the body to be processed by etching using the organic film to which the pattern has been transferred as a mask.

In addition, the present invention provides a patterning process, in which an organic film is formed on a body to be processed using the composition for forming an organic film, a resist middle layer film is formed on the organic film using a resist middle layer film material containing a silicon atom, an organic antireflective film or an adhesive film is formed on the resist middle layer film, a resist upper layer film is formed on the organic antireflective film or adhesive film using a resist upper layer film material composed of a photoresist composition, a circuit pattern is formed in the resist upper layer film, the pattern is transferred to the organic antireflective film or adhesive film and the resist middle layer film by etching using the resist upper layer film in which the circuit pattern has been formed as a mask, the pattern is transferred to the organic film by etching using the resist middle layer film to which the pattern has been transferred as a mask, and the pattern is further transferred to the body to be processed by etching using the organic film to which the pattern has been transferred as a mask.

In addition, the present invention provides a patterning process, in which an organic film is formed on a body to be processed using the composition for forming an organic film, an inorganic hard mask selected from a silicon oxide film, a silicon nitride film, and a silicon oxynitride film is formed on the organic film, a resist upper layer film is formed on the inorganic hard mask using a resist upper layer film material composed of a photoresist composition, a circuit pattern is formed in the resist upper layer film, the pattern is transferred to the inorganic hard mask by etching using the resist upper layer film in which the circuit pattern has been formed as a mask, the pattern is transferred to the organic film by etching using the inorganic hard mask to which the pattern has been transferred as a mask, and the pattern is further transferred to the body to be processed by etching using the organic film to which the pattern has been transferred as a mask.

In addition, the present invention provides a patterning process, in which an organic film is formed on a body to be processed using the composition for forming an organic film, an inorganic hard mask selected from a silicon oxide film, a silicon nitride film, and a silicon oxynitride film is formed on the organic film, an organic antireflective film or an adhesive film is formed on the inorganic hard mask, a resist upper layer film is formed on the organic antireflective film or adhesive film using a resist upper layer film material composed of a photoresist composition, a circuit pattern is formed in the resist upper layer film, the pattern is transferred to the organic antireflective film or adhesive film and the inorganic hard mask by etching using the resist upper layer film in which the circuit pattern has been formed as a mask, the pattern is transferred to the organic film by etching using the inorganic hard mask to which the pattern has been transferred as a mask, and the pattern is further transferred to the body to be processed by etching using the organic film to which the pattern has been transferred as a mask.

As described above, the composition for forming an organic film of the present invention can be suitably used in a variety of patterning processes, such as a three-layer resist process in which a silicon-containing resist middle layer film or an inorganic hard mask is used or a four-layer resist process in which, in addition to the silicon-containing resist middle layer film or the inorganic hard mask, an organic antireflection film or an adhesive film is used, and such a patterning process of the present invention makes it possible to highly accurately transfer and form a circuit pattern of a resist upper layer film onto a body to be processed.

The inorganic hard mask is preferably formed by a CVD method or an ALD method.

In the patterning process of the present invention, it is possible to form an inorganic hard mask by, for example, such a method as described above.

In addition, in the formation of the circuit pattern, the circuit pattern is preferably formed by lithography using light having a wavelength of 10 nm or longer and 300 nm or shorter, direct writing using an electron beam, nanoimprinting, or a combination thereof.

In addition, in the formation of the circuit pattern, the circuit pattern is preferably developed by alkali development or with an organic solvent.

In the patterning process of the present invention, it is possible to suitably use such forming means and developing means of the circuit pattern as described above.

In addition, the body to be processed is preferably a semiconductor device substrate or the semiconductor device substrate having any one of a metal film, a metal carbide film, a metal oxide film, a metal nitride film, a metal oxycarbide film, and a metal oxynitride film formed thereon.

At this time, a metal that constitutes the body to be processed is preferably silicon, titanium, tungsten, hafnium, zirconium, chromium, germanium, copper, silver, gold, aluminum, indium, gallium, arsenic, palladium, iron, tantalum, iridium, molybdenum, or an alloy thereof.

According to the patterning process of the present invention, it is possible to form a pattern by processing a body to be processed as described above.

In addition, the present invention provides a compound represented by the following general formula (1): 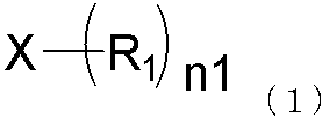
wherein R₁ is a terminal group represented by the following general formula (2), X is an n1-valent organic group having 2 to 50 carbon atoms, and n1 represents an integer of 2 to 8: 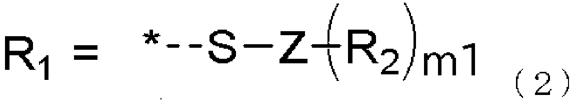
wherein R₂ is a monovalent organic group, wherein the monovalent organic group contains at least any of structures having fluorine atoms represented by the following general formula (3), m1 is 1 or 2, wherein when m1 is 1, Z represents a single bond or a divalent organic group optionally containing a heteroatom, and when m1 is 2, Z represents a trivalent organic group optionally containing a heteroatom, and * represents an attachment point, 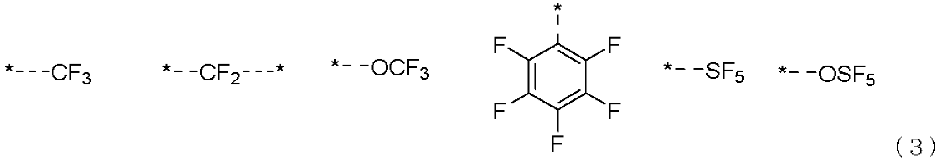
wherein * represents an attachment point.

Such a compound for forming an organic film can form an organic film that is excellent in terms of in-plane uniformity and embedding characteristics and suppresses the formation of a hump attributed to the influence of a removal agent in an EBR process. In addition, such a fluorine-containing compound has a partial structure having fluorine atoms introduced into a terminal group of the compound. Therefore, when used in an organic lower layer film, the (B) fluorine-containing compound evaporates from the organic film surface and is removed from the inside of the film by baking for the formation of a cured film, due to the action of the terminal group introduced via S. Thus, the compound can be used for a composition for forming an organic film that can form an organic film excellent in coatability of a silicon-containing middle layer film, and is excellent in process tolerance.

In addition, the terminal group represented by the general formula (2) is preferably any one or more of the following general formulae (4) to (8): 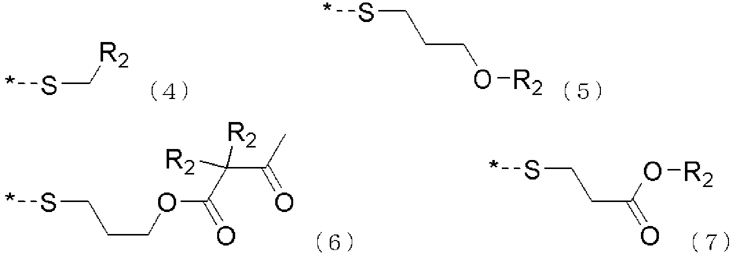 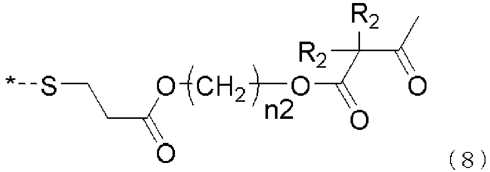 wherein R₂ is as defined above, and n2 represents an integer of 2 to 6.

The compound having such a structure is capable of enhancing the interaction between fluorine atoms by the action of the substituent R₂ containing a fluorine atom introduced into the terminal group, which makes it possible to impart favorable film formability when the compound of the present invention is used as a surfactant.

In addition, the R₂ is preferably any of groups represented by the following general formula (9):

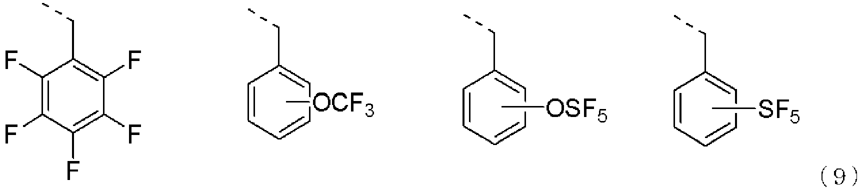

As described above, the above-described compound having a fluorine structure can be expected to serve as a material having a small environmental impact. In particular, the compound can be expected to serve as an environmentally conscious material capable of coping with the recent PFAS regulations.

### ADVANTAGEOUS EFFECTS OF INVENTION

As described above, the present invention is capable of providing a composition for forming an organic film that is excellent in terms of film formability (in-plane uniformity) and embedding characteristics on a substrate (wafer) and film formability on an organic film when used as an organic lower layer film and suppresses a hump during an EBR process. Furthermore, the present invention can be applied to a variety of organic film-forming materials by combining a specific terminal group into the compound. Particularly, the composition for forming an organic film of the present invention is excellent in terms of film formability, embedding characteristics, and a property of suppressing a hump that is generated during an EBR process and is thus extremely useful as a film-forming material for manufacturing a semiconductor device, such as an organic film material, a photoresist material, or a silicon-containing resist middle layer film material for use in multi-layer resist processes, such as a two-layer resist process, a three-layer resist process in which a silicon-containing resist middle layer film or an inorganic hard mask is used, or a four-layer resist process in which a silicon-containing resist middle layer film or an inorganic hard mask and an organic antireflection film or an adhesive film are used. Therefore, in the method for forming an organic film of the present invention, it is possible to form an organic film in which a hump is suppressed, and a semiconductor element or the like can be thus efficiently manufactured. In addition, it is also possible to reduce the environmental impact by selecting a specific substituent structure.

### BRIEF DESCRIPTION OF DRAWINGS

FIG. 1 is one example of a graph of the heights of humps measured using a contact-type profiler in a composition for forming an organic film in which a hump is suppressed;
FIG. 2 is one example of a graph of the heights of humps measured using the contact-type profiler in a composition for forming an organic film in which a hump is not suppressed;
FIG. 3 is an explanatory view of one example of a patterning process by a three-layer resist process of the present invention; and
FIG. 4 is an explanatory view of a method for evaluating embedding characteristics in Examples.

### DESCRIPTION OF EMBODIMENTS

As described above, there has been a demand for a material not only imparting film formability particularly as an organic film material but also having a surfactant effect with a small environmental impact. In addition, there has been a demand for development of a composition for forming an organic film that is excellent in terms of film formability (in-plane uniformity) and embedding characteristics on a substrate (wafer) at the time of using an organic film material as an organic lower layer film material and suppresses a hump during an EBR process.

Normally, at the time of forming an organic film, a resin for forming an organic film or an additive is dissolved in an organic solvent to produce a composition, this composition is applied onto a substrate on which a structure, wiring, or the like has been formed with a coater/developer, the composition is spread by rotating the substrate, the composition at an end portion is removed by an EBR process, and the composition is then baked, thereby forming an organic film.

In a case where the surfactant effect of the composition is not sufficient, it is considered that the distribution of the surfactant in the film becomes uneven, which generates a void at the time of embedding a hole or trench having an extremely high aspect ratio and generates a hump at the outer peripheral portion of the organic film in a case where the resin for forming an organic film or the additive is poorly soluble in a removal agent for use in the EBR process.

The present inventors further repeated intensive studies, found that a composition for forming an organic film becomes excellent in terms of film formability, advanced embedding characteristics, and suppression of a hump during an EBR process by blending a compound having a specific terminal structure therewith, and completed the present invention. In addition, when a specific substituent structure is selected, reduction of the environmental impact can also be expected, and the composition for forming an organic film can also be expected to serve as an industrially available composition for forming an organic film.

That is, the present invention is a composition for forming an organic film containing (A) a resin or compound for forming an organic film, (B) a compound represented by the general formula (1), and (C) a solvent.

Hereinafter, the present invention will be described in detail, but the present invention is not limited thereto.

### [Compound]

A compound of the present invention is a compound represented by the following general formula (1) and is a compound (B) for use in a composition for forming an organic film of the present invention, which will be described below, (hereinafter referred to as the (B) compound): 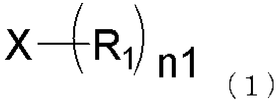
wherein R₁ is a terminal group represented by the following general formula (2), X is an n1-valent organic group having 2 to 50 carbon atoms, and n1 represents an integer of 2 to 8: 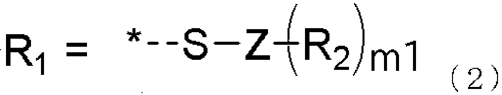
wherein R₂ is a monovalent organic group, wherein the monovalent organic group contains at least any of structures having fluorine atoms represented by the following general formula (3), m1 is 1 or 2, wherein when m1 is 1, Z represents a single bond or a divalent organic group optionally containing a heteroatom, and when m1 is 2, Z represents a trivalent organic group optionally containing a heteroatom, and * represents an attachment point, 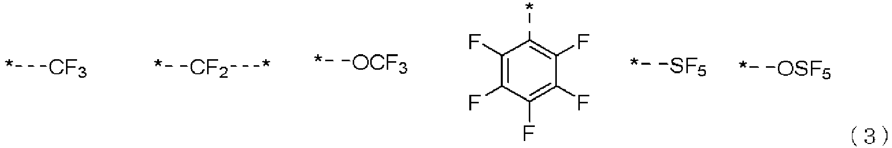
wherein * represents an attachment point.

Examples of X in the general formula (1) of the (B) compound include the following. Among the following, n1-valent organic groups for which n1 is an integer of 3 or more are preferable, and compounds for which n1 is 4 or 6 are more preferable. When n1 is 3 or more, an excellent surfactant effect can be exhibited by the interaction of a fluorine structure represented by R₂ in the terminal group structure.

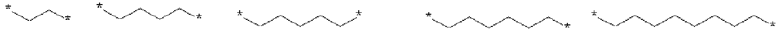

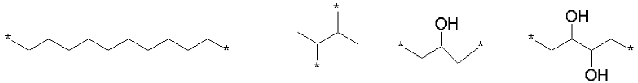

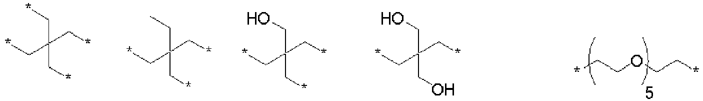

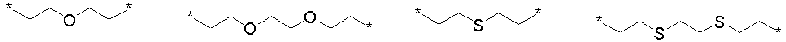

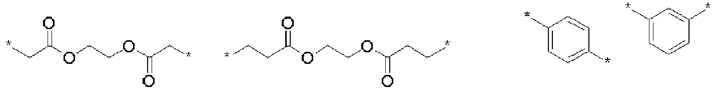

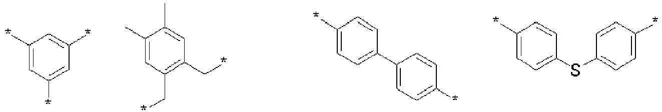

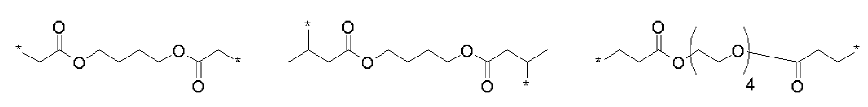

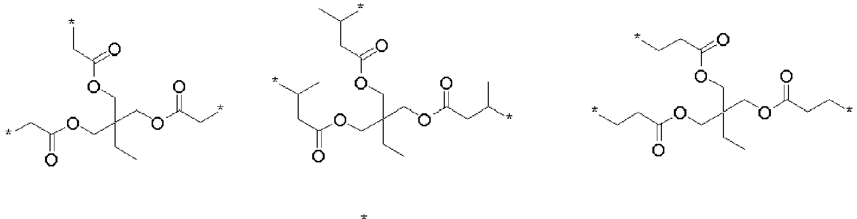

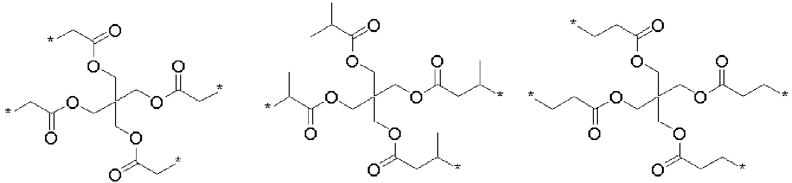

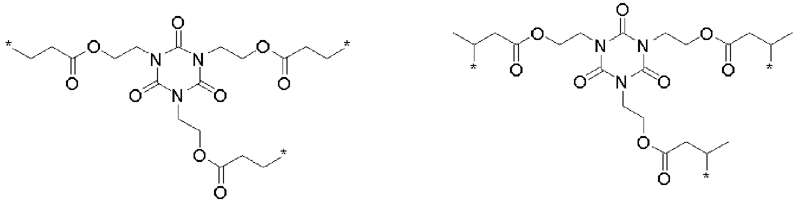

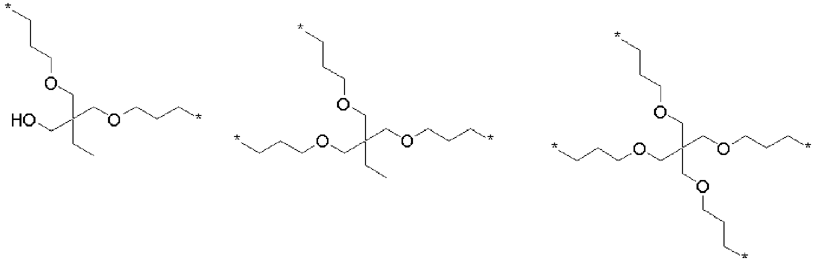

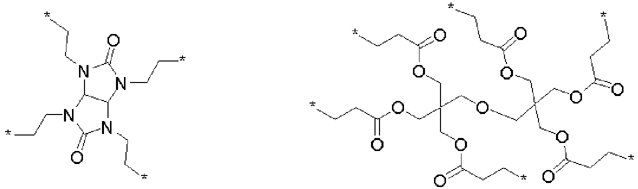

In the general formula (2) that represents R₁, the number of carbon atoms in the organic group optionally containing a divalent or trivalent heteroatom represented by Z is not particularly limited, and the number of carbon atoms can be set to, for example, 1 to 20. In addition, in the general formula (2), the monovalent organic group as R₂ is also not particularly limited as long as the monovalent organic group contains any of structures having fluorine atoms represented by the general formula (3) and can be set to, for example, a monovalent organic group having 1 to 30 carbon atoms and optionally having a heteroatom.

The substituent R₂ having fluorine that is contained in the terminal group of the (B) compound is preferably an aromatic group substituted with fluorine atoms, an aromatic group substituted with a trifluoromethoxy group, an aromatic group substituted with a pentasulfanyl group, or an aromatic group substituted with a pentasulfanyloxy group, represented by the following general formula (9).

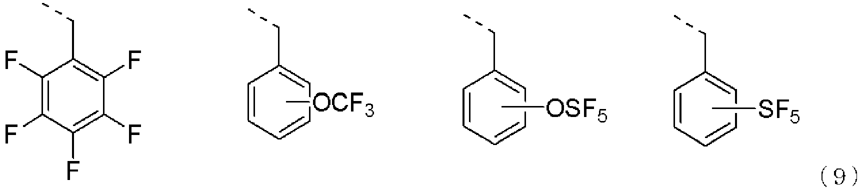

With the above structure, the use as a compound for a material for forming an organic film having a fluorine structure that is not subject to PFAS regulations can be expected.

The terminal group represented by the general formula (2) of the (B) compound is preferably any one or more of the following general formulae (4) to (8): 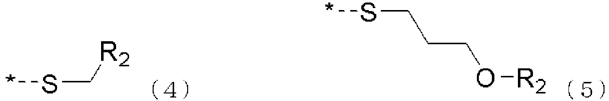 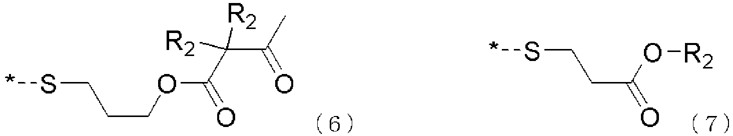 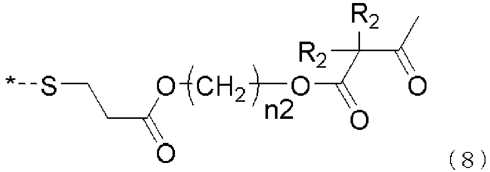 wherein R₂ is as defined above, and n2 represents an integer of 2 to 6.

### [Compound: Terminal structure represented by general formula (4)]

Examples of a terminal structure represented by the general formula (4) include the following. Among these, a terminal structure represented by (9) is preferably contained.

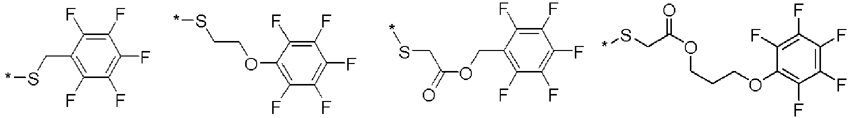

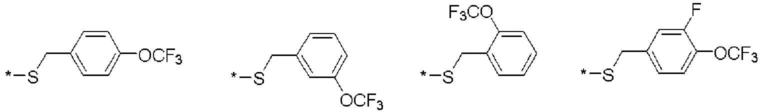

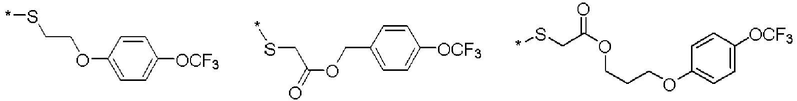

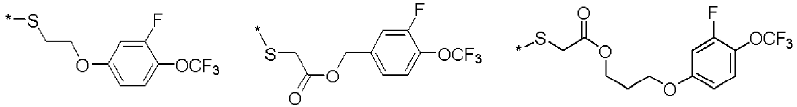

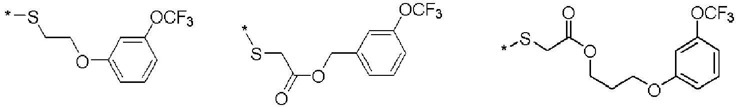

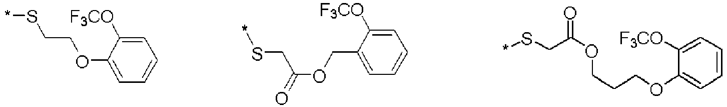

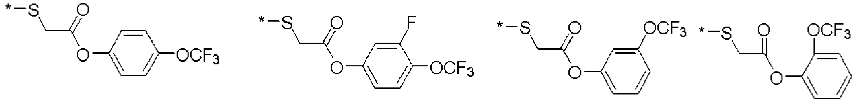

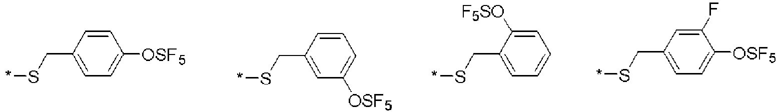

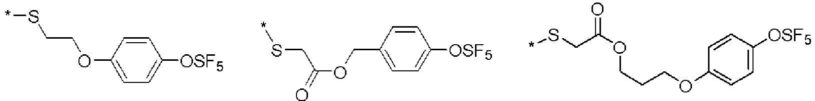

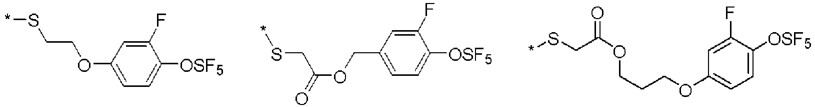

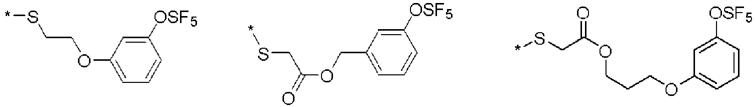

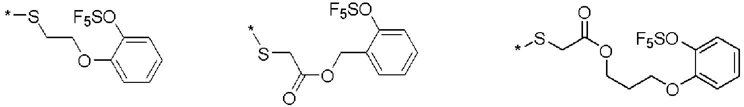

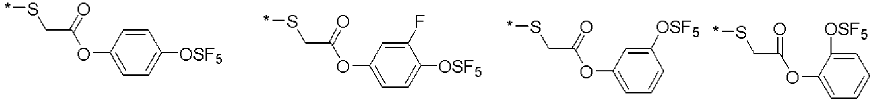

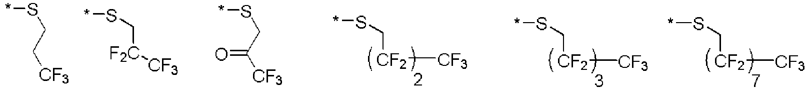

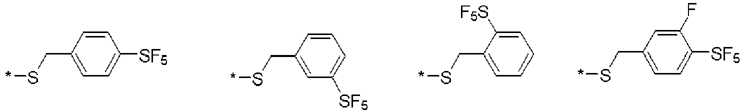

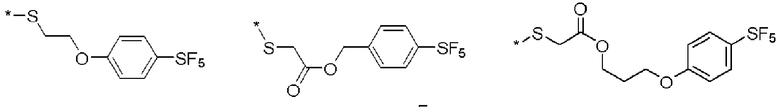

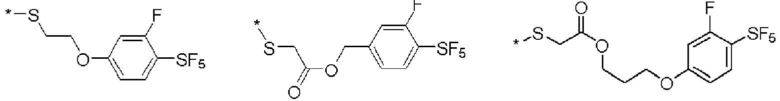

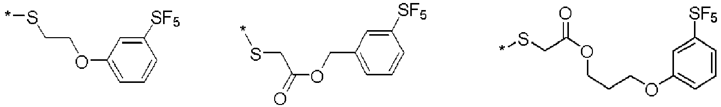

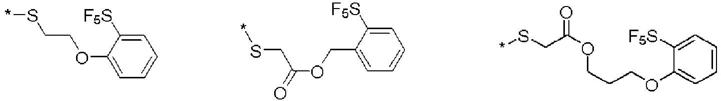

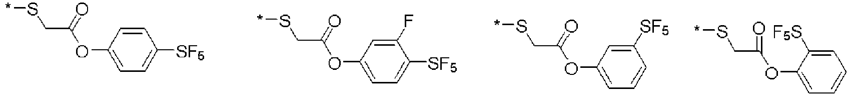

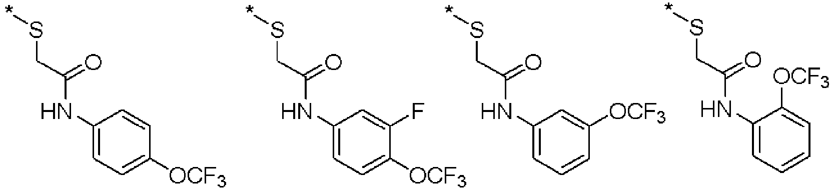

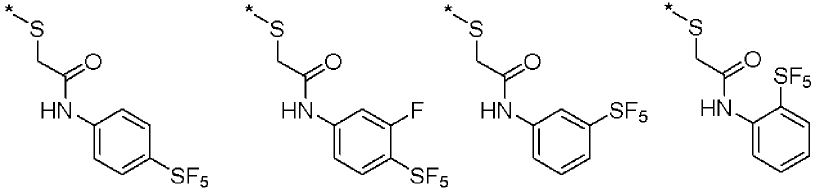

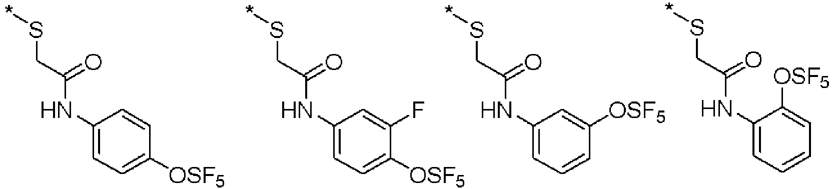

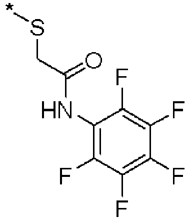

### [Compound: Method for producing terminal structure represented by general formula (4)]

Examples of a method for producing the compound having the terminal structure represented by the general formula (4) include a method in which the compound is obtained by an addition reaction between a halide, mesylate, tosylate, or the like having R₂ as a substituent and a thiol compound under a base catalyst shown below. 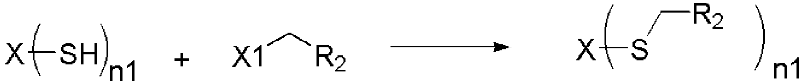 wherein X1 represents a halide, mesylate, or tosylate, and X, R₂, and n1 are as defined above.

Examples of the base catalyst that is used at this time include inorganic base compounds such as sodium bicarbonate, sodium carbonate, potassium carbonate, calcium carbonate, cesium carbonate, sodium hydroxide, potassium hydroxide, sodium hydride, and potassium phosphate; organic amine compounds such as triethylamine, pyridine, and N-methylmorpholine, and these may be used singly or two or more thereof may be used in combination. The amount of this catalyst used is within a range of 0.1 to 20 mol, preferably 0.2 to 10 mol, based on the mole number of a thiol group in the raw material.

A solvent that is used at this time is not particularly limited as long as the solvent is inert to the reaction, and for example, ether-based solvents such as diethyl ether, tetrahydrofuran, and dioxane; aromatic solvents such as benzene, toluene, and xylene; acetonitrile, dimethyl sulfoxide, N,N-dimethylformamide, N-methylpyrrolidone, and water can be used singly or as a mixture. These solvents can be used within a range of 0 to 2000 parts by mass based on 100 parts by mass of the reaction raw materials, and the reaction temperature is preferably -50°C to about the boiling point of the solvent and more preferably room temperature to 150°C. The reaction time is selected as appropriate from 0.1 to 100 hours.

Examples of a reaction method include a method in which the thiol compound, the halide, mesylate, or tosylate having R₂ as a substituent, and the catalyst are charged at once, a method in which the thiol compound and the halide, mesylate, or tosylate having R₂ as a substituent are dispersed or dissolved and the catalyst is then added at once or diluted with a solvent and added dropwise, and a method in which the catalyst is dispersed or dissolved and the thiol compound and the halide, mesylate, or tosylate having R₂ as a substituent are then added at once or diluted with a solvent and added dropwise. After the end of the reaction, the compound may be used as it is as an organic film material, but the compound can also be diluted into an organic solvent and then collected by performing separate cleaning thereon to remove the unreacted raw material, catalyst, or the like present in the system.

An organic solvent that is used at this time is not particularly limited as long as the organic solvent is capable of dissolving the compound and is separated into two layers when mixed with water, and examples thereof include hydrocarbons such as hexane, heptane, benzene, toluene, and xylene; esters such as ethyl acetate, n-butyl acetate, and propylene glycol methyl ether acetate; ketones such as methyl ethyl ketone, methyl amyl ketone, cyclohexanone, and methyl isobutyl ketone; ethers such as diethyl ether, diisopropyl ether, methyl tert-butyl ether, and ethyl cyclopentyl methyl ether; chlorine-based solvents such as methylene chloride, chloroform, dichloroethane, and trichloroethylene, and mixtures thereof. As cleaning water that is used at this time, normally, water called deionized water or ultrapure water may be used. The number of times of the cleaning needs to be once or more and is preferably about once to five since it is not always true that a matching cleaning effect can be obtained even when the cleaning is performed 10 or more times.

In order to remove the unreacted raw material or an acidic component in the system during the separate cleaning, the cleaning may be performed with a basic aqueous solution. Specific examples of a base include alkali metal hydroxides, alkali metal carbonates, alkaline earth metal hydroxides, alkaline earth metal carbonates, ammonia, and organic ammonium.

Furthermore, in order to remove the unreacted raw material and a metal impurity or a base component in the system during the separate cleaning, the cleaning may be performed with an acidic aqueous solution. Specific examples of an acid include inorganic acids such as hydrochloric acid, hydrobromic acid, sulfuric acid, nitric acid, phosphoric acid, and heteropolyacids; organic acids such as oxalic acid, trifluoroacetic acid, methanesulfonic acid, benzenesulfonic acid, p-toluenesulfonic acid, and trifluoromethanesulfonic acid.

The separate cleaning may be separate cleaning with any one of the basic aqueous solution or the acidic aqueous solution alone, but the separate cleaning can also be performed with a combination of the basic aqueous solution and the acidic aqueous solution. The separate cleaning is preferably performed with the basic aqueous solution and the acidic aqueous solution in order from the viewpoint of removing the metal impurity.

After the separate cleaning with the basic aqueous solution and the acidic aqueous solution, the system may be subsequently cleaned with neutral water. The number of times of the cleaning needs to be once or more and is preferably about once to five times. As the neutral water, deionized water or ultrapure water described above may be used. The number of times of the cleaning needs to be once or more, but there is a case where the base component and the acidic component cannot be removed if the number of times of the cleaning is small. The number of times of the cleaning is preferably about once to five times since it is not always true that a matching cleaning effect can be obtained even when the cleaning is performed 10 or more times.

Furthermore, a reaction product after the separation operation can also be collected as a powder by performing concentration and drying or a crystallization operation on the solvent at reduced pressure or normal pressure, but it is also possible to put the reaction product into a solution state with an appropriate concentration to improve operability at the time of preparing the organic film material. The concentration at this time is preferably 0.1 to 50 mass% and more preferably 0.5 to 30 mass%. At such a concentration, since the viscosity is less likely to become high, it is possible to prevent the operability from being impaired, and the amount of the solvent does not become excessive, which is economically efficient.

The solvent at this time is not particularly limited as long as the solvent is capable of dissolving the compound, specific examples thereof include ketones such as cyclohexanone and methyl-2-amyl ketone; alcohols such as 3-methoxybutanol, 3-methyl-3-methoxybutanol, 1-methoxy-2-propanol, and 1-ethoxy-2-propanol; ethers such as propylene glycol monomethyl ether, ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono tert-butyl ether acetate, and these can be used singly or two or more thereof can be used as a mixture.

Furthermore, for the production of the compound that is obtained by this method, it is possible to use a method in which a plurality of types of halides, tosylate, or mesylate having R₂ as a substituent are used, or it is possible to use a combination with a halide, mesylate, or tosylate that is different from R₂, and a variety of combinations can be selected in accordance with required performance.

### [Compound: Terminal structure represented by general formula (5)]

Examples of a terminal structure R₂ in the general formula (5) include the following. Among these, the terminal structure represented by (9) is preferably contained.

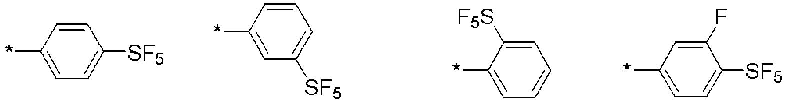

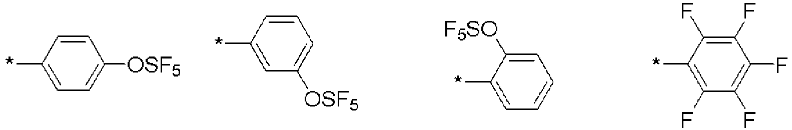

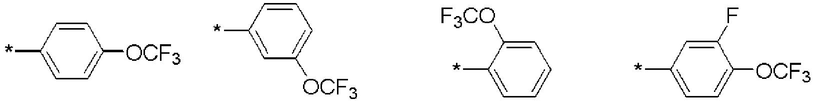

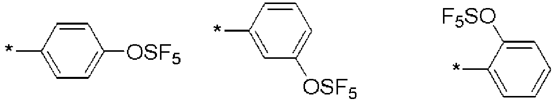

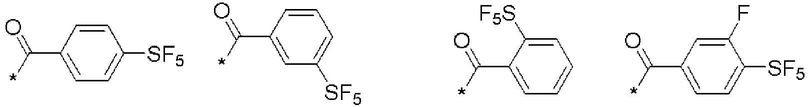

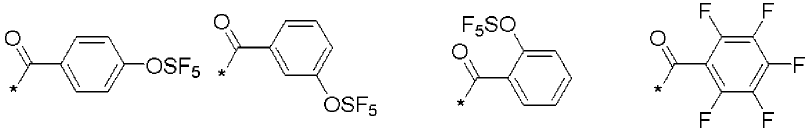

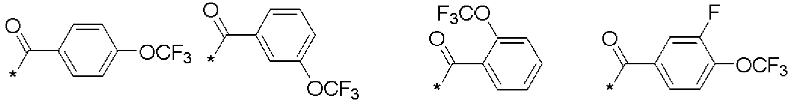

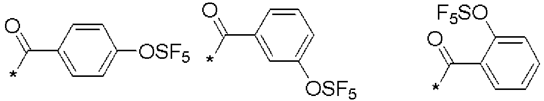

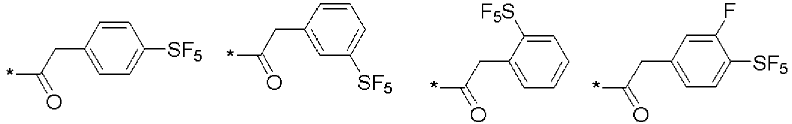

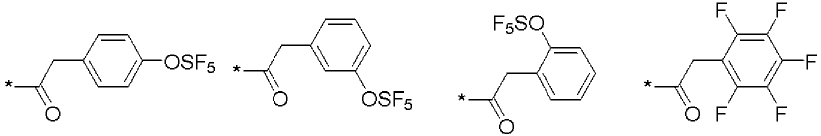

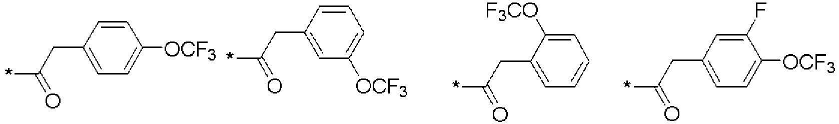

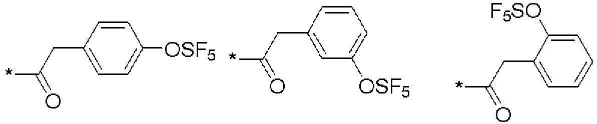

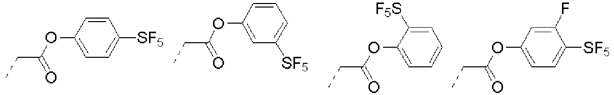

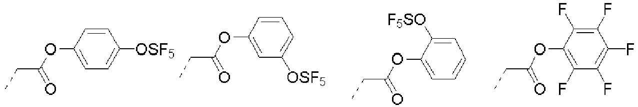

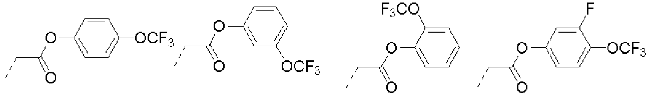

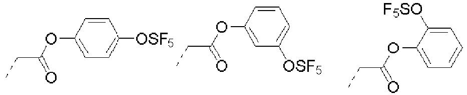

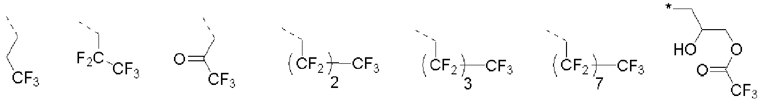

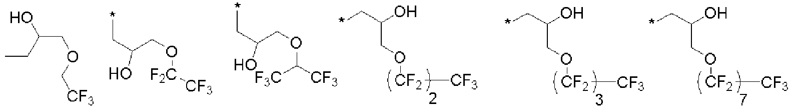

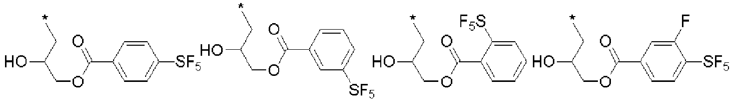

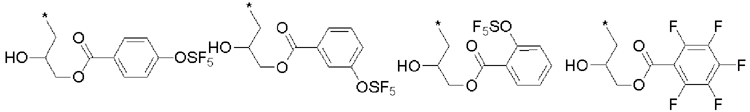

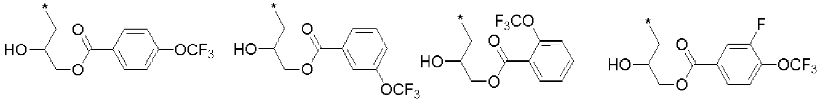

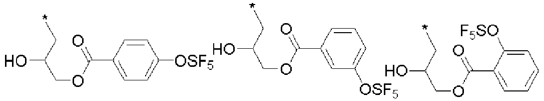

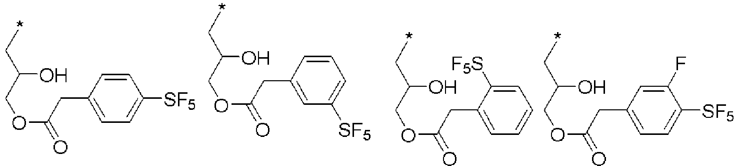

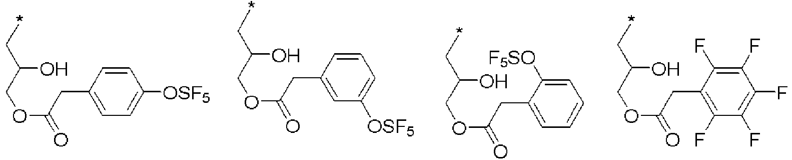

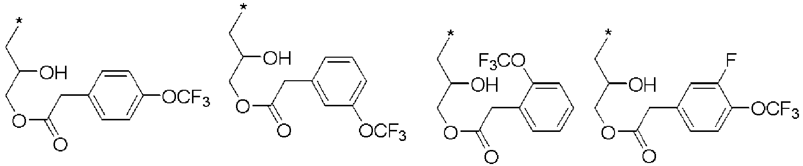

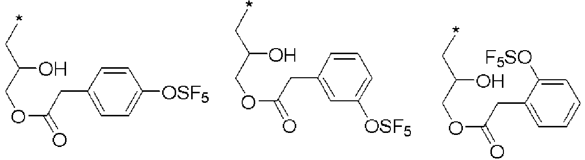

### [Compound: Method for producing terminal structure represented by general formula (5)]

Examples of a method for producing the compound having the terminal structure represented by the general formula (5) include a method in which the compound is obtained by an addition reaction between an allyl ether compound and a thiol compound, using a radical initiator, shown below. 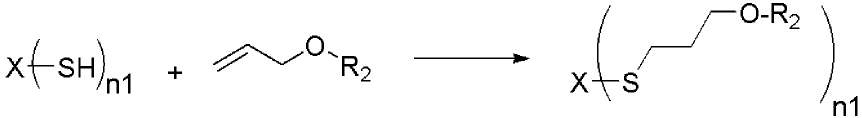 wherein X, R₂, and n1 are as defined above.

Examples of the radical initiator include 2,2'-azobisisobutyronitrile (AIBN), 2,2'-azobis(2,4-dimethylvaleronitrile), dimethyl-2,2-azobis(2-methylpropionate), 1,1'-azobis(1-acetoxy-1-phenylethane), benzoyl peroxide, and lauroyl peroxide. The amount of this initiator added is preferably 0.01 to 25 mol% based on the total of monomers to be polymerized.

Examples of an organic solvent that is used during the addition reaction include toluene, benzene, THF, diethyl ether, dioxane, cyclohexane, cyclopentane, methyl ethyl ketone (MEK), propylene glycol monomethyl ether acetate (PGMEA), and γ-butyrolactone (GBL). The reaction temperature is preferably 50°C to 150°C and more preferably 60°C to 100°C. These solvents can be used within a range of 0 to 2000 parts by mass based on 100 parts by mass of the reaction raw materials, and the reaction temperature is preferably -50°C to about the boiling point of the solvent and more preferably room temperature to 150°C. The reaction time is selected as appropriate from 0.1 to 100 hours. The reaction time is preferably two to 24 hours in consideration of the half-life of the radical initiator or the like and more preferably two to 18 hours from the viewpoint of the production efficiency.

The radical initiator may be added to a thiol compound solution prepared in advance and supplied to a reaction vessel, and an initiator solution different from the thiol compound solution may be prepared, and the thiol compound solution and the initiator solution may be each independently supplied to a reaction vessel.

A plurality of allyl ether compounds may be used in accordance with required performance. The allyl ether compound may be appropriately set in accordance with required performance, as in the reaction with the halide.

A reaction solution obtained by the production method may be used as it is in the composition for forming an organic film or a powder obtained by a purification process, such as a reprecipitation method in which the reaction solution is added to a poor solvent and a powder is obtained may be handled as a final product. A compound solution obtained by dissolving a powder obtained by a purification process for removing the radical initiator or an unreacted raw material in a solvent is preferably handled as a final product. In addition, the obtained powder can also be collected by performing the separate cleaning with water described in the method for producing the terminal structure represented by the general formula (4) or the like thereon.

### [Compound: Terminal structure represented by general formula (6)]

Examples of a terminal structure represented by the general formula (6) include the following. Among these, the terminal structure represented by the general formula (9) is preferably contained.

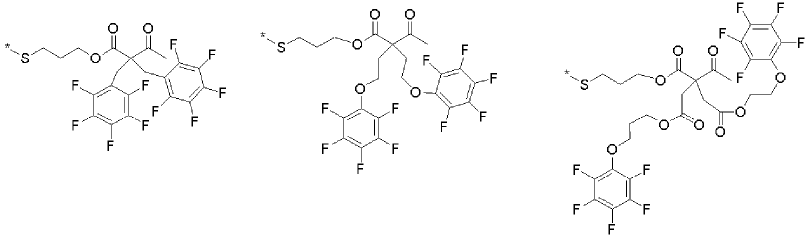

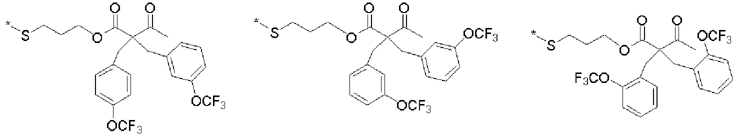

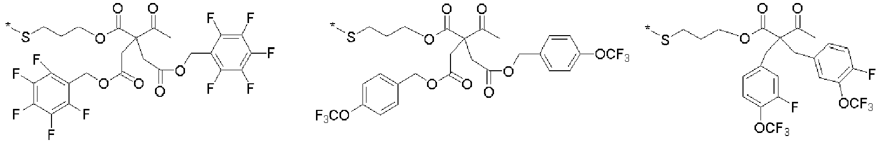

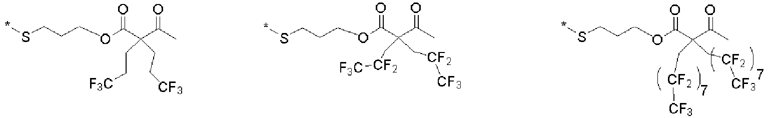

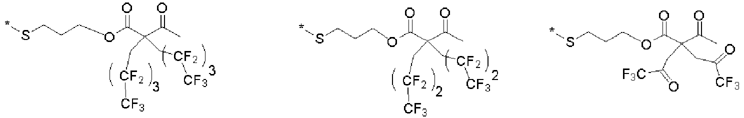

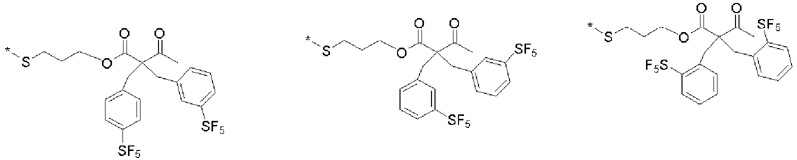

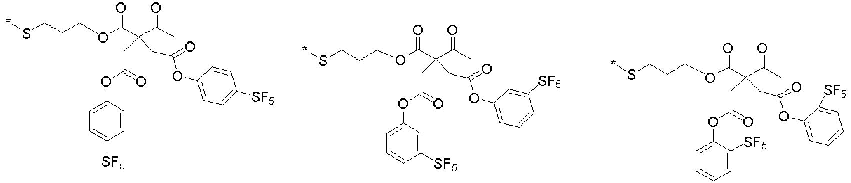

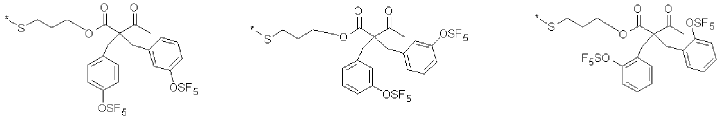

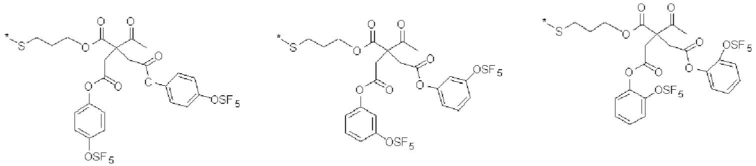

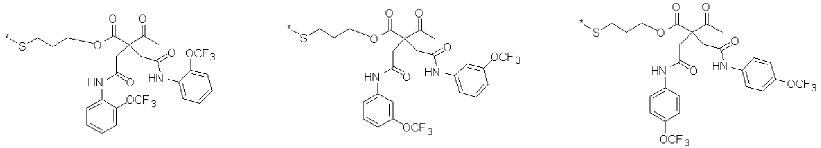

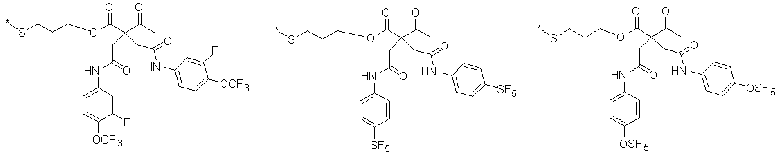

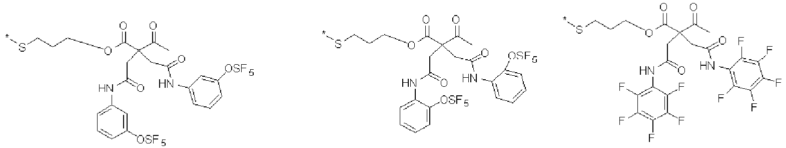

### [Compound: Method for producing terminal structure represented by general formula (6)]

Examples of a method for producing the compound having the terminal structure represented by the general formula (6) include a method in which the compound is obtained by an addition reaction between an allyl ether compound and a thiol compound, using a radical initiator, shown below. 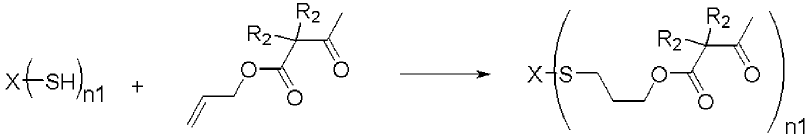 wherein X, R₂, and n1 are as defined above.

The compound can be produced by the method described in the method for producing the terminal structure represented by the general formula (5) except that an allyl ether compound corresponding to the terminal structure of the general formula (6) is used as a raw material in the reaction. In these reactions, a plurality of types of allyl ether compounds can be used, and it is possible to select allyl ether compounds as appropriate in accordance with required performance so that, for example, an allyl ether corresponding to the terminal structure of the general formula (5) is combined with an allyl ether compound corresponding to the terminal structure of the general formula (6).

### [Compound: Terminal structure represented by general formula (7)]

Examples of a terminal structure represented by the general formula (7) include the following. Among these, the terminal structure represented by the general formula (9) is preferably contained.

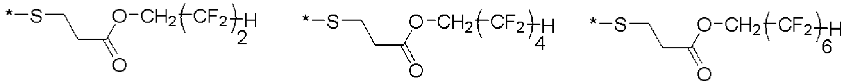

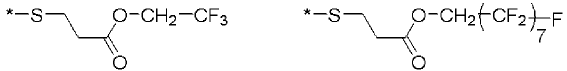

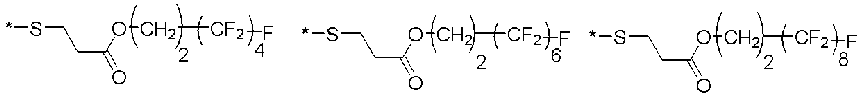

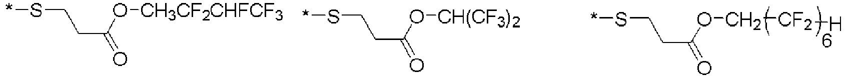

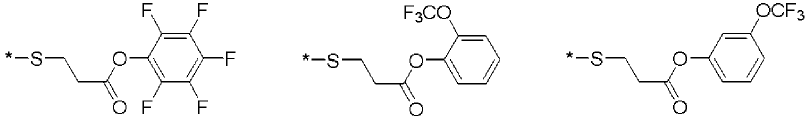

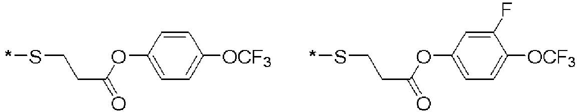

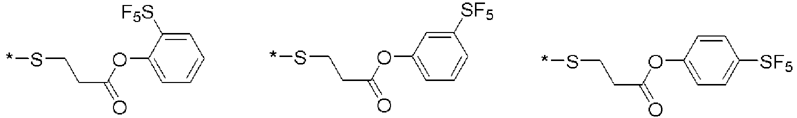

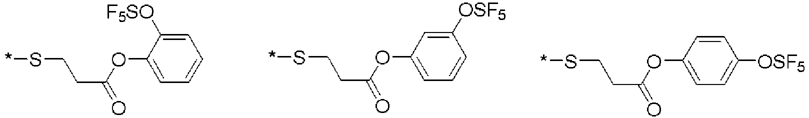

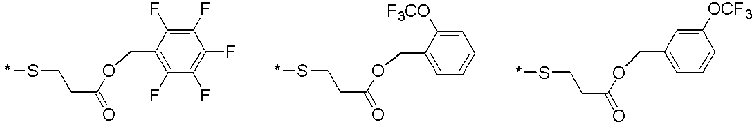

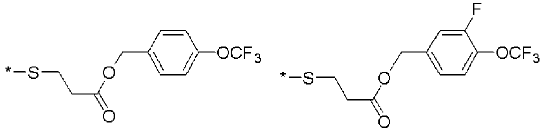

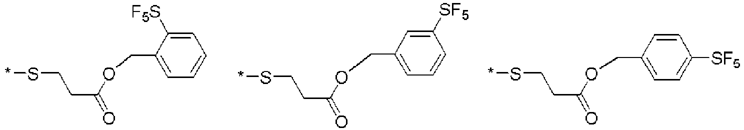

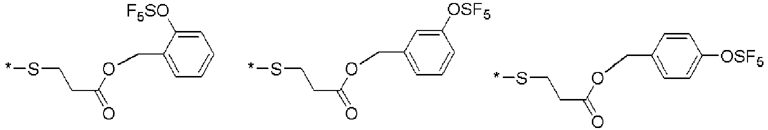

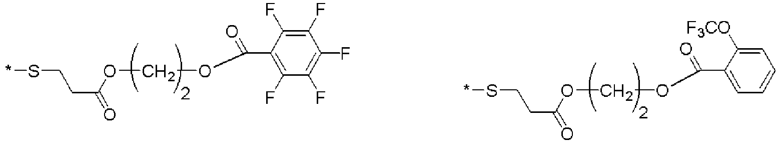

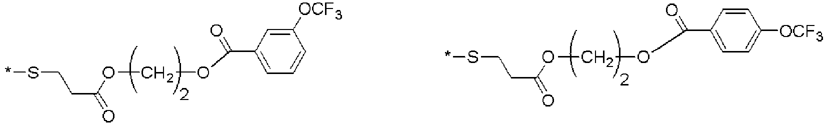

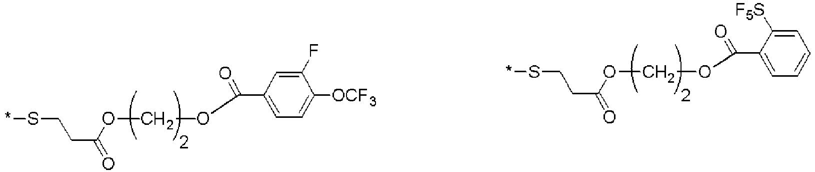

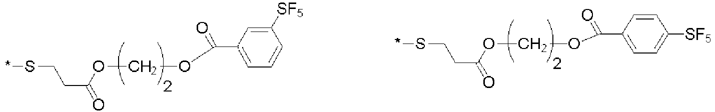

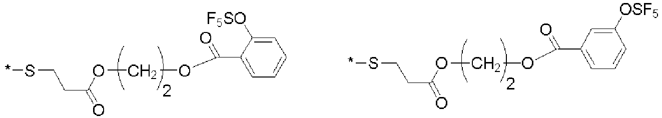

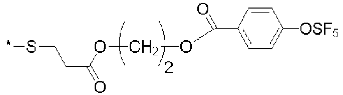

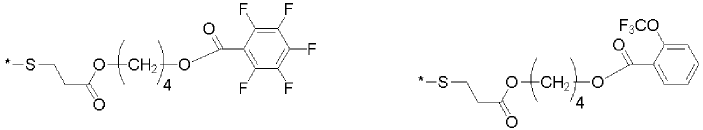

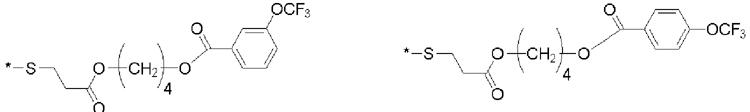

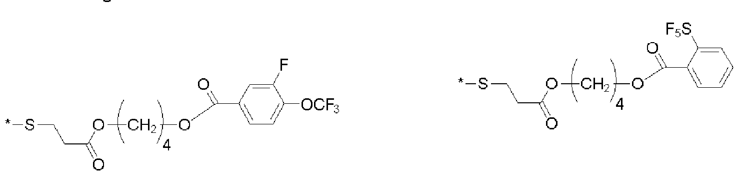

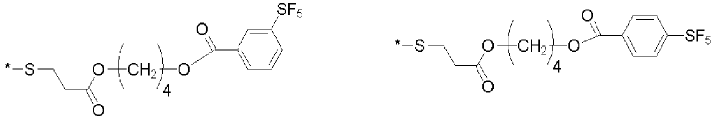

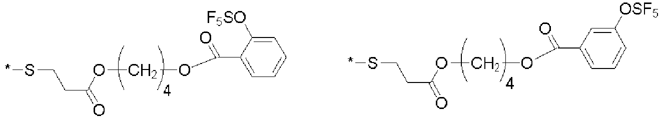

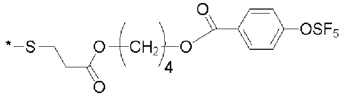

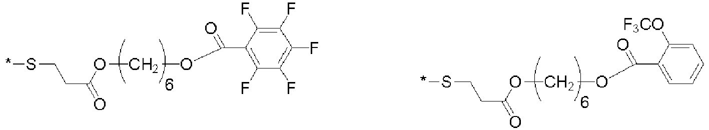

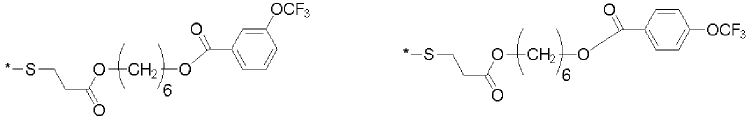

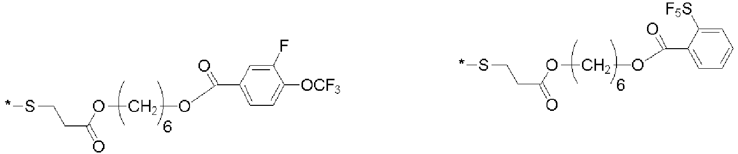

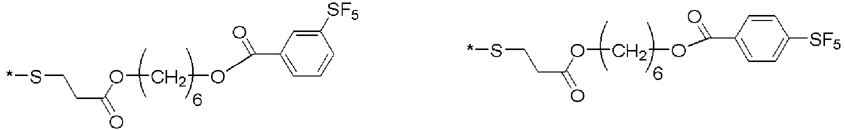

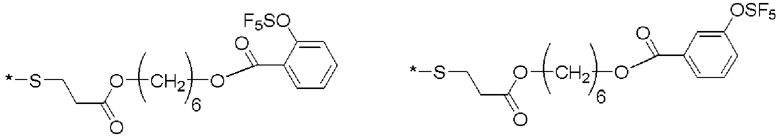

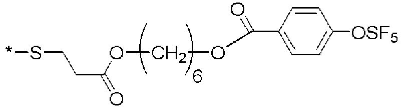

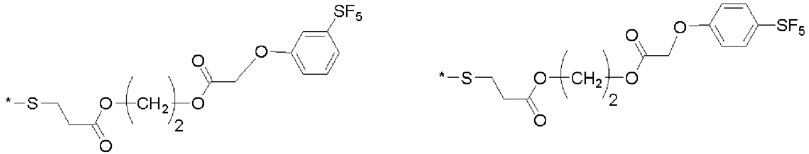

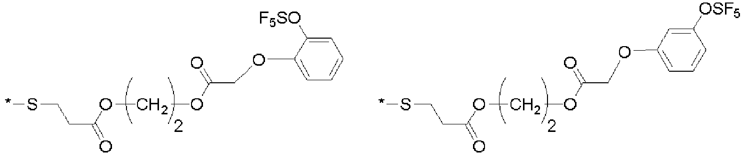

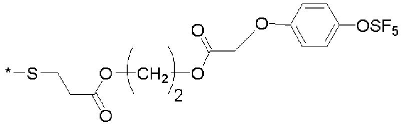

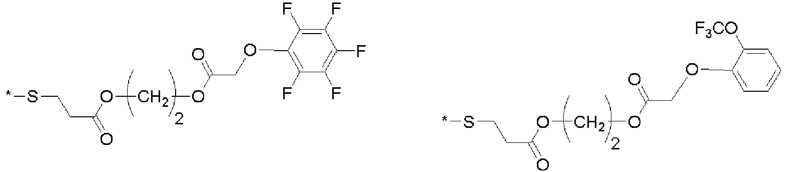

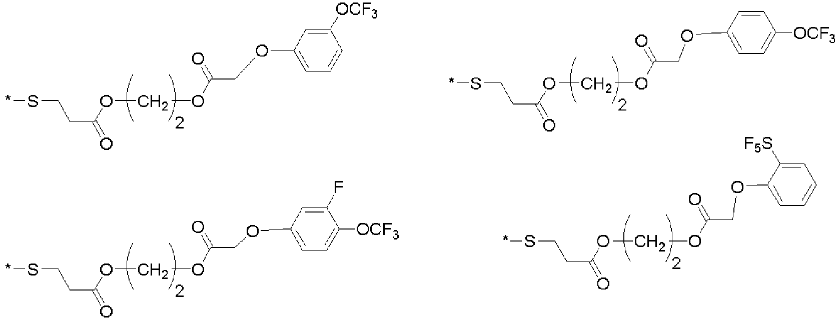

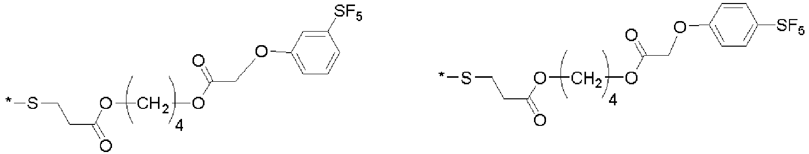

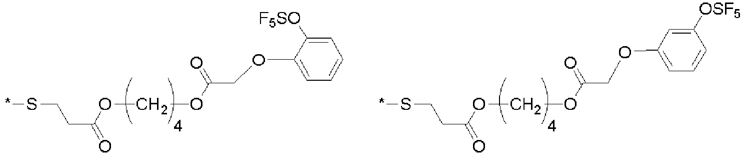

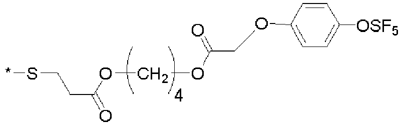

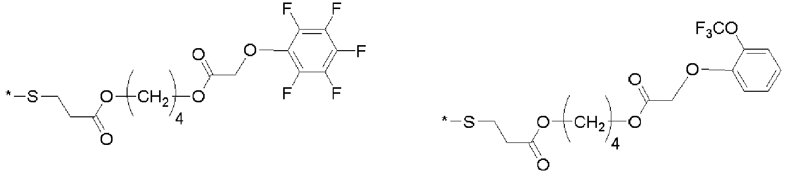

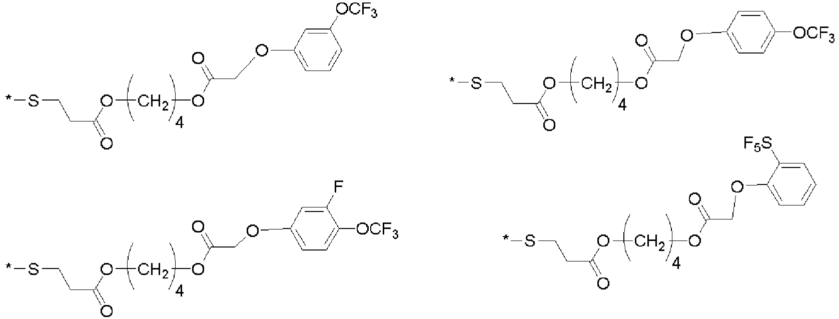

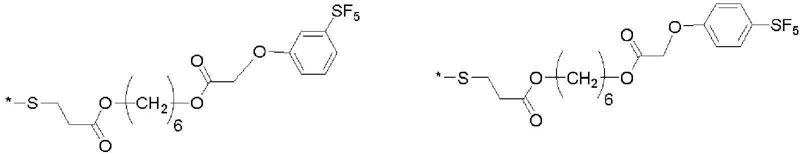

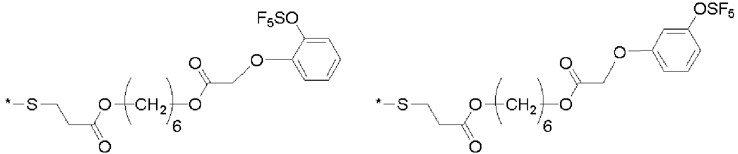

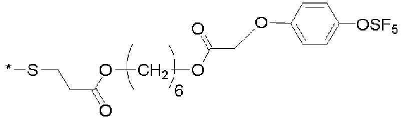

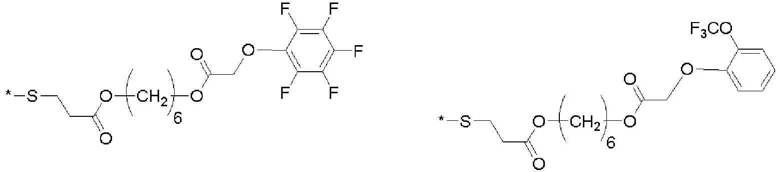

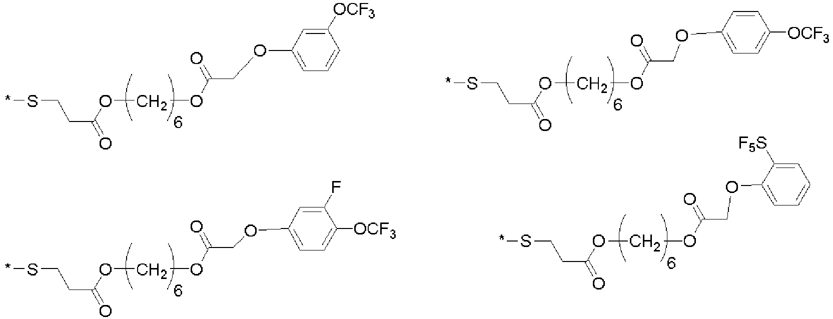

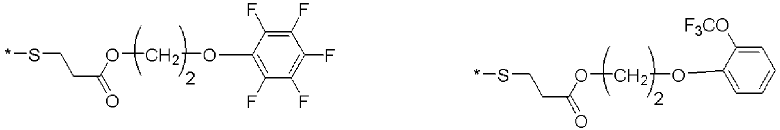

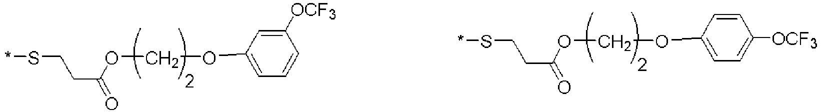

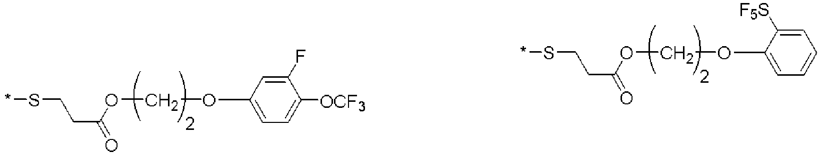

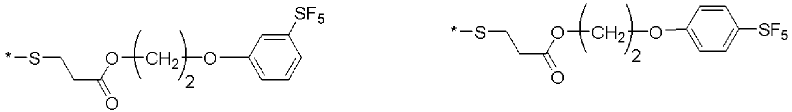

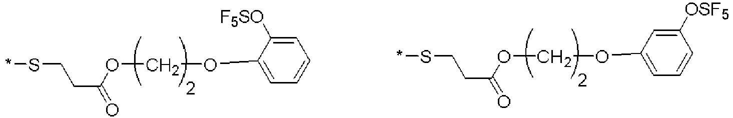

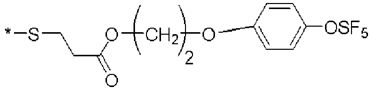

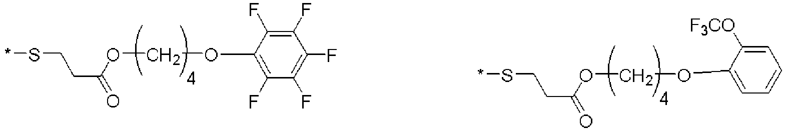

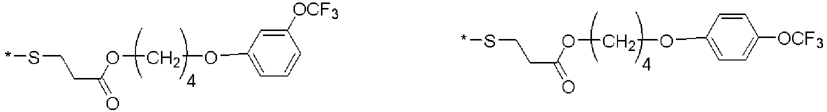

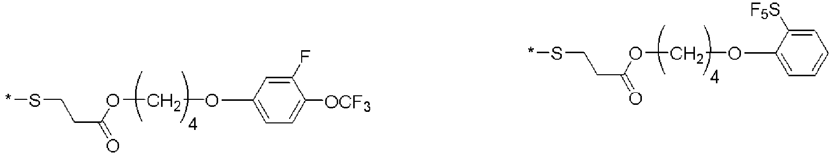

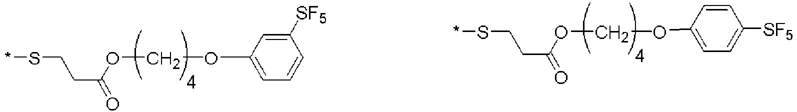

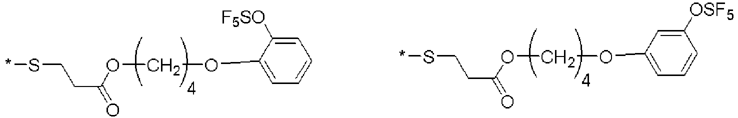

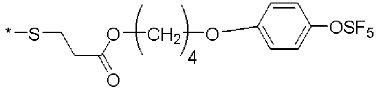

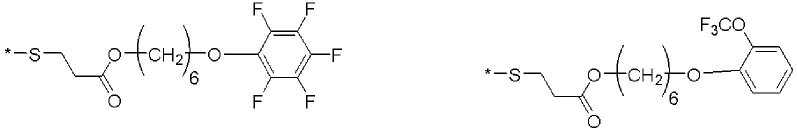

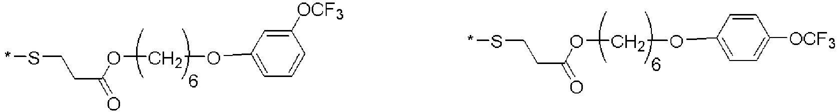

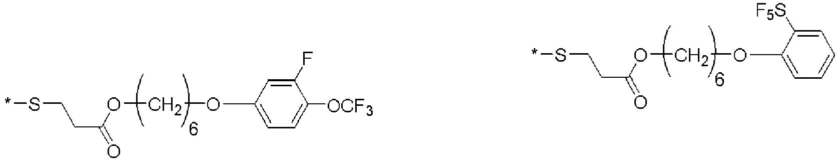

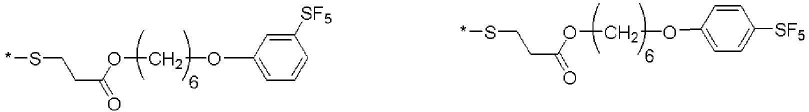

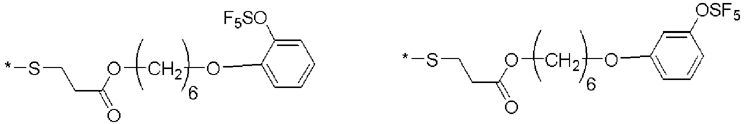

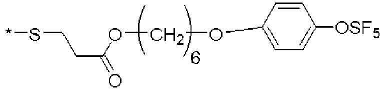

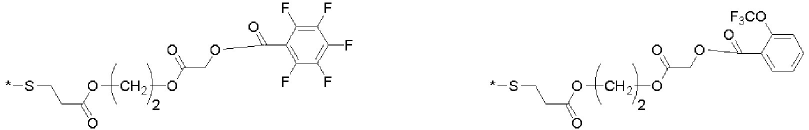

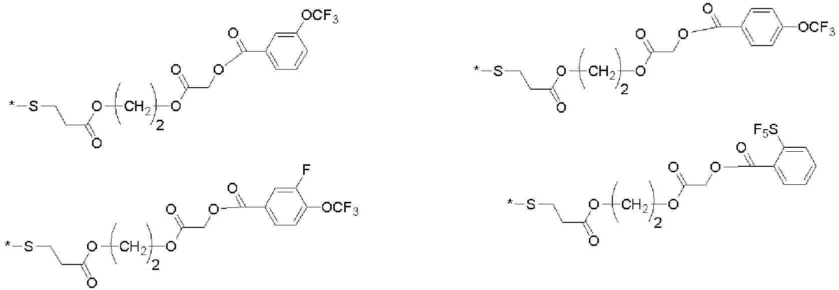

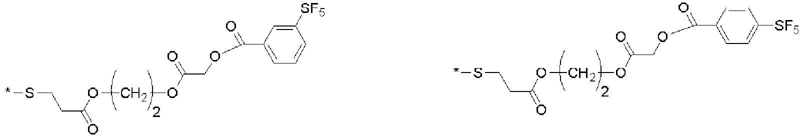

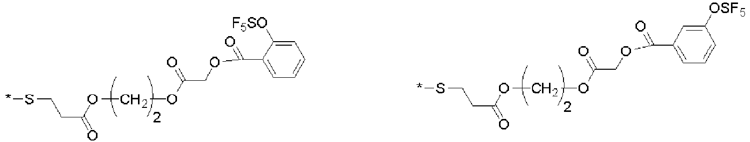

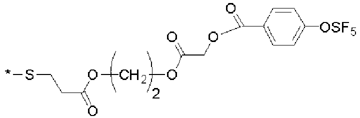

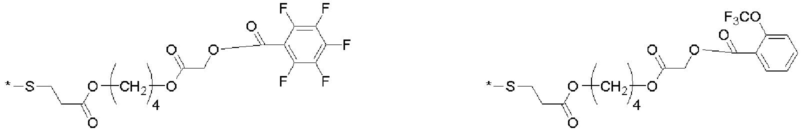

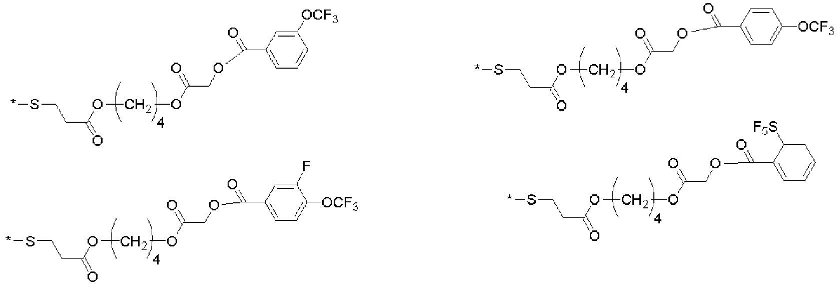

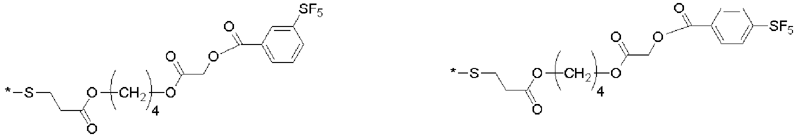

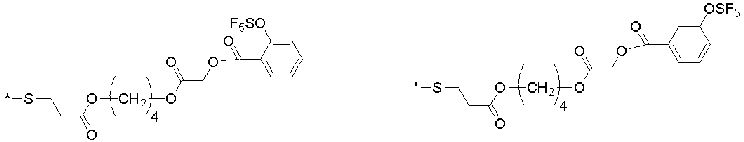

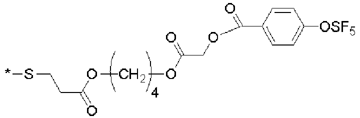

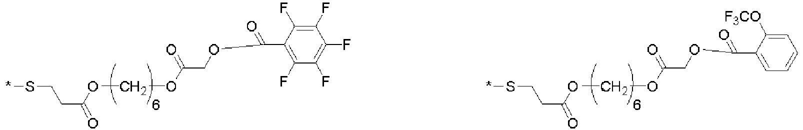

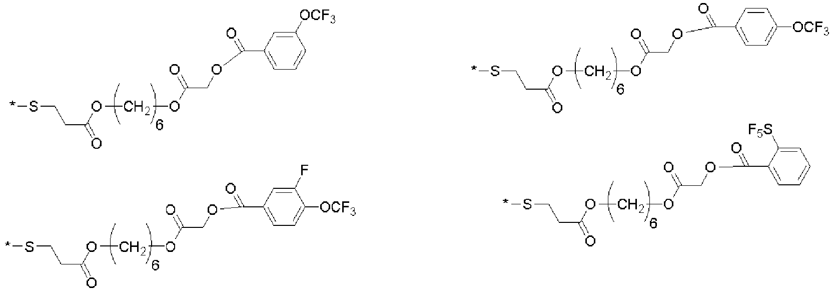

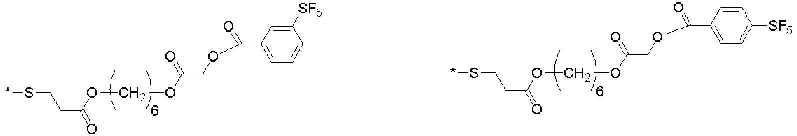

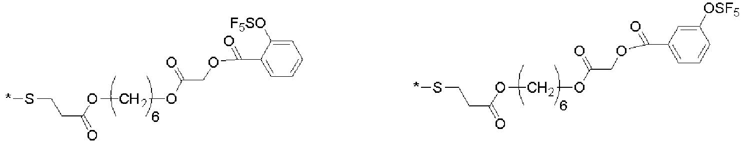

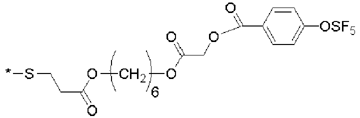

### [Compound: Method for producing terminal structure represented by general formula (7)]

Examples of a method for producing the compound having the terminal structure represented by the general formula (7) include a method in which the compound is obtained by an addition reaction with an acrylate using a base catalyst shown below. 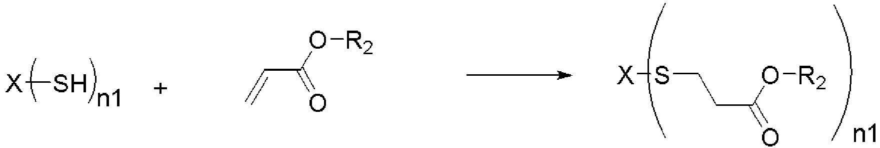 wherein X, R₂, and n1 are as defined above.

The compound can be produced by the method described in the method for producing the terminal structure represented by the general formula (5) except that an acrylate compound corresponding to the terminal structure of the general formula (7) is used instead of the allyl ether compound as a raw material in the reaction. In these reactions, a plurality of types of acrylate compounds can be used at the same time, and it is possible to select acrylate compounds as appropriate in accordance with required performance so that, for example, an acrylate corresponding to the terminal structure of the general formula (7) and an acrylate compound corresponding to the terminal structure of the general formula (8) are combined together.

### [Compound: Terminal structure represented by general formula (8)]

Examples of a terminal structure represented by the general formula (8) include the following. Among these, the terminal structure represented by the general formula (9) is preferably contained. 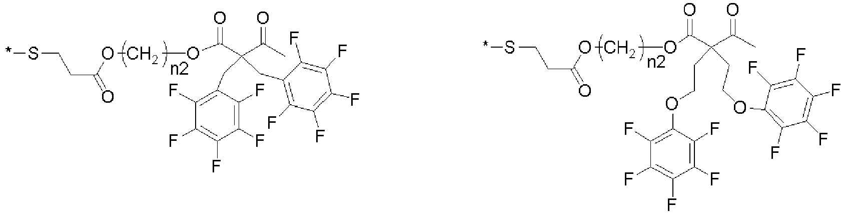 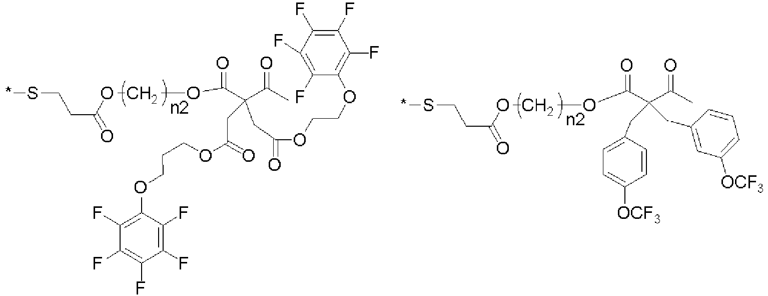 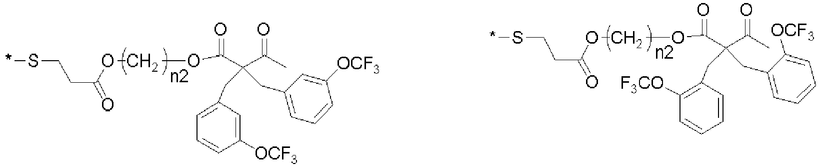 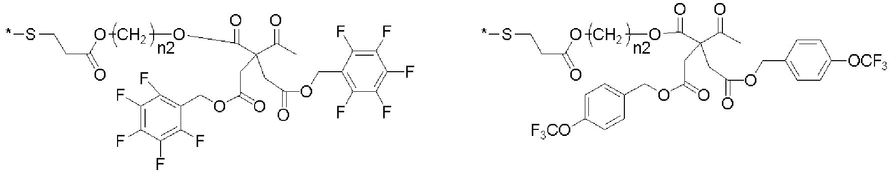 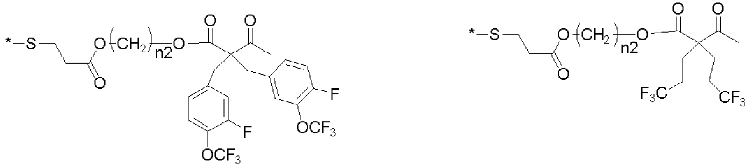 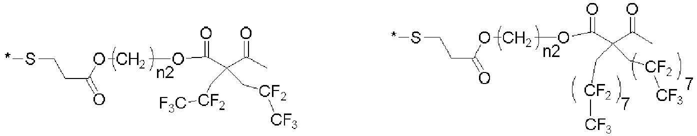 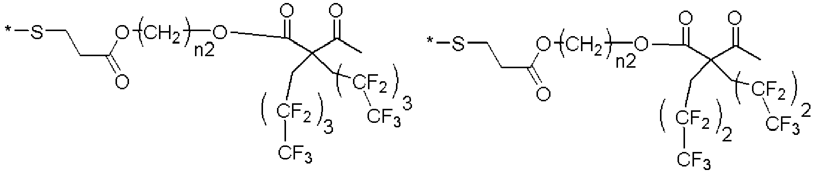 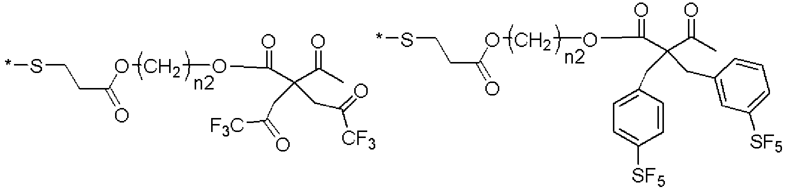 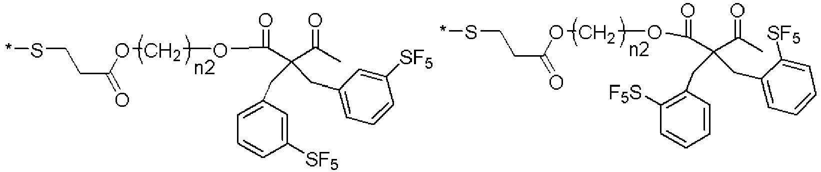 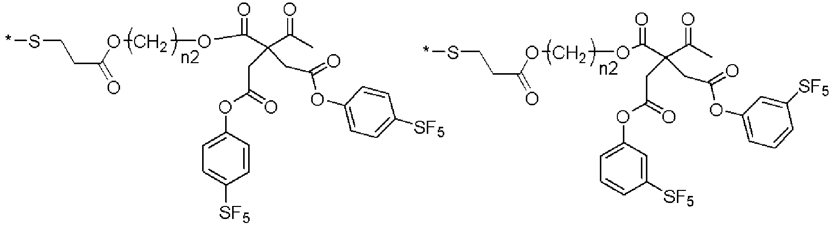 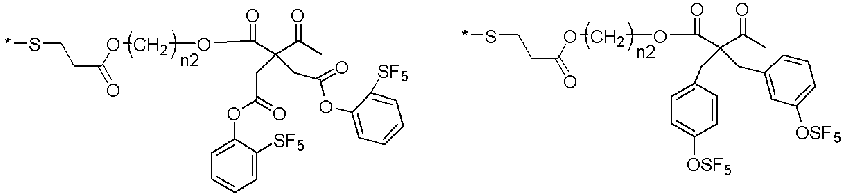 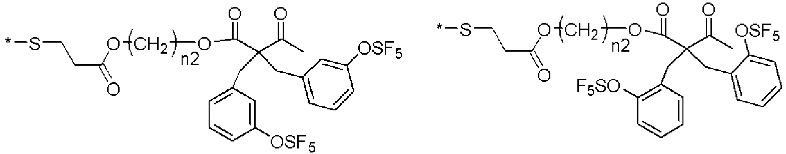 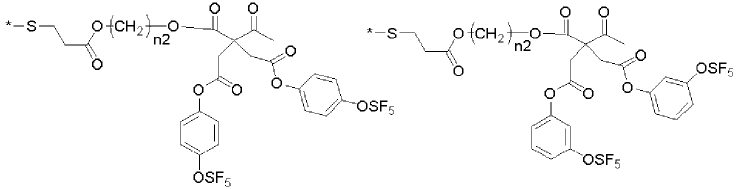 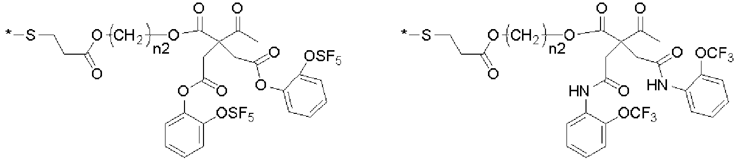 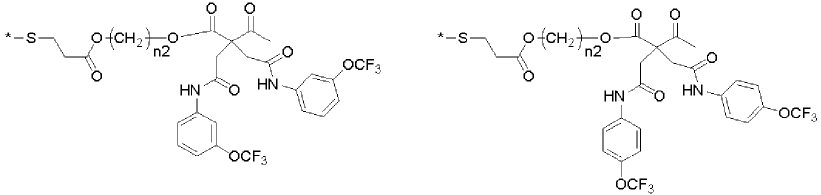 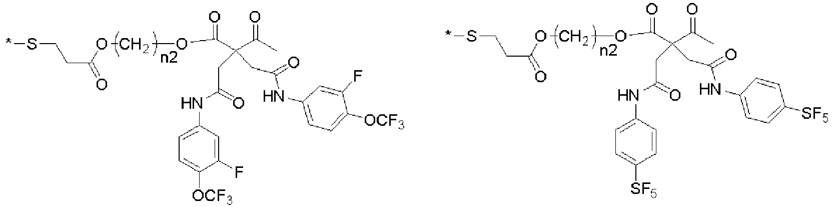 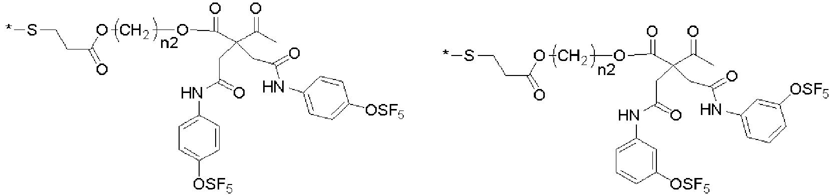 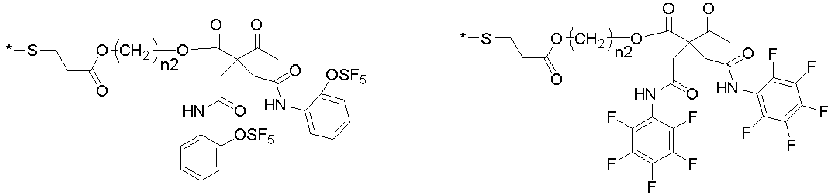 wherein n2 is as defined above.

### [Compound: Method for producing terminal structure represented by general formula (8)]

Examples of a method for producing the compound having the terminal structure represented by the general formula (8) include a method in which the compound is obtained by an addition reaction with an acrylate using a base catalyst shown below. 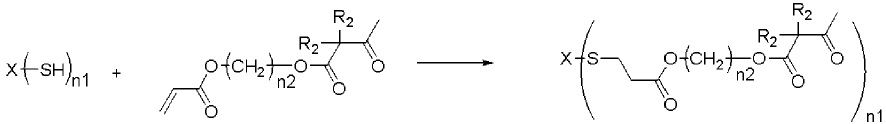 wherein X, R₂, n1, and n2 are as defined above.

The compound can be produced by the method described in the method for producing the terminal structure represented by the general formula (5) except that an acrylate compound corresponding to the terminal structure of the general formula (8) is used instead of the allyl ether compound as a raw material in the reaction. In these reactions, a plurality of types of acrylate compounds can be used at the same time, and it is possible to select acrylate compounds as appropriate in accordance with required performance so that, for example, an acrylate corresponding to the terminal structure of the general formula (7) and an acrylate compound corresponding to the terminal structure of the general formula (8) are combined together.

### [Another method for producing compound having terminal structure represented by general formula (6) or (8)]

In addition, examples of another method for producing a compound having terminal structure represented by the general formula (6) or (8) include a method in which a compound having an acetylacetone structure (R₂ is a structure corresponding to a hydrogen atom) is synthesized by the same method as the method for producing a compound represented by (6) or (8) as described below (Step 1), and the compound is then obtained by an addition reaction with a halide, mesylate, tosylate, or the like having R₂ as a substituent under a base catalyst (Step 2).
[1] Another method for producing compound having terminal structure represented by general formula (6) (Step 1) Addition reaction with allyl ether compound 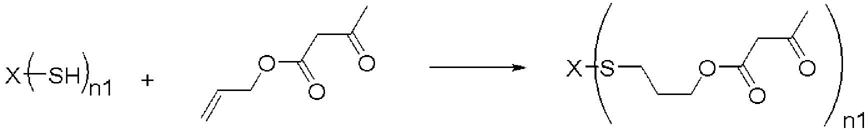 (Step 2) Addition reaction with halide or the like 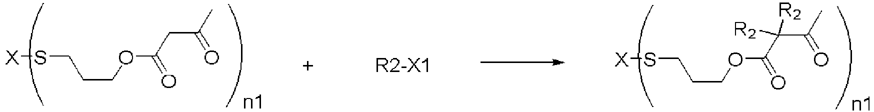
[2] Another method for producing compound having terminal structure represented by general formula (8) (Step 1) Addition reaction with acrylate compound 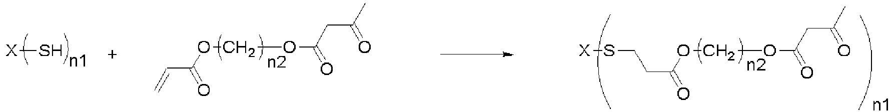 (Step 2) Addition reaction with halide or the like 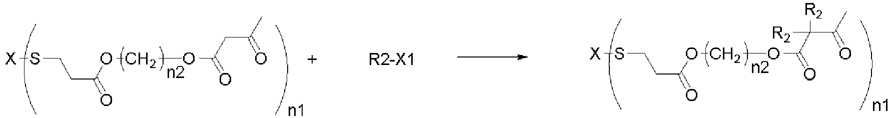 wherein X1 represents a halide, mesylate, or tosylate, and X, R₂, n1, and n2 are as defined above.

In the case of using the above-described production method, when the reaction in STEP 2 is performed using a halide or the like having a single R₂ structure, it is possible to produce a compound to which only R₂ has been introduced as a substituent in advance, like the allyl ether compound or the acrylic compound to be reacted described in the previous sections; however, when a plurality of R₂'s is used, obtained is a mixture of compounds having a plurality of terminal groups, as described below. Conversely, in a reaction product with the allyl ether compound or acrylic compound to which a plurality of R₂'s has been introduced in advance described in the previous sections, a plurality of structures is not introduced as the same substituent on the acetylacetone except that the terminal structure is changed, and a compound only different in the terminal structure is generated. At this time, it is possible to control the reaction rate with respect to the whole reaction sites by adjusting the overall charging ratio, and a combination of substituents can be used for a plurality of or single R₂ in accordance with required performance.
(1) Another method: Product when synthesized using halide or the like having plurality of R₂'s as substituent. 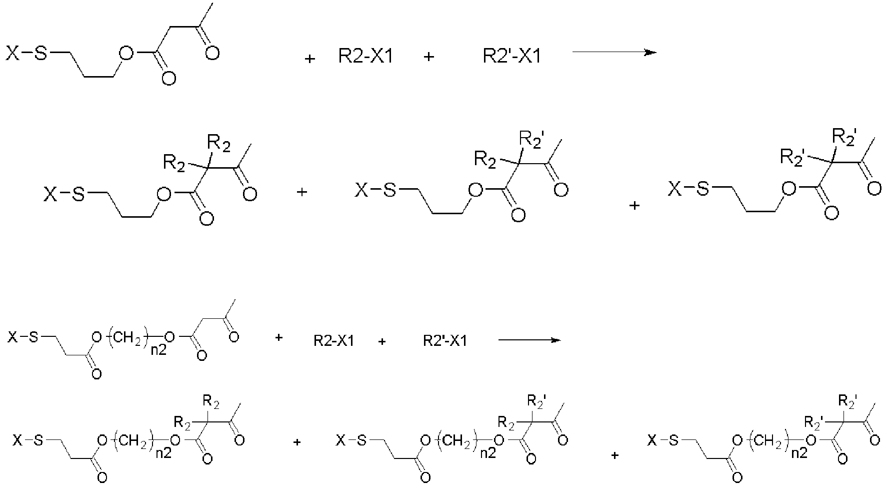
(2) Method described in previous section: Product when synthesized using allyl ether compound or acrylic compound having plurality of R₂'s as substituent 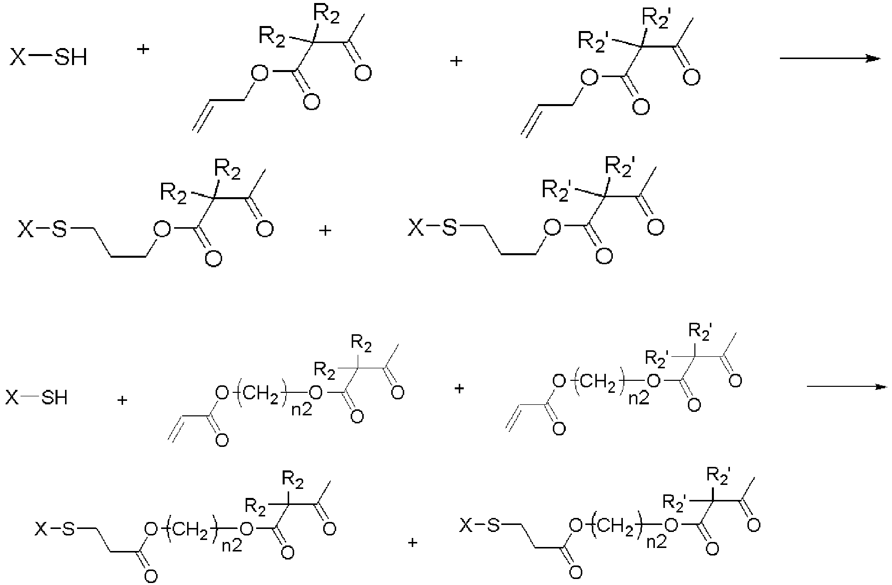 wherein X, X1, R₂, and n2 are as defined above, R₂' is a substituent other than R₂ satisfying R₂' ≠ R₂. What has been described above is about a compound that is generated at the time of using two kinds of halides (R₂ - X1 and R₂' - X1) (the case of n1 = 1 for the sake of convenience for easy understanding), and in the case of n1 ≥ 2, a combination of a substituent further increases, which makes it more complex.

As a method for obtaining the compound, the compound can be produced by the method described in the method for producing the terminal structure represented by the general formula (4) except that, regarding the reaction in Step 1, the addition reaction with the allyl ether and the addition reaction with the acrylic compound are changed to reactions corresponding to the compound that is obtained in Step 1. The compound that is obtained in Step 1 can be used in the addition reaction in Step 2.

Regarding Step 2, the compound can be produced by the same method except that a substrate in the method for producing the terminal structure represented by the general formula (4) is changed to a compound represented by R₂ - X1.

The weight-average molecular weight of the (B) compound is preferably 300 to 4000. When the weight-average molecular weight is 300 or more, it is possible to suppress reduction in the blending effect due to volatilization or the like, and a sufficient blending effect can be obtained. In addition, when the weight-average molecular weight is 4000 or less, it is possible to suppress the (B) compound remaining in the film, and an organic film having a superior process tolerance can be formed. Furthermore, the weight-average molecular weight is preferably 3500 or less. When the weight-average molecular weight is 3500 or less, it is possible to further suppress the risk of the surfactant remaining in the film. In addition, the weight-average molecular weight is preferably 500 or more. When the weight-average molecular weight is 500 or more, the compound can remain in the film without evaporation during film formation, and can exhibit a sufficient surfactant effect.

The ratio Mw/Mn of the weight-average molecular weight Mw to the number-average molecular weight Mn of the (B) compound in terms of polystyrene by gel permeation chromatography is preferably 1.00 ≤ Mw/Mn ≤ 1.20.

Usually, a single compound theoretically has Mw/Mn of 1.00. However, it is difficult even for a single compound to have Mw/Mn of exactly 1.00, for example, in view of the fact that the compound (B) included in the composition for forming an organic film of the present invention may have a combination of a plurality of terminal structures R1 or a plurality of substituents R2 of the terminal structure, and a combination of a plurality of types results in a complicated mixture, as described in another method for producing the same; and the fact that in the gel permeation chromatography method, compound properties such as the polarity of the compound or errors in measurement conditions, such as the temperature or pressure of the column affect the separation performance of the compound. Therefore, the above-described range is defined as an index that indicates monomolecular properties for distinction from a compound having a distribution in the ratio Mw/Mn.

When the compound obtained in these methods and used in the composition for forming an organic film is produced, a plurality of R₂ structures and the introduction ratio thereof can be controlled and adjusted in accordance with required performance, and the method for producing the compound can be rearranged in accordance with required performance. For example, it is possible to arbitrarily combine a side chain structure contributing to improvement in flattening characteristics or a structure having a fluorine-containing substituent or the like for changing the surfactant capability by controlling surface tension, or the like. Therefore, it becomes possible for these compounds to satisfy not only the surfactant effect that contributes to excellent film formability improvement but also a variety of performances such as film formability and embedding characteristics when the compound is used in a lower layer film as the composition for forming an organic film on a high level.

### [Composition for forming organic film]

The composition for forming an organic film contains (A) a resin or compound for forming an organic film, the (B) compound described above, and (C) a solvent.

In the composition for forming an organic film of the present invention, the (B) compound, the (A) resin or compound for forming an organic film, and the (C) solvent can be each used singly or two or more thereof can be used in combination.

The (B) compound of the present invention functions as a surfactant that imparts excellent film formability and high leveling performance. The use thereof is not limited to an organic lower layer film, the (B) compound can be generally used in coating materials for photolithography, and examples thereof include photosensitive resist materials, and top coat-forming materials that are formed on resist films. Furthermore, the (B) compound can also be applied not only to organic film-forming materials but also to silicon-containing resist middle layer films, and can be used as a surfactant suitable for developing highly versatile film formability that can be also applied to a variety of film-forming materials.

Furthermore, the (B) compound of the present invention can be used singly or two or more thereof can be used in combination. Regarding the amount of these compounds added, the content of the (B) compound is preferably 0.01 parts by mass to 5 parts by mass based on 100 parts by mass of the (A) resin or compound for forming an organic film.

### [(A) Resin or compound for forming organic film]

The (A) resin or compound for forming an organic film for use in the composition for forming an organic film of the present invention is not particularly limited as long as the resin or compound satisfies the film formability in spin coating and curability, and a resin or compound containing an aromatic skeleton is more preferable from the viewpoint of etching resistance, optical characteristics, and heat resistance.

Examples of the aromatic skeleton include benzene, naphthalene, anthracene, pyrene, indene, fluorene, furan, pyrrole, thiophene, phosphole, pyrazole, oxazole, isoxazole, thiazole, pyridine, pyrazine, pyrimidine, pyridazine, triazine, and carbazole. Among these, benzene, naphthalene, fluorene, and carbazole are particularly preferable.

Examples of the (A) resin or compound for forming an organic film for use in the present invention include resins containing the following structures described in JP 2012-001687 A and JP 2012-077295 A: 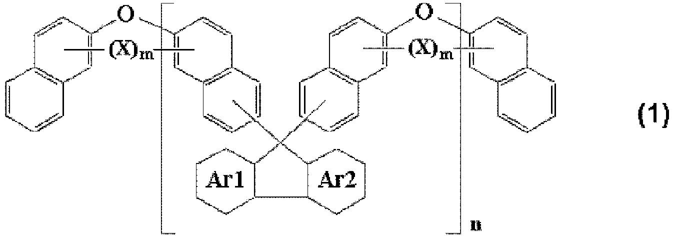
wherein ring structures Ar1 and Ar2 represent benzene rings or naphthalene rings; X represents a single bond or an alkylene group having 1 to 20 carbon atoms; m represents 0 or 1; and "n" represents any natural number that provides a molecular weight of 100,000 or less; wherein the symbols in the formula are applied only to this formula, 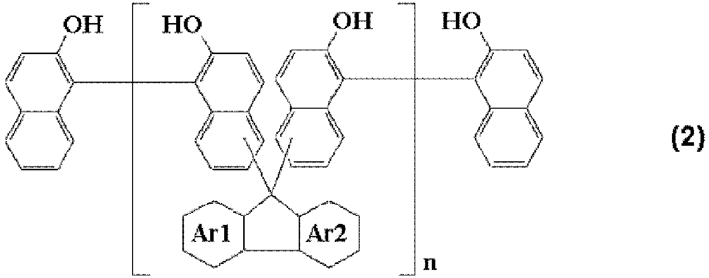
wherein ring structures Ar1 and Ar2 represent benzene rings or naphthalene rings; and "n" represents any natural number that provides a polystyrene-equivalent weight-average molecular weight of 100,000 or less as determined by gel permeation chromatography; wherein the symbols in the formula are applied only to this formula.

Examples of the (A) resin or compound for forming an organic film for use in the present invention include resins containing the following structures described in JP 2004-264710 A, JP 2005-043471 A, JP 2005-250434 A, JP 2007-293294 A, and JP 2008-065303 A: 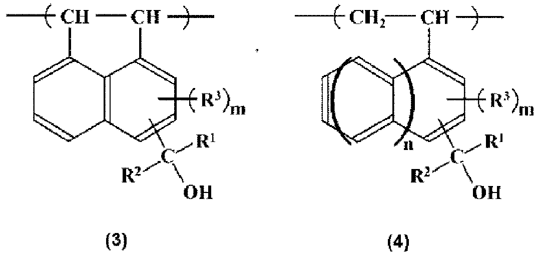
wherein R¹ and R² represent hydrogen atoms, or alkyl groups having 1 to 3 carbon atoms, or aryl groups; R³ represents an alkyl group having 1 to 3 carbon atoms, a vinyl group, an allyl group, or an optionally substituted aryl group; "n" represents 0 or 1; and "m" represents 0, 1, or 2; wherein the symbols in the formulae are applied only to these formulae, 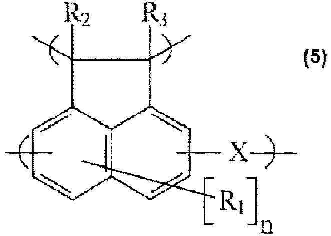
wherein R₁ is a monovalent atom or group other than a hydrogen atom; n is an integer of 0 to 4, provided that when n is 2 to 4, the plurality of R₁ may be the same as or different from each other; R₂ and R₃ are independently monovalent atoms or groups; and X is a divalent group; wherein the symbols in the formula are applied only to this formula, 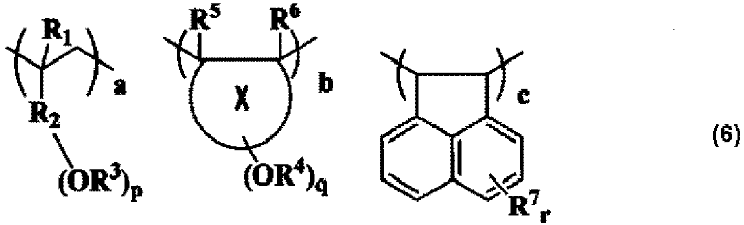
wherein R₁ is a hydrogen atom or a methyl group; R₂ is any of a single bond, a linear, branched, or cyclic alkylene group having 1 to 20 carbon atoms, or an arylene group having 6 to 10 carbon atoms, each of which optionally has any of an ether, an ester, a lactone, and an amide; R³ and R⁴ are each a hydrogen atom or a glycidyl group; X represents any polymer of a hydrocarbon having an indene skeleton, a cycloolefin having 3 to 10 carbon atoms, and maleimide, each of which optionally has any of an ether, an ester, a lactone, and a carboxylic anhydride; R⁵ and R⁶ are each a hydrogen atom, a fluorine atom, a methyl group, and a trifluoromethyl group; R⁷ is any of a hydrogen atom, a linear, branched, or cyclic alkyl group having 1 to 6 carbon atoms, hydroxy group, or alkoxycarbonyl group; "p" and "q" are each an integer of 1 to 4; "r" is an integer of 0 to 4; and "a", "b", and "c" are within ranges of 0.5 ≤ a + b + c ≤ 1, 0 ≤ a ≤ 0.8, 0 ≤ b ≤ 0.8, 0.1 ≤ a + b ≤ 0.8, 0.1 ≤ c ≤ 0.8; wherein the symbols in the formula are applied only to this formula. 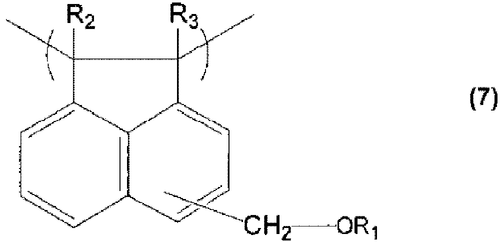
wherein R₁ represents a hydrogen atom or a monovalent organic group; and R₂ and R₃ independently of each other represent monovalent atoms or monovalent organic groups; wherein the symbols in the formula are applied only to this formula.

Specific examples of the (A) resin or compound for forming an organic film for use in the present invention include resins containing the following structures described in JP 2004-205685 A, JP 2007-171895 A, and JP 2009-014816 A: 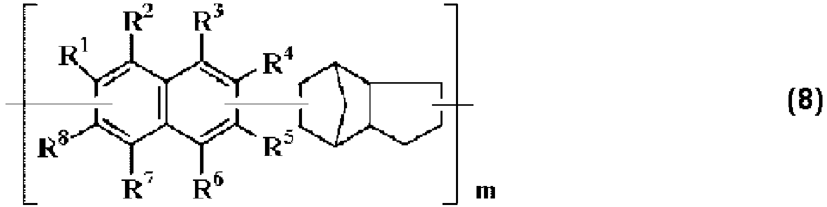 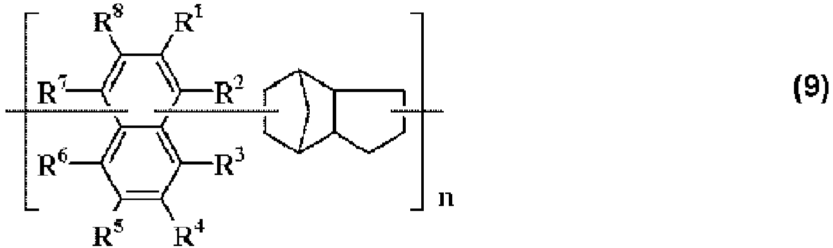
wherein R¹ to R⁸ are independently of each other hydrogen atoms, hydroxy groups, optionally substituted alkyl groups having 1 to 6 carbon atoms, optionally substituted alkoxy groups having 1 to 6 carbon atoms, optionally substituted alkoxycarboxyl groups having 2 to 6 carbon atoms, optionally substituted aryl groups having 6 to 10 carbon atoms, hydroxyalkyl groups having 1 to 6 carbon atoms, isocyanate groups, or glycidyl groups; and "m" and "n" are positive integers; wherein the symbols in the formula are applied only to this formula, 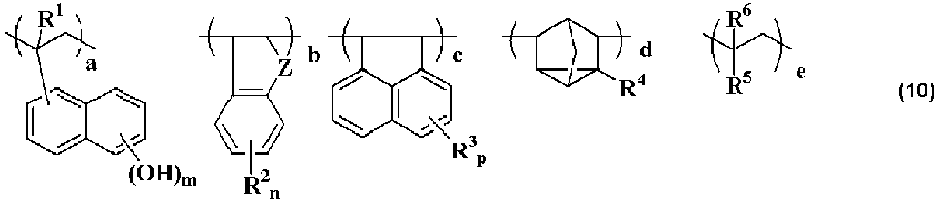
wherein R¹ and R⁶ are hydrogen atoms or methyl groups; R², R³ and R⁴ are hydrogen atoms, alkyl groups, alkoxy groups, hydroxy groups, acetoxy groups, or alkoxycarbonyl groups having 1 to 4 carbon atoms, or aryl groups having 6 to 10 carbon atoms; R⁵ is a polycyclic condensed hydrocarbon group having 13 to 30 carbon atoms, -O-R⁷, -C(=O)-O-R⁷, -O-C(=O)-R⁷, or -C(=O)-NR⁸-N⁷, m is 1 or 2, n is an integer of 0 to 4, and p is an integer of 0 to 6; R⁷ is an organic group having 7 to 30 carbon atoms; R⁸ is a hydrogen atom or a hydrocarbon group having 1 to 6 carbon atoms; Z is any of a methylene group, -O-, -S-, and -NH-; and "a", "b", "c", "d", and "e" are within ranges of 0 < a < 1.0, 0 ≤ b ≤ 0.8, 0 ≤ c ≤ 0.8, 0 ≤ d ≤ 0.8, 0 ≤ e ≤ 0.8, 0 < b + c + d + e < 1.0; wherein the symbols in the formula are applied only to this formula, 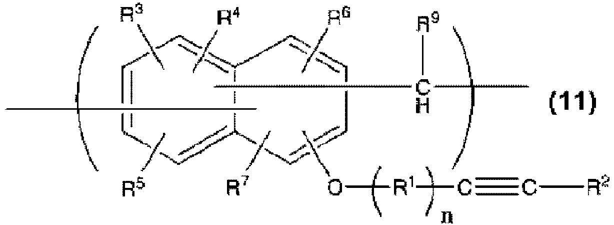
wherein "n" represents 0 or 1; R¹ represents an optionally substituted methylene group, an optionally substituted alkylene group having 2 to 20 carbon atoms, or an optionally substituted arylene group having 6 to 20 carbon atoms; R² represents a hydrogen atom, an optionally substituted alkyl group having 1 to 20 carbon atoms, or an optionally substituted aryl group having 6 to 20 carbon atoms; R³ to R⁷ represent hydroxy groups, optionally substituted alkyl groups having 1 to 6 carbon atoms, optionally substituted alkoxy groups having 1 to 6 carbon atoms, optionally substituted alkoxycarbonyl groups having 2 to 10 carbon atoms, optionally substituted aryl groups having 6 to 14 carbon atoms, or optionally substituted glycidyl ether groups having 2 to 6 carbon atoms; and R⁹ represents a hydrogen atom, a linear, branched, or cyclic alkyl group having 1 to 10 carbon atoms, a linear, branched, or cyclic alkyl ether group having 1 to 10 carbon atoms, or an aryl group having 6 to 10 carbon atoms; wherein the symbols in the formula are applied only to this formula.

Examples of the formula (11) include the following resins:

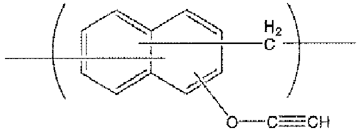

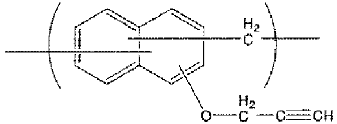

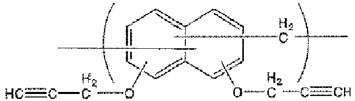

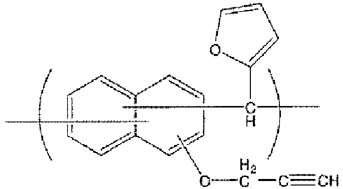

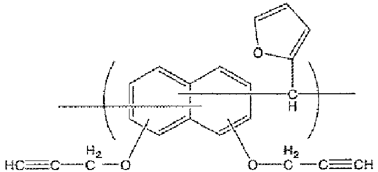

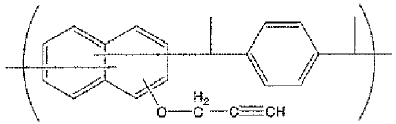

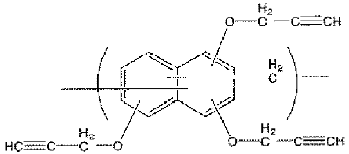

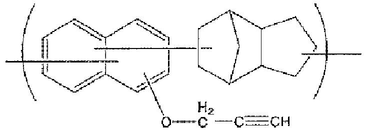

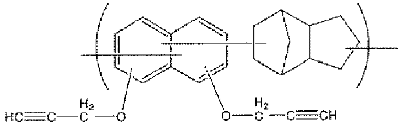

Examples of the (A) resin or compound for forming an organic film for use in the present invention include resins containing the following structures described in JP 2007-199653 A, JP 2008-274250 A, and JP 2010-122656 A: 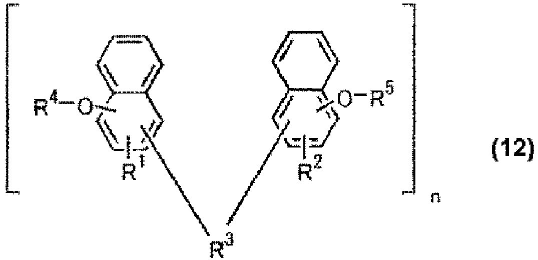
wherein R¹ and R² are independently the same as or different from each other and are hydrogen atoms, linear, branched, or cyclic alkyl groups having 1 to 10 carbon atoms, aryl groups having 6 to 10 carbon atoms, or alkenyl groups having 2 to 10 carbon atoms; R³ is a single bond or an alkylene group having 1 to 30 carbon atoms and having a linear, branched, or cyclic structure, which optionally has a bridged cyclic hydrocarbon group, a double bond, a heteroatom, or an aromatic group having 6 to 30 carbon atoms; R⁴ and R⁵ are each independently a hydrogen atom or a glycidyl group; and "n" is an integer of 1 to 4; wherein the symbols in the formula are applied only to this formula. 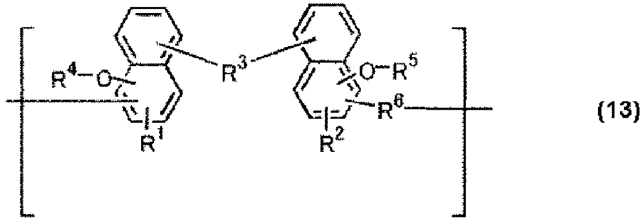
wherein R¹ and R² are independently the same as or different from each other and are hydrogen atoms, linear, branched, or cyclic alkyl groups having 1 to 10 carbon atoms, aryl groups having 6 to 10 carbon atoms, or alkenyl groups having 2 to 10 carbon atoms; R³ is a single bond or an alkylene group having a linear, branched, or cyclic structure having 1 to 30 carbon atoms, which optionally has a bridged cyclic hydrocarbon group, a double bond, a heteroatom, or an aromatic group having 6 to 30 carbon atoms; R⁴ and R⁵ are each independently a hydrogen atom or a glycidyl group; and R⁶ is a single bond or a linear or branched alkylene group having 1 to 10 carbon atoms; wherein the symbols in the formula are applied only to this formula.) 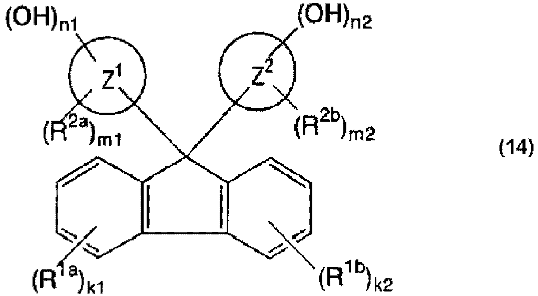
wherein a ring Z¹ and a ring Z² are polycyclic condensed aromatic hydrocarbon rings; and R^{1a}, R^{1b}, R^{2a}, and R^{2b} are the same as or different from each other and represent substituents; "k1" and "k2" are the same as or different from each other and represent an integer of 0 or 1 to 4; "m1" and "m2" each represent an integer of 0 or 1 or more; and "n1" and "n2" each represent an integer of 0 or 1 or more, provided that n1 + n2 ≥ 1; wherein the symbols in the formula are applied only to this formula.) 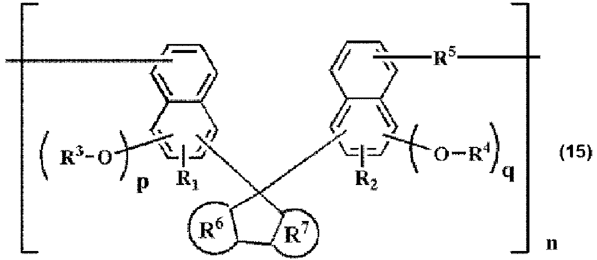
wherein R¹ and R² are the same as or different from each other and are hydrogen atoms, linear, branched, or cyclic alkyl groups having 1 to 10 carbon atoms, aryl groups having 6 to 10 carbon atoms, or alkenyl groups having 2 to 10 carbon atoms; R³ and R⁴ are each a hydrogen atom or a glycidyl group; R⁵ is a single bond or a linear or branched alkylene group having 1 to 10 carbon atoms; R⁶ and R⁷ are benzene rings or naphthalene rings; "p" and "q" are each 1 or 2; and "n" satisfies 0 < n ≤ 1; wherein the symbols in the formula are applied only to this formula.

Examples of the formula (15) include the following resins:

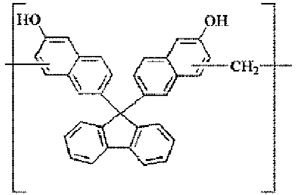

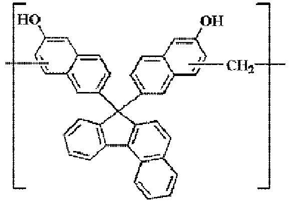

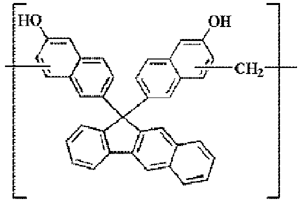

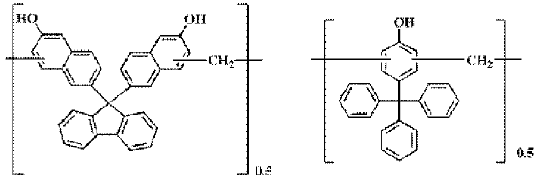

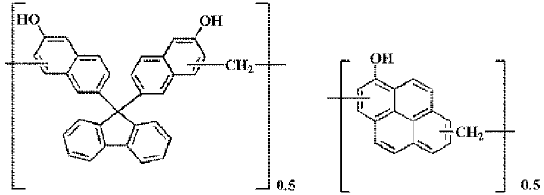

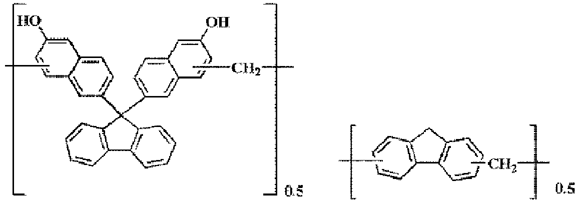

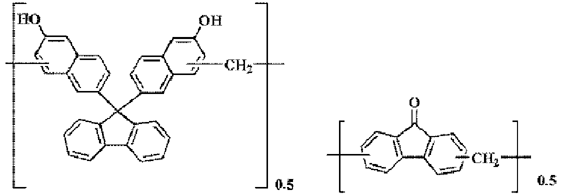

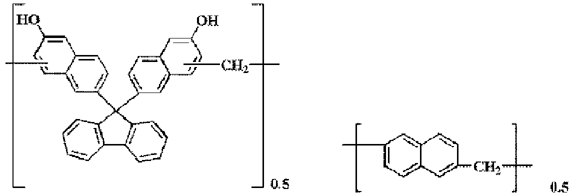

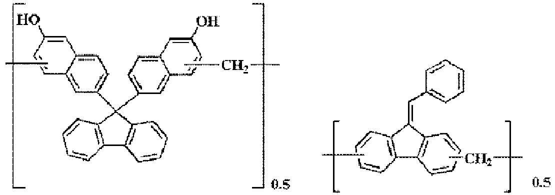

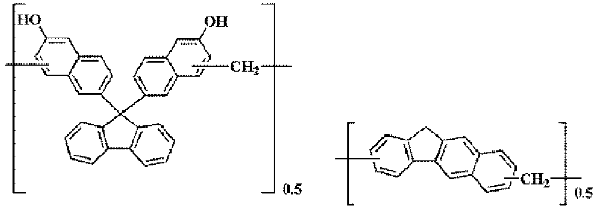

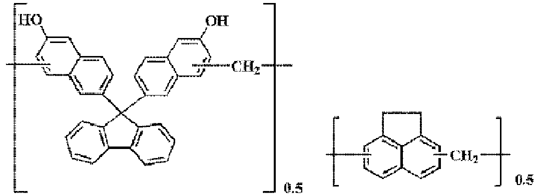

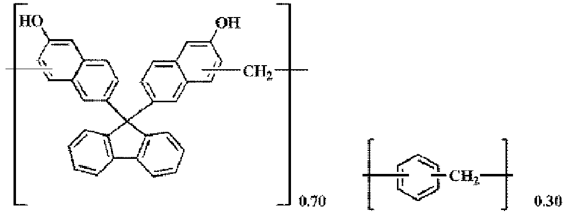

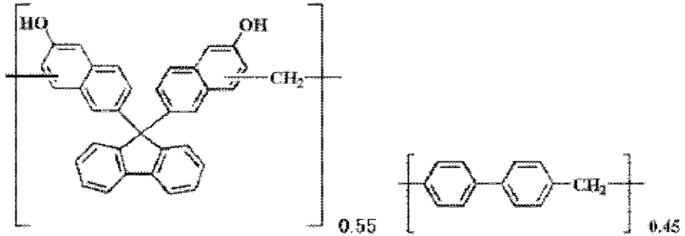

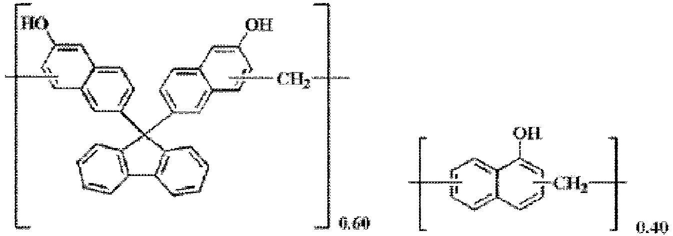

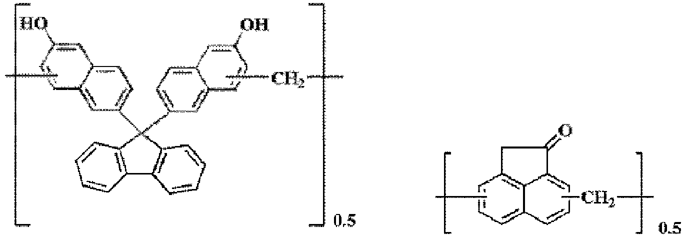

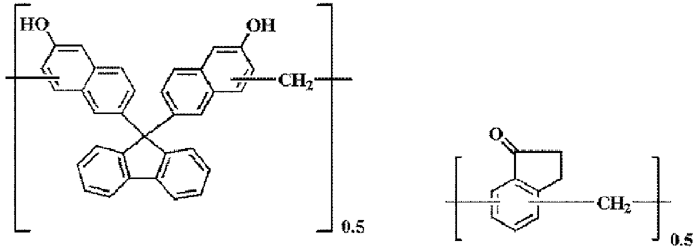

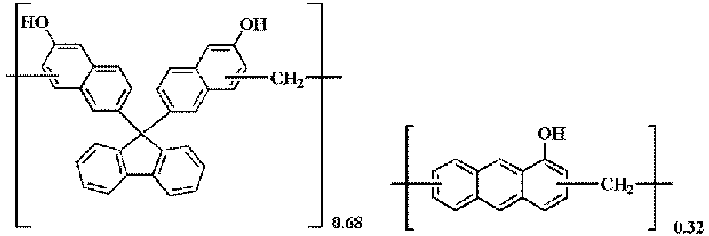

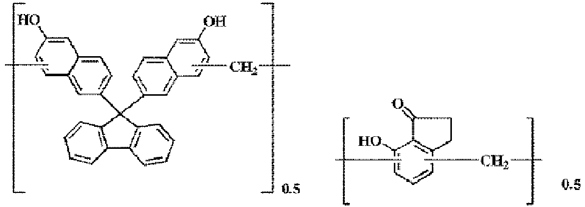

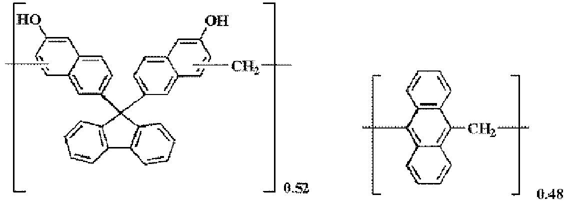

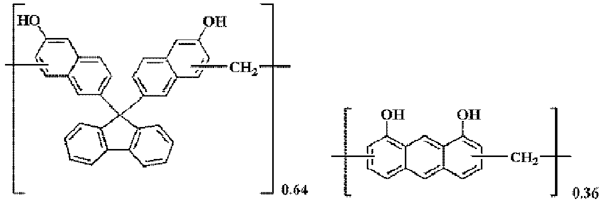

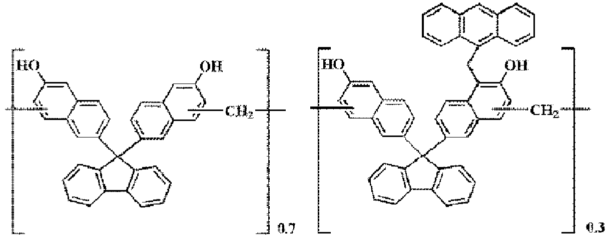

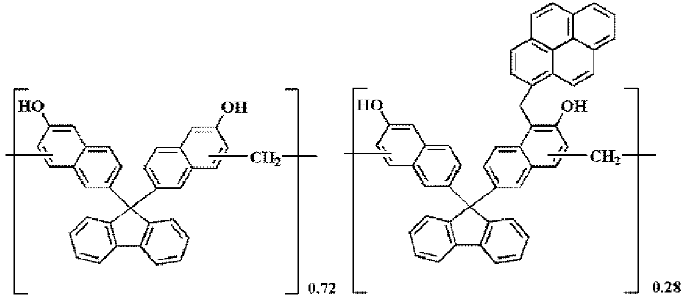

Examples of the (A) resin or compound for forming an organic film for use in the present invention include resins containing the following structures described in JP 2012-214720 A: 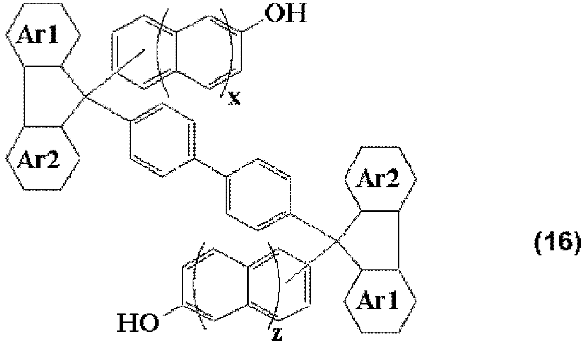 wherein ring structures Ar1 and Ar2 are benzene rings or naphthalene rings; and "x" and "z" each independently represent 0 or 1; wherein the symbols in the formula are applied only to this formula.

Examples of the (A) resin or compound for forming an organic film for use in the present invention include resins described in JP 2014-29435 A: 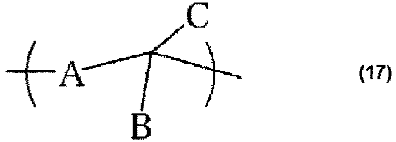 wherein A represents a structure having carbazole; B represents a structure having an aromatic ring; and C represents a structure having a hydrogen atom, an alkyl group, or an aromatic ring, wherein B and C optionally together form a ring; a structure formed by combining A, B, and C together has one to four carboxyl groups, a salt thereof, or a carboxylic acid ester group; wherein the symbols in the formula are applied only to this formula.)

In addition, examples of the (A) resin or compound for forming an organic film for use in the present invention include polymers including a unit structure represented by the following formula (18) and a unit structure represented by the following formula (19) described in WO 2012/077640 A1, in which the ratio between the unit structure represented by the formula (18) and the unit structure represented by the formula (19) is from 3:97 to 97:3 in terms of molar ratio: 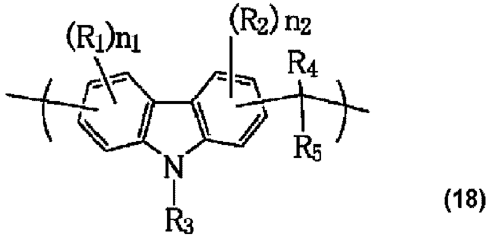
wherein R₁ and R₂ each independently represent a hydrogen atom, a halogen atom, a nitro group, an amino group, a hydroxy group, an alkyl group having 1 to 10 carbon atoms, an alkenyl group having 2 to 10 carbon atoms, an aryl group having 6 to 40 carbon atoms, or a combination of those groups that may contain an ether bond, a ketone bond, or an ester bond; R₃ represents a hydrogen atom, an alkyl group having 1 to 10 carbon atoms, an alkenyl group having 2 to 10 carbon atoms, an aryl group having 6 to 40 carbon atoms, or a combination of those groups that may contain an ether bond, a ketone bond, or an ester bond; R₄ represents a hydrogen atom, or an aryl group having 6 to 40 carbon atoms or a heterocyclic group, optionally substituted with a halogen atom, a nitro group, an amino group, or a hydroxy group; R₅ represents a hydrogen atom, or an alkyl group having 1 to 10 carbon atoms, an aryl group having 6 to 40 carbon atoms, or a heterocyclic group, optionally substituted with a halogen atom, a nitro group, an amino group, or a hydroxy group, and R₄ and R₅ optionally together form a ring; and "n1" and "n2" each represent an integer of 1 to 3; wherein the symbols in the formula are applied only to this formula, 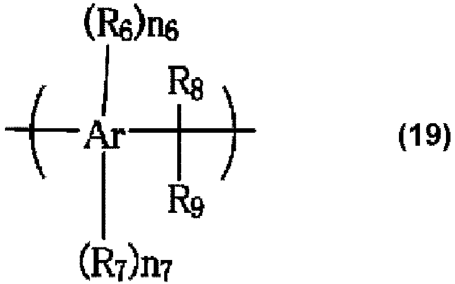
wherein Ar represents an aromatic ring group having 6 to 20 carbon atoms; R₆ represents a hydroxy group; and R₇ represents a hydrogen atom, a halogen atom, a nitro group, an amino group, an alkyl group having 1 to 10 carbon atoms, an alkenyl group having 2 to 10 carbon atoms, an aryl group having 6 to 40 carbon atoms, or a combination of those groups optionally containing an ether bond, a ketone bond, or an ester bond; R₈ represents a hydrogen atom, or an aryl group having 6 to 40 carbon atoms or a heterocyclic group, optionally substituted with a halogen atom, a nitro group, an amino group, or a hydroxy group; R₉ represents a hydrogen atom, or an alkyl group having 1 to 10 carbon atoms, an aryl group having 6 to 40 carbon atoms, or a heterocyclic group, optionally substituted with a halogen atom, a nitro group, an amino group, or a hydroxy group, and R₈ and R₉ optionally together form a ring; "n6" represents an integer of 1 to p; and "n7" represents an integer of p - n6, where "p" represents the maximum number of substituents that can be in the aromatic ring group Ar; wherein the symbols in the formula are applied only to this formula.)

Examples of the (A) resin or compound for forming an organic film for use in the present invention include polymers containing a unit structure represented by the following formula (20) described in WO 2010/147155 A1: 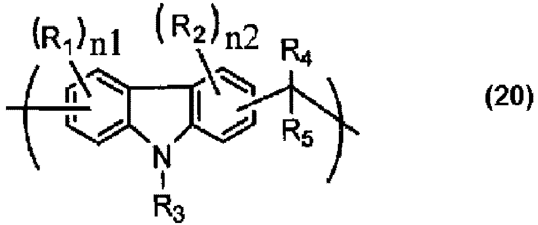 wherein R₁ and R₂ each represent a group selected from the group consisting of a hydrogen atom, a halogen group, a nitro group, an amino group, a hydroxy group, an alkyl group having 1 to 10 carbon atoms, an alkenyl group having 2 to 10 carbon atoms, an aryl group having 6 to 40 carbon atoms, and combinations thereof, wherein the alkyl group, the alkenyl group, or the aryl group optionally contains an ether bond, a ketone bond, or an ester bond; R₃ represents a group selected from the group consisting of a hydrogen atom, an alkyl group having 1 to 10 carbon atoms, an alkenyl group having 2 to 10 carbon atoms, an aryl group having 6 to 40 carbon atoms, and combinations thereof, wherein the alkyl group, the alkenyl group, or the aryl group may contain an ether bond, a ketone bond, or an ester bond; R₄ represents an aryl group having 6 to 40 carbon atoms or a heterocyclic group, optionally substituted with a halogen group, a nitro group, an amino group, or a hydroxy group; R₅ represents a hydrogen atom, or an alkyl group having 1 to 10 carbon atoms, an aryl group having 6 to 40 carbon atoms, or a heterocyclic group, optionally substituted with a halogen group, a nitro group, an amino group, or a hydroxy group, and R₄ and R₅ optionally form a ring together with a carbon atom to which they are bonded; and "n1" and "n2" are each an integer of 1 to 3; wherein the symbols in the formula are applied only to this formula.

Examples of the (A) resin or compound for forming an organic film for use in the present invention include novolac resins obtained by reacting one or more of phenols such as phenol, cresol, xylenol, catechol, resorcinol, hydroquinone, pyrogallol, hydroxyquinol, and phloroglucinol and one or more of aldehyde sources such as formaldehyde, paraformaldehyde, and trioxane using an acidic catalyst, and resins containing a repeating unit structure represented by the following formula (21) described in WO 2012/176767 A1: 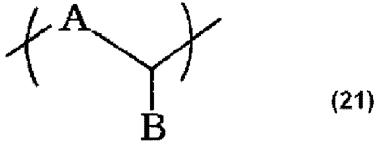 wherein A represents a hydroxy group-substituted phenylene group derived from polyhydroxybenzene; and B represents a monovalent condensed aromatic hydrocarbon ring group in which two to six benzene rings are condensed together; wherein the symbols in the formula are applied only to this formula.

Examples of the (A) resin or compound for forming an organic film for use in the present invention include resins that are novolac resins having a fluorene or tetrahydrospirobiindene structure described in JP 2005-128509 A, JP 2006-259249 A, JP 2006-259482 A, JP 2006-293298 A, and JP 2007-316282 A and containing a repeating unit structure represented by the following formula (22-1) or (22-2): 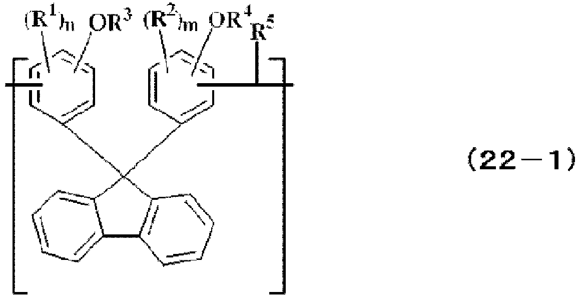 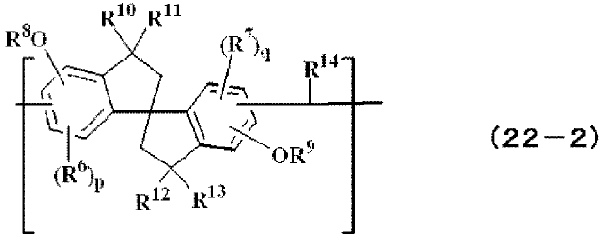 wherein R¹, R², R⁶, and R⁷ are independently a hydrogen atom, a linear, branched, or cyclic alkyl group having 1 to 10 carbon atoms, an aryl group having 6 to 10 carbon atoms, an allyl group, or a halogen atom; R³, R⁴, R⁸, and R⁹ are independently a hydrogen atom, a linear, branched, or cyclic alkyl group having 1 to 6 carbon atoms, a linear, branched, or cyclic alkenyl group having 2 to 6 carbon atoms, an aryl group having 6 to 10 carbon atoms, or a glycidyl group; R⁵ and R¹⁴ are independently a hydrogen atom, a linear, branched, or cyclic alkyl group having 1 to 10 carbon atoms, or an aryl group having 6 to 10 carbon atoms; "n", "m", "p", and "q" are integers of 1 to 3; R¹⁰ to R¹³ are independently a hydrogen atom, a halogen atom, a hydroxy group, a linear, branched or cyclic alkyl group having 1 to 6 carbon atoms, or a linear, branched or cyclic alkoxy group having 1 to 6 carbon atoms; wherein the symbols in the formula are applied only to this formula.

Examples of the (A) resin or compound for forming an organic film for use in the present invention include reaction products that are obtained by the method described in JP 2012-145897 A. More specific examples thereof include polymers that are obtained by condensing one or more compounds represented by the following general formula (23-1) and/or (23-2) and one or more compounds represented by the following general formula (24-1) and/or (24-2) and/or equivalents thereof: 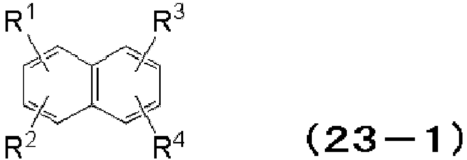 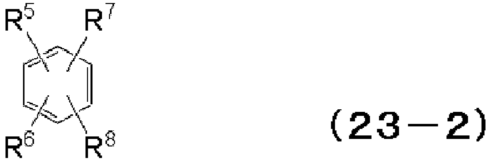
wherein R¹ to R⁸ are independently of each other a hydrogen atom, a halogen atom, a hydroxy group, an isocyanato group, a glycidyloxy group, a carboxyl group, an amino group, an alkoxy group having 1 to 30 carbon atoms, an alkoxycarbonyl group having 1 to 30 carbon atoms, an alkanoyloxy group having 1 to 30 carbon atoms, or an optionally substituted saturated or unsaturated organic group having 1 to 30 carbon atoms, and any two substituents selected from R¹ to R⁴ or R⁵ to R⁸ in each molecule are optionally bonded together to further form a cyclic substituent; wherein the symbols in the formulae are applied only to these formulae. 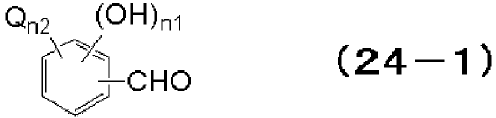 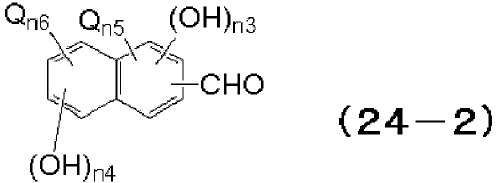
wherein Q is an optionally substituted organic group having 1 to 30 carbon atoms, and any two Q selected in the molecule are optionally bonded to form a cyclic substituent; and "n1" to "n6" are the numbers of each substituents, "n1" to "n6" are 0, 1, or 2, and in the formula (24-1), hydroxybenzaldehyde is excluded, and in the formula (24-2), the relationships of 0 ≤ n3 + n5 ≤ 3, 0 ≤ n4 + n6 ≤ 4, and 1 ≤ n3 + n4 ≤ 4 are satisfied; wherein the symbols in the formulae are applied only to these formulae.)

In addition, examples thereof include polymers that are obtained by condensing one or more compounds represented by the general formula (23-1) and/or (23-2), one or more compounds represented by the general formula (24-1) and/or (24-2) and/or equivalents thereof, and one or more compounds represented by the following general formula (25) and/or equivalents thereof: 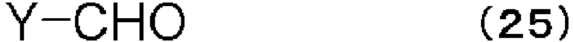 wherein Y is a hydrogen atom or a monovalent organic group having 30 or less carbon atoms and optionally having a substituent, and the formula (25) is different from the general formula (24-1) and the general formula (24-2); wherein the symbols in the formulae are applied only to these formulae.

Examples of the (A) resin or compound for forming an organic film for use in the present invention include compounds containing the following structure described in JP 2017-119671 A: 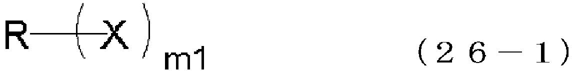
wherein R is a single bond or an organic group having 1 to 50 carbon atoms; X is a group represented by the following general formula (26-2); and "m1" is an integer satisfying 2 ≤ m1 ≤ 10; wherein the symbols in the formulae are applied only to these formulae, 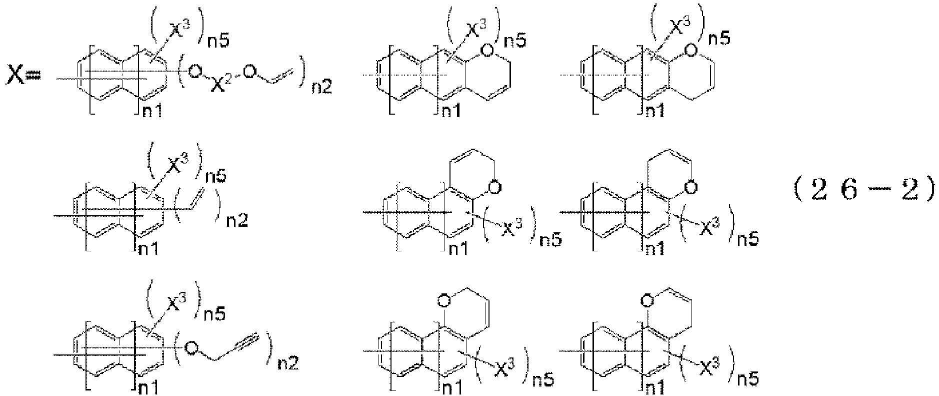
wherein X² is a divalent organic group having 1 to 10 carbon atoms; "n1" is 0 or 1; "n2" is 1 or 2; X³ is a group represented by the following general formula (26-3); and "n5" is 0, 1, or 2; wherein the symbols in the formulae are applied only to these formulae, 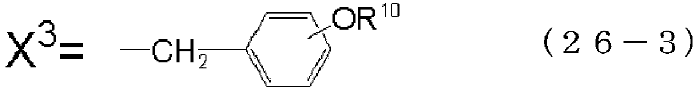
wherein R¹⁰ is a hydrogen atom or a saturated or unsaturated hydrocarbon group having 1 to 10 carbon atoms, and a hydrogen atom on the benzene ring in the formula is optionally replaced by a methyl group or a methoxy group; wherein the symbols in the formulae are applied only to these formulae.

Examples of a compound containing the above structure include the following compounds:

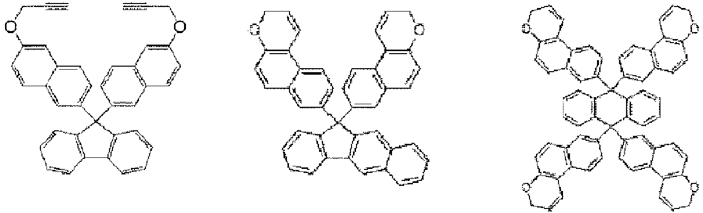

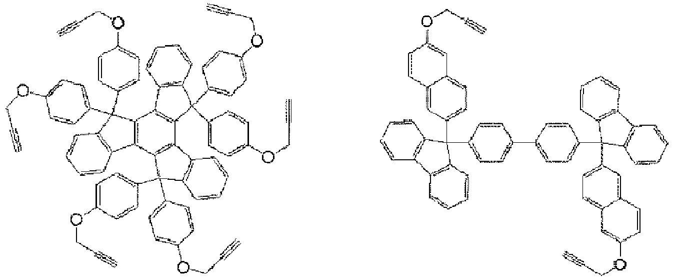

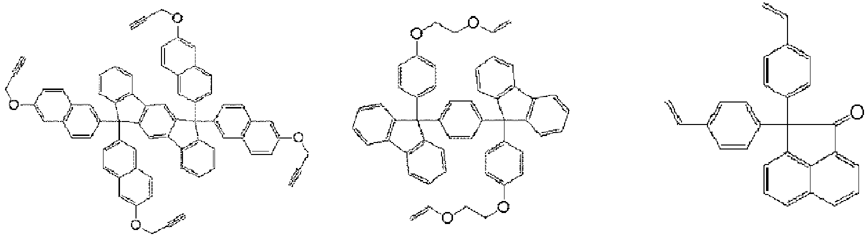

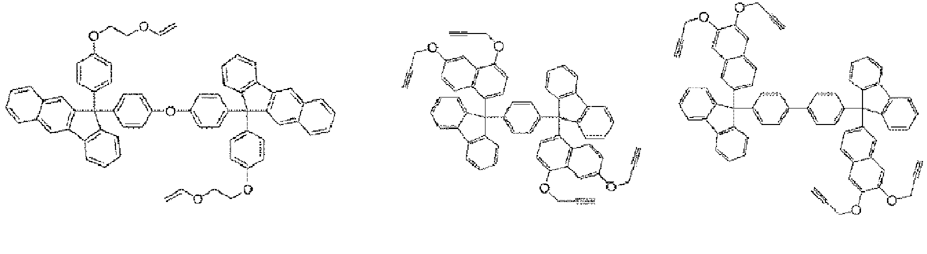

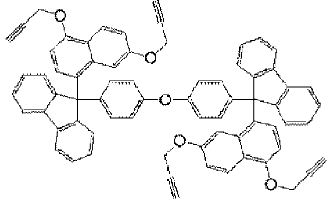

Examples of the (A) resin or compound for forming an organic film for use in the present invention include polymers having a repeating unit represented by the following general formula (27-1) described in JP 2019-044022 A: 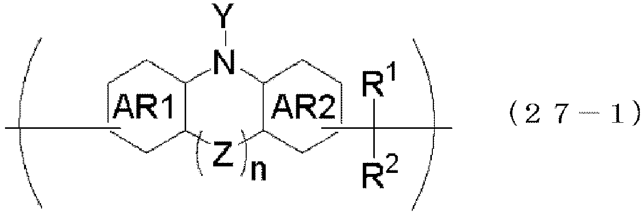
wherein AR1 and AR2 are benzene rings or naphthalene rings optionally having a substituent; R¹ and R² are each independently a hydrogen atom or an organic group having 1 to 30 carbon atoms, and when R¹ and R² are organic groups, R¹ and R² are optionally bonded to each other in the molecule to form a cyclic organic group; "n" is 0 or 1, and when n = 0, AR1 and AR2 do not form a bridged structure between aromatic rings of AR1 and AR2 through Z, and when n = 1, AR1 and AR2 form a bridged structure between the aromatic rings of AR1 and AR2 through Z, and Z is any of a single bond or the following formula (27-2); and Y is a group represented by the following formula (27-3); wherein the symbols in the formula are applied only to this formula.

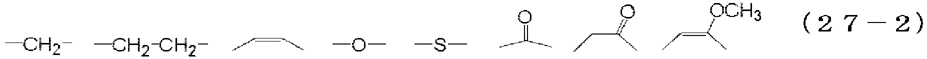

   **----**R³-C≡C-R⁴ (27-3)
wherein R³ is a single bond or a divalent organic group having 1 to 20 carbon atoms; R⁴ is a hydrogen atom or a monovalent organic group having 1 to 20 carbon atoms; and the dashed lines represent an attachment point; wherein the symbols in the formulae are applied only to these formulae.

Examples of the polymers having the repeating unit represented by the general formula (27-1) include the following polymers:

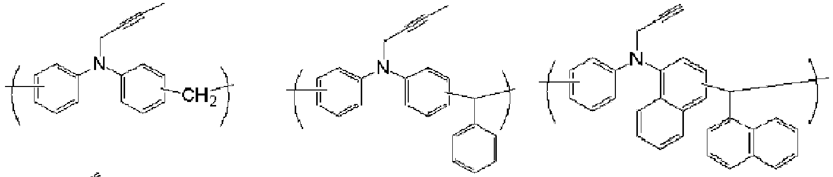

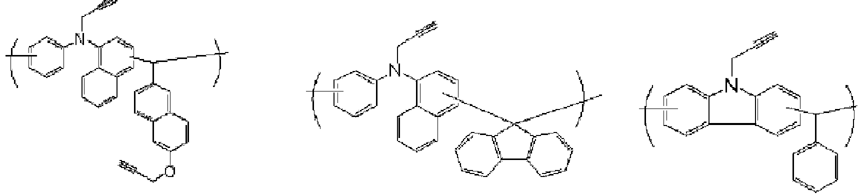

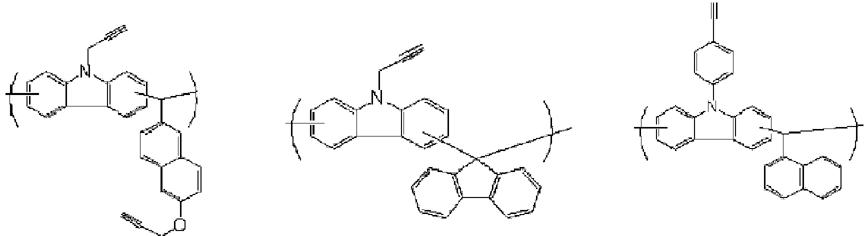

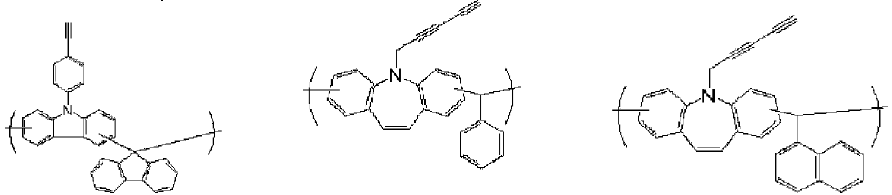

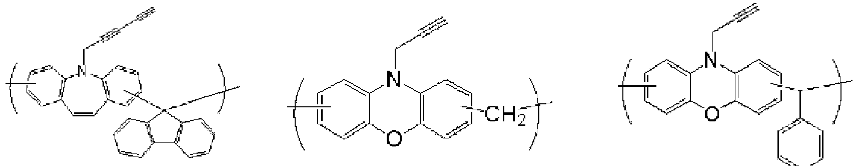

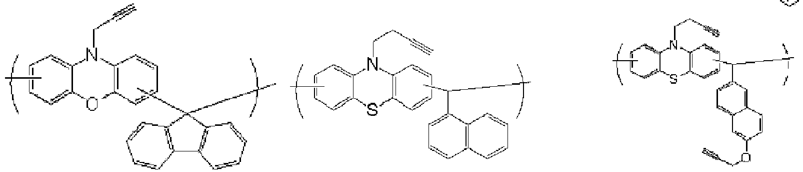

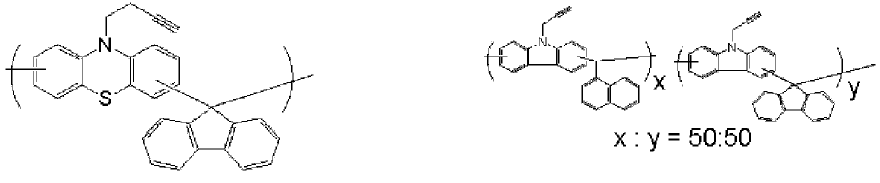

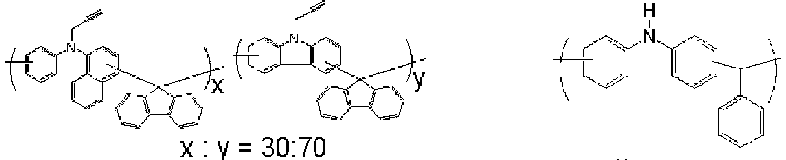

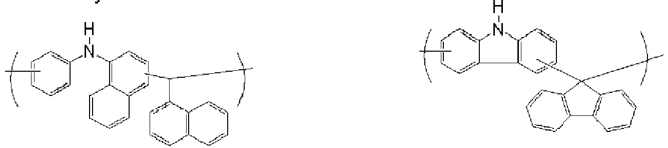

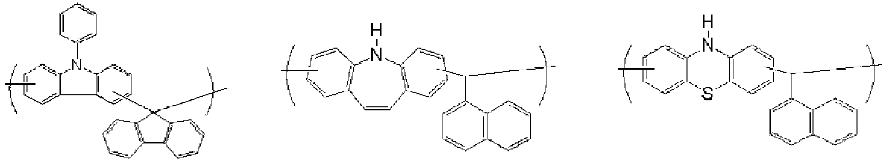

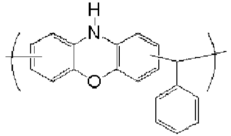

The (A) resin or compound for forming an organic film may be synthesized by a known method or a commercially available product that can be commercially procured may be used.

The amount of the (A) resin or compound for forming an organic film blended is not particularly limited as long as the film formability of the compound for forming an organic film by spin coating is satisfied, and the content of the (A) resin or compound for forming an organic film is preferably 10 to 40 parts by mass, more preferably 10 to 30 parts by mass, and still more preferably 10 to 25 parts by mass based on 100 parts by mass of the composition for forming an organic film. For example, in the case of embedding a hole or trench having an extremely high aspect ratio in a 3D NAND memory architecture with the composition for forming an organic film, the amount of the resin for forming an organic film blended needs to be increased, but such a composition for forming an organic film is highly viscous, and the in-plane uniformity or the embedding characteristics after spin coating deteriorate. The composition for forming an organic film of the present invention is capable of forming an organic film having excellent in-plane uniformity and embedding characteristics even when the rate of the (A) resin for forming an organic film blended is as described above, and thus can be suitably applied.

The content of the (B) compound is preferably 0.01 parts by mass to 5 parts by mass based on 100 parts by mass of the (A) resin or compound for forming an organic film. When the composition for forming an organic film contains such a content of the compound, the in-plane uniformity of the formed organic film is superior.

### [(C) Solvent]

The (C) solvent that can be used in the composition for forming an organic film of the present invention is not particularly limited as long as the solvent is capable of dissolving the (A) resin or compound for forming an organic film and the (B) compound, and a solvent also capable of dissolving an acid generator, a crosslinking agent, a surfactant, and the like, which will be described below, is preferable. Specifically, it is possible to use solvents having a boiling point of lower than 180°C, such as the solvents described in Paragraphs (0091) and (0092) of JP 2007-199653 A. Among them, propylene glycol monomethyl ether acetate, propylene glycol monomethyl ether, 2-heptanone, cyclopentanone, cyclohexanone, and mixtures of two or more thereof are preferably used.

The content of the (C) solvent is 200 to 10,000 parts by mass and preferably 300 to 5,000 parts by mass based on 100 parts by mass of the (A) resin or compound for forming an organic film. When the content thereof is made to be within such a range, it is possible to adjust the concentration in accordance with a film thickness to be consumed.

Furthermore, to the composition for forming an organic film of the present invention, it is also possible to add a high-boiling point solvent having a boiling point of 180°C or higher to the solvent having a boiling point of lower than 180°C (a mixture of the solvent having a boiling point of lower than 180°C and the solvent having a boiling point of 180°C or higher) as an organic solvent. The high-boiling point solvent is not particularly limited as long as the solvent is capable of dissolving compounds for forming an organic film, such as hydrocarbons, alcohols, ketones, esters, ethers, and chlorinated solvents. Specific examples thereof include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, ethylene glycol monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butyl methyl ether, triethylene glycol dimethyl ether, triethylene glycol monomethyl ether, triethylene glycol-n-butyl ether, triethylene glycol butyl methyl ether, triethylene glycol diacetate, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol monomethyl ether acetate, dipropylene glycol methyl-n-propyl ether, dipropylene glycol methyl ether acetate, 1,4-butanediol diacetate, 1,3-butylene glycol diacetate, 1,6-hexanediol diacetate, triethylene glycol diacetate, γ-butyrolactone, dihexyl malonate, diethyl succinate, dipropyl succinate, dibutyl succinate, dihexyl succinate, dimethyl adipate, diethyl adipate, and dibutyl adipate, and these may be used singly or as a mixture.

The boiling point of the high-boiling point solvent may be selected as appropriate in accordance with a temperature of a heat treatment on the composition for forming an organic film, and the boiling point of the high-boiling point solvent to be added is preferably 180°C to 300°C and more preferably 200°C to 300°C. When the boiling point is as described above, since there is no concern of volatilization during baking (heat treatment) being too rapid due to a boiling point that is too low, it is possible to obtain sufficient thermal fluidity. In addition, when the boiling point is as described above, since the boiling point is high, the solvent does not remain without volatilizing in the film even after baking, and there is thus no concern that film physical properties, such as etching resistance, may not be adversely affected.

In addition, in the case of using the above-described high-boiling point solvent, the amount of the high-boiling point solvent blended is preferably 1 to 30 parts by mass based on 100 parts by mass of the solvent having a boiling point of lower than 180°C. When the amount blended is as described above, there is no concern that it may become impossible to impart sufficient thermal fluidity during baking due to the amount blended being too small or the solvent may remain in the film due to the amount blended being too high and film physical properties, such as etching resistance, may deteriorate.

When the composition for forming an organic film is as described above, thermal fluidity is imparted by the addition of the high-boiling point solvent to the resin or compound for forming an organic film, whereby the composition for forming an organic film obtains both high-level embedding/flattening characteristics.

### [Other components]

In addition, to the composition for forming an organic film of the present invention, it is possible to add an acid generator or a crosslinking agent to further accelerate a crosslinking reaction. As the acid generator, there is an acid generator that generates an acid by pyrolysis or an acid generator that generates an acid by light irradiation, and any acid generator can be added thereto. Specific examples of the acid generator include acid generators described in Paragraphs [0061] to [0085] of JP 2007-199653 A. The acid generators can be used singly or two or more thereof can be used in combination. In the case of adding the acid generator, the amount added is preferably 0.05 to 50 parts by mass and more preferably 0.1 to 10 parts by mass based on 100 parts by mass of the (A) resin or compound for forming an organic film. When the amount added is such an amount, it becomes possible to accelerate a crosslinking reaction and to form a dense film.

In addition, specific examples of the crosslinking agent include crosslinking agents described in Paragraphs [0055] to [0060] of JP 2007-199653 A. The crosslinking agents can be used singly or two or more thereof can be used in combination. The amount of the crosslinking agent added is preferably 1 to 100 parts by mass and more preferably 5 to 50 parts by mass based on 100 parts by mass of the (A) resin or compound for forming an organic film. When the amount added is such an amount, it becomes possible to enhance curability and to further suppress the intermixing with an upper layer film.

In addition, to the composition for forming an organic film of the present invention, it is also possible to add a surfactant other than the (B) compound of the present invention in order to further improve the in-plane uniformity in spin coating. Specific examples of the surfactant include the surfactants described in Paragraphs [0142] to [0147] of JP 2009-269953 A. The surfactants can be used singly or two or more thereof can be used in combination. In the case of adding the surfactant, the amount added is preferably 0.01 to 10 parts by mass and more preferably 0.05 to 5 parts by mass based on 100 parts by mass of the resin or compound for forming an organic film. When the amount added is such an amount, it becomes possible to form an organic film having excellent in-plane uniformity.

Furthermore, to the composition for forming an organic film of the present invention, it is possible to add a basic compound to improve storage stability. The basic compound plays a role of an acid quencher to prevent a trace amount of an acid generated by the acid generator from causing a crosslinking reaction to progress. Specific examples of such a basic compound include basic compounds described in Paragraphs [0086] to [0090] of JP 2007-199653 A. The basic compounds can be used singly or two or more thereof can be used in combination. In the case of adding the basic compound, the amount added is preferably 0.05 to 50 parts by mass and more preferably 0.1 to 10 parts by mass based on 100 parts by mass of the (A) resin or compound for forming an organic film. When the amount added is such an amount, it becomes possible to improve the storage stability of the composition for forming an organic film.

As described above, the composition for forming an organic film of the present invention makes compositions for forming an organic film be excellent in terms of hump suppression during an EBR process. Therefore, the composition for forming an organic film of the present invention is extremely useful as a resist lower layer film material (organic film material) for multi-layer resist processes, such as a two-layer resist process, a three-layer resist process in which a silicon-containing resist middle layer film or a silicon-containing inorganic hard mask middle layer film is used, or a four-layer resist process in which a silicon-containing resist middle layer film or a silicon-containing inorganic hard mask middle layer film and an organic antireflection film or an adhesive film are used.

### [Method for forming organic film]

The present invention provides a method for forming an organic film for use in a process of manufacturing a semiconductor device, in which spin coating of the above-described composition for forming an organic film of the present invention is performed on a substrate to be processed, and a heat treatment is performed on the substrate coated with the composition for forming an organic film at a temperature of 100°C or higher and 600°C or lower for a range of 10 to 600 seconds to cure the composition, thereby forming an organic film.

In this method for forming an organic film, first, spin coating of the above-described composition for forming an organic film of the present invention is performed on a substrate to be processed. The use of a spin coating method makes it possible to obtain favorable embedding characteristics. After a coating film at an end portion is removed by an EBR process, baking (heat treatment) is performed to cause a crosslinking reaction to progress. This baking makes it possible to evaporate the solvent in the composition, and it is thus possible to prevent mixing even in a case where a resist upper layer film or a silicon-containing resist middle layer film is formed on an organic film.

The baking is performed at a temperature of 100°C or higher and 600°C or lower for a range of 10 to 600 seconds and preferably performed at a temperature of 200°C or higher and 500°C or lower for a range of 10 to 300 seconds. When the influence on device damage or wafer deformation is taken into account, the upper limit of the heating temperature in a wafer process of lithography is preferably 600°C or lower and more preferably 500°C or lower. When the heat treatment is performed under such a condition, a crosslinking reaction is caused to progress, and it is possible to form an organic film that does not mix with a film that is formed in an upper layer.

### [Patterning process]

Hereinafter, a patterning process in which the composition for forming an organic film of the present invention is used will be described.

### [Three-layer resist process in which silicon-containing resist middle layer film is used]

The present invention provides a patterning process,
in which an organic film is formed on a body to be processed using the above-described composition for forming an organic film,
a silicon-containing resist middle layer film is formed on the organic film using a silicon-containing resist middle layer film material,
a resist upper layer film is formed on the silicon-containing resist middle layer film using a resist upper layer film material composed of a photoresist composition,
a circuit pattern is formed in the resist upper layer film,
the pattern is transferred to the silicon-containing resist middle layer film by etching using the resist upper layer film in which the circuit pattern has been formed as a mask,
the pattern is transferred to the organic film by etching using the silicon-containing resist middle layer film to which the pattern has been transferred as a mask, and
the pattern is further transferred to the body to be processed by etching using the organic film to which the pattern has been transferred as a mask.

As the body to be processed, it is preferable to use a semiconductor device substrate or the semiconductor device substrate on which any of a metal film, a metal carbide film, a metal oxide film, a metal nitride film, a metal oxycarbide film, and a metal oxynitride film has been formed, and more specifically, a substrate of Si, α-Si, p-Si, SiO₂, SiN, SiON, W, TiN, Al, or the like, the substrate on which the above-described metal film or the like has been formed as a layer to be processed, or the like can be used while the body to be processed is not particularly limited thereto.

As the layer to be processed, it is possible to use a variety of low-k films of Si, SiO₂, SiON, SiN, p-Si, α-Si, W, W-Si, Al, Cu, Al-Si, and the like and stopper films thereof, which can be formed in a thickness of normally 50 to 10,000 nm, particularly, 100 to 5,000 nm. In the case of forming the layer to be processed, the substrate and the layer to be processed that are made of different materials are used.

As a metal that constitutes the body to be processed, it is preferable to use silicon, titanium, tungsten, hafnium, zirconium, chromium, germanium, copper, silver, gold, aluminum, indium, gallium, arsenic, palladium, iron, tantalum, iridium, molybdenum, or an alloy thereof.

At the time of forming an organic film using the composition for forming an organic film of the present invention on the body to be processed, the above-described method for forming an organic film of the present invention may be applied.

Next, a resist middle layer film (silicon-containing resist middle layer film) is formed on the organic film using a resist middle layer film material containing a silicon atom. As a resist middle layer film material containing a silicon atom, a polysiloxane-based middle layer film material is preferable. When an antireflection effect is provided to the silicon-containing resist middle layer film, it is possible to suppress reflection. Particularly, for exposure to 193 nm, when a material containing many aromatic groups and having high etching selectivity with the substrate is used as the composition for forming an organic film, the k value becomes high, and substrate reflection becomes high, but reflection can be suppressed by making the silicon-containing resist middle layer film absorb light so that the k value becomes appropriate, and it is possible to make substrate reflection be 0.5% or less. As the silicon-containing resist middle layer film having an antireflection effect, a polysiloxane that has anthracene for exposure to 248 nm and 157 nm and a phenyl group or a light absorption group having a silicon-silicon bond for exposure to 193 nm in a pendant structure and crosslinks with an acid or heat is preferable used.

Next, a resist upper layer film is formed on the silicon-containing resist middle layer film using a resist upper layer film material composed of a photoresist composition. The resist upper layer film material may be any of a positive material or a negative material, and the same material as the photoresist composition being normally used can be used. After spin coating of the resist upper layer film material, it is preferable to perform prebaking at 60°C to 180°C for a range of 10 to 300 seconds. After that, exposure is performed according to a normal method, furthermore, post exposure bake (PEB) and development are performed to obtain a resist upper layer film pattern. The thickness of the resist upper layer film is not particularly limited, and is preferably 30 to 500 nm and particularly preferably 50 to 400 nm.

Next, a circuit pattern (resist upper layer film pattern) is formed in the resist upper layer film. In the formation of the circuit pattern, it is preferable to form the circuit pattern by lithography for which light having a wavelength of 10 nm or longer and 300 nm or shorter is used, direct writing with an electron beam, nanoimprinting, or a combination thereof.

Examples of exposure light include high-energy rays having a wavelength of 300 nm or shorter, specifically, far ultraviolet rays, KrF excimer laser light (248 nm), ArF excimer laser light (193 nm), F₂ laser light (157 nm), Kr₂ laser light (146 nm), Ar₂ laser light (126 nm), 3 to 20 nm soft X-rays (EUV), electron beams (EB), ion beams, and X-rays.

In addition, in the formation of the circuit pattern, the circuit pattern is preferably developed by alkali development or with an organic solvent.

Next, the pattern is transferred to the silicon-containing resist middle layer film by etching using the resist upper layer film in which the circuit pattern has been formed as a mask. The etching of the silicon-containing resist middle layer film using the resist upper layer film pattern as the mask is preferably performed using a fluorocarbon-based gas. This makes the silicon-containing resist middle layer film pattern transferred.

Next, the pattern is transferred to the organic film by etching using the silicon-containing resist middle layer film to which the pattern has been transferred as a mask. Since the silicon-containing resist middle layer film exhibits resistance to etching with an oxygen gas or a hydrogen gas, the etching of the organic film using the silicon-containing resist middle layer film pattern as the mask is preferably performed using an etching gas mainly containing an oxygen gas or a hydrogen gas. This makes the organic film pattern transferred.

Next, the pattern is transferred to the body to be processed by etching using the organic film to which the pattern has been transferred as a mask. The following etching of the body to be processed (layer to be processed) can be performed by a normal method, for example, when the body to be processed is a SiO₂, SiN, or silica-based low dielectric constant insulating film, the etching is performed with a gas mainly containing a chlorofluorocarbon-based gas, and when the body to be processed is p-Si, Al, or W, the etching is performed with a gas mainly containing a chlorine-based gas or a bromine-based gas. In the case of performing substrate processing by etching with a chlorofluorocarbon-based gas, the silicon-containing resist middle layer film pattern is peeled off at the same time as the substrate processing. On the other hand, in the case of performing substrate processing by etching with a chlorine-based gas or a bromine-based gas, in order to peel off the silicon-containing resist middle layer film pattern, there is a need to separately perform dry etching peeling with a chlorofluorocarbon-based gas after the substrate processing.

The organic film that is obtained using the composition for forming an organic film of the present invention can be made to be excellent in terms of etching resistance during the above-described etching of the body to be processed.

### [Four-layer resist process in which silicon-containing resist middle layer film and organic antireflection film or adhesive film are used]

In addition, the present invention provides a patterning process,
in which an organic film is formed on a body to be processed using the above-described composition for forming an organic film,
a silicon-containing resist middle layer film is formed on the organic film using a silicon-containing resist middle layer film material,
an organic antireflective film or an adhesive film is formed on the silicon-containing resist middle layer film,
a resist upper layer film is formed on the organic antireflective film or adhesive film using a resist upper layer film material composed of a photoresist composition,
a circuit pattern is formed in the resist upper layer film,
the pattern is transferred to the organic antireflective film or adhesive film and the silicon-containing resist middle layer film by etching using the resist upper layer film in which the circuit pattern has been formed as a mask,
the pattern is transferred to the organic film by etching using the silicon-containing resist middle layer film to which the pattern has been transferred as a mask, and
furthermore, the pattern is further transferred to the body to be processed by etching using the organic film to which the pattern has been transferred as a mask.

This method can be performed in the same manner as the above-described three-layer resist process in which the silicon-containing resist middle layer film is used except that the organic antireflective film (BARC) or the adhesive film is formed between the silicon-containing resist middle layer film and the resist upper layer film.

The organic antireflective film and the adhesive film can be formed by spin coating using a known organic antireflection film material.

### [Three-layer resist process in which inorganic hard mask is used]

In addition, the present invention provides a patterning process,
in which an organic film is formed on a body to be processed using the above-described composition for forming an organic film,
an inorganic hard mask selected from a silicon oxide film, a silicon nitride film, and a silicon oxynitride film is formed on the organic film,
a resist upper layer film is formed on the inorganic hard mask using a resist upper layer film material composed of a photoresist composition,
a circuit pattern is formed in the resist upper layer film,
the pattern is transferred to the inorganic hard mask by etching using the resist upper layer film in which the circuit pattern has been formed as a mask,
the pattern is transferred to the organic film by etching using the inorganic hard mask to which the pattern has been transferred as a mask, and
furthermore, the pattern is further transferred to the body to be processed by etching using the organic film to which the pattern has been transferred as a mask.

This method can be performed in the same manner as the above-described three-layer resist process in which the silicon-containing resist middle layer film is used except that the inorganic hard mask is formed on the organic film instead of the silicon-containing resist middle layer film.

The inorganic hard mask selected from a silicon oxide film, a silicon nitride film, and a silicon oxynitride film (SiON film) can be formed by a CVD method, an ALD method, or the like. A method for forming the silicon nitride film is described in, for example, JP 2002-334869 A, and WO 2004/066377 A1. The film thickness of the inorganic hard mask is preferably 5 to 200 nm and more preferably 10 to 100 nm. As the inorganic hard mask, a SiON film having a high effect as the antireflection film is most preferably used.

### [Four-layer resist process in which inorganic hard mask and organic antireflection film or adhesive film are used]

In addition, the present invention provides a patterning process,
in which an organic film is formed on a body to be processed using the above-described composition for forming an organic film,
an inorganic hard mask selected from a silicon oxide film, a silicon nitride film, and a silicon oxynitride film is formed on the organic film,
an organic antireflective film or an adhesive film is formed on the inorganic hard mask,
a resist upper layer film is formed on the organic antireflective film or adhesive film using a resist upper layer film material composed of a photoresist composition,
a circuit pattern is formed in the resist upper layer film,
the pattern is transferred to the organic antireflective film or adhesive film and the inorganic hard mask by etching using the resist upper layer film in which the circuit pattern has been formed as a mask,
the pattern is transferred to the organic film by etching using the inorganic hard mask to which the pattern has been transferred as a mask, and
furthermore, the pattern is further transferred to the body to be processed by etching using the organic film to which the pattern has been transferred as a mask.

This method can be performed in the same manner as the above-described three-layer resist process in which the inorganic hard mask is used except that the organic antireflective film (BARC) or the adhesive film is formed between the inorganic hard mask and the resist upper layer film.

Particularly, in the case of using a SiON film as the inorganic hard mask, a two-layer antireflection film of the SiON film and BARC makes it possible to suppress reflection even in high NA (more than 1.0) immersion lithography. Another merit of forming BARC is an effect of reducing the hemming of the resist upper layer film pattern directly above the SiON film.

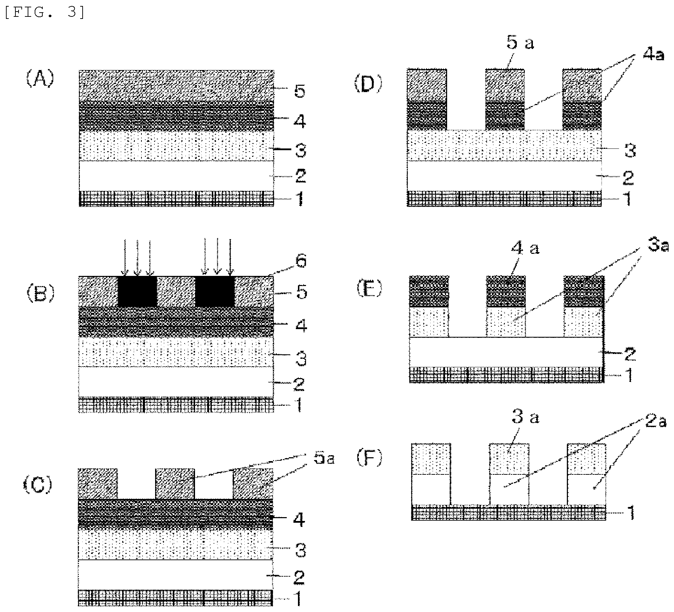

Here, one example of the patterning process by the three-layer resist process of the present invention will be shown in FIGS. 3(A) to 3(F). In the case of the three-layer resist process, after an organic film 3 is formed on a layer to be processed 2 formed on a substrate 1 using the composition for forming an organic film of the present invention as shown in FIG. 3(A), a silicon-containing resist middle layer film 4 is formed, and a resist upper layer film 5 is formed thereon. Next, exposure portions 6 of the resist upper layer film 5 are exposed as shown in FIG. 3(B), and PEB (post exposure bake) is performed. Next, a resist upper layer film pattern 5a is formed by development as shown in FIG. 3(C). Next, the silicon-containing resist middle layer film 4 is dry-etched using a chlorofluorocarbon-based gas and using a resist upper layer film pattern 5a as a mask to form a silicon-containing resist middle layer film pattern 4a as shown in FIG. 3(D). Next, the resist upper layer film pattern 5a is removed, and oxygen plasma etching is then performed on the organic film 3 using the silicon-containing resist middle layer film pattern 4a as a mask to form an organic film pattern 3a as shown in FIG. 3(E). Furthermore, the silicon-containing resist middle layer film pattern 4a is removed, and the layer to be processed 2 is then etched using an organic film pattern 3a as a mask to form a pattern 2a as shown in FIG. 3(F). A hump is suppressed during the formation of the organic film, which makes it possible to reduce a defect derived from a hump in the organic film during the dry etching of FIGS. (D), (E), and (F).

In the case of forming the inorganic hard mask, the silicon-containing resist middle layer film 4 may be changed to an inorganic hard mask, and in the case of forming BARC or an adhesive film, BARC or an adhesive film may be formed between the silicon-containing resist middle layer film 4 and the resist upper layer film 5. The etching of BARC or the adhesive film may be continuously performed prior to the etching of the silicon-containing resist middle layer film 4 or after only BARC or the adhesive film is etched, the silicon-containing resist middle layer film 4 may be etched by changing an etching device or the like.

As described above, in the patterning process of the present invention, the multi-layer resist process forms a fine pattern highly accurately on a body to be processed and suppresses the formation of a hump in an organic film, whereby it becomes possible to reduce a hump-derived defect in the organic film.

### EXAMPLE

Hereinafter, the present invention will be more specifically described by showing Synthesis Examples, Comparative Synthesis Examples, Examples, and Comparative Examples, but the present invention is not limited thereto. As a method for measuring the molecular weight, specifically, the molecular weight is measured by the following method. The polystyrene-equivalent weight average molecular weight (Mw) and number average molecular weight (Mn) were obtained by gel permeation chromatography (GPC) in which tetrahydrofuran was used as an eluent (solvent), and the dispersibility (Mw/Mn) was obtained.

### [Synthesis of compounds (A1) to (A41)]

Fluorine-containing compounds (A1) to (A41) used in Examples of the present invention were synthesized using thiol compounds (a1) to (a5) and modifiers (b1) to (b18) shown below.

### [Thiol compounds]

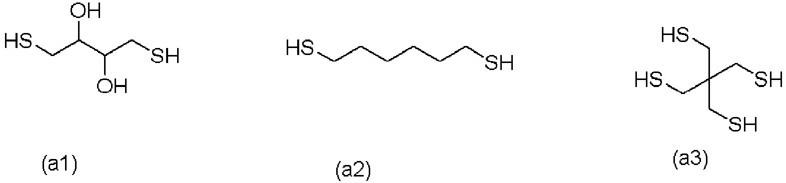

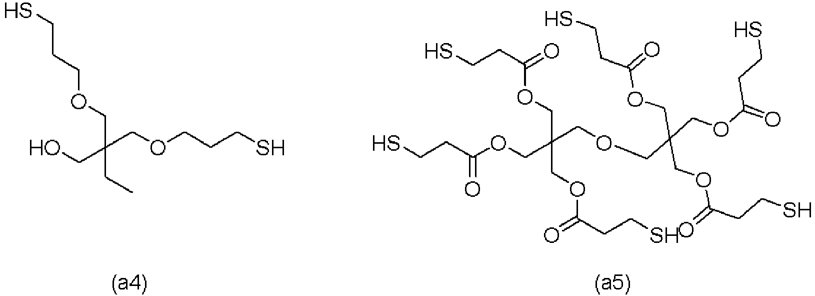

### [Modifiers]

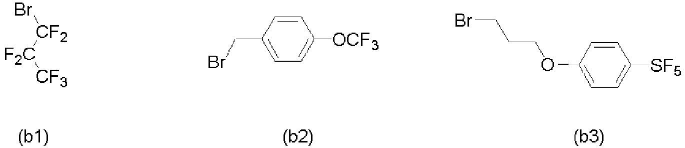

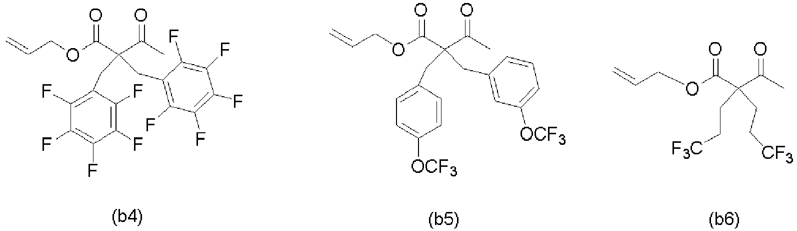

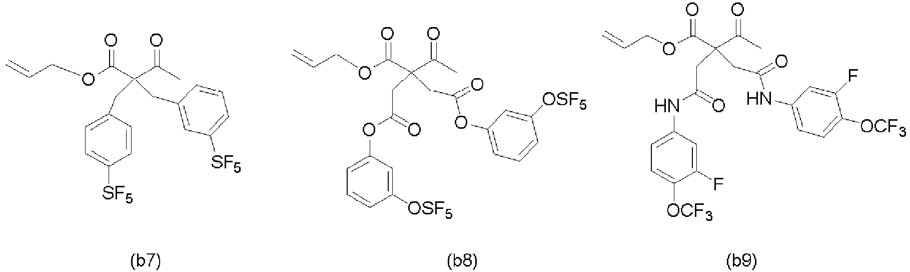

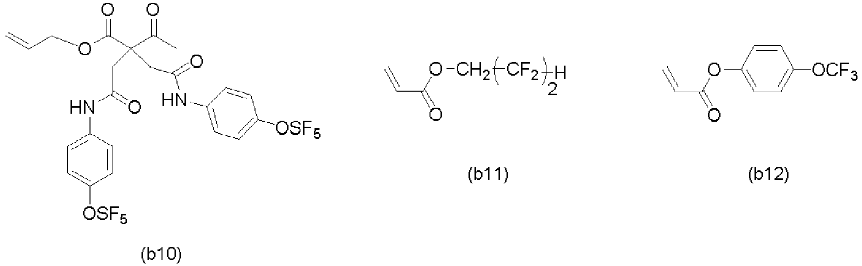

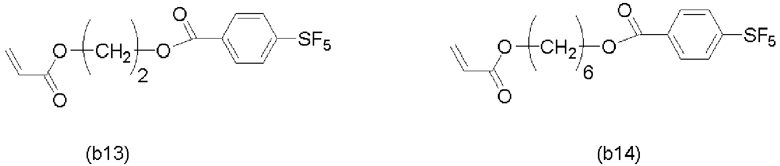

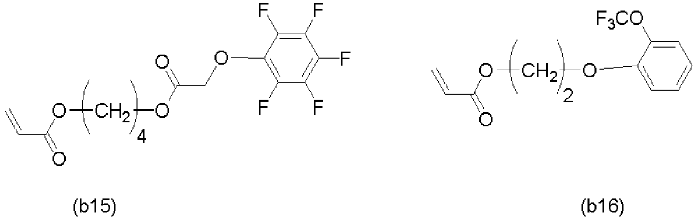

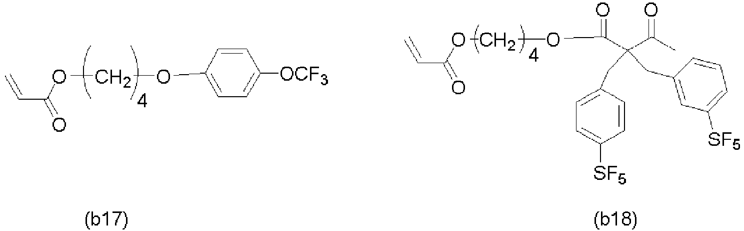

### [Synthesis of compound (A1)]

5.0 g of the thiol compound (a1), 8.96 g of potassium carbonate, and 40 g of DMF were added under a nitrogen atmosphere to produce a uniform dispersion at an internal temperature of 50°C. 16.1 g of the modifier (b1) was slowly added thereto, and a reaction was performed at an internal temperature of 50°C for 24 hours. 300 ml of methyl isobutyl ketone and 300 g of pure water were added to the reaction liquid to dissolve the precipitated salt, the separated water layer was then removed, cleaning was performed six times with 100 g of a 3% nitric acid aqueous solution and 100 g of pure water, and the organic layer was then dried under reduced pressure, thereby obtaining a compound (A1).

The weight average molecular weight (Mw) and the dispersibility (Mw/Mn) were obtained by GPC, and the results were as described below.
(A1):Mw = 700, Mw/Mn = 1.01

### [Synthesis of compound (A2)]

5.0 g of the thiol compound (a1), 8.96 g of potassium carbonate, and 40 g of DMF were added under a nitrogen atmosphere to produce a uniform dispersion at an internal temperature of 50°C. 16.5 g of the modifier (b2) was slowly added thereto, and a reaction was performed at an internal temperature of 50°C for 24 hours. 300 ml of methyl isobutyl ketone and 300 g of pure water were added to the reaction liquid to dissolve the precipitated salt, the separated water layer was then removed, cleaning was performed six times with 100 g of a 3% nitric acid aqueous solution and 100 g of pure water, and an organic layer was then dried under reduced pressure, thereby obtaining a compound (A2).

The weight average molecular weight (Mw) and the dispersibility (Mw/Mn) were obtained by GPC, and the results were as described below.
(A2):Mw = 720, Mw/Mn = 1.02

### [Synthesis of compound (A3)]

5.0 g of the thiol compound (a1), 8.96 g of potassium carbonate, and 40 g of DMF were added under a nitrogen atmosphere to produce a uniform dispersion at an internal temperature of 50°C. 22.1 g of the modifier (b3) was slowly added thereto, and a reaction was performed at an internal temperature of 50°C for 24 hours. 300 ml of methyl isobutyl ketone and 300 g of pure water were added to the reaction liquid to dissolve the precipitated salt, the separated water layer was then removed, cleaning was performed six times with 100 g of a 3% nitric acid aqueous solution and 100 g of pure water, and the organic layer was then dried under reduced pressure, thereby obtaining a compound (A3).

The weight average molecular weight (Mw) and the dispersibility (Mw/Mn) were obtained by GPC, and the results were as described below.
(A3):Mw = 840, Mw/Mn = 1.02

### [Synthesis of compound (A4)]

5.0 g of the thiol compound (a1), 32.6 g of the modifier (b4), and 100 g of THF were added under a nitrogen atmosphere to produce a uniform solution at an internal temperature of 60°C. 0.75 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A4).

The weight average molecular weight (Mw) and the dispersibility (Mw/Mn) were obtained by GPC, and the results were as described below.
(A4):Mw = 1190, Mw/Mn = 1.03

### [Synthesis of compound (A5)]

5.0 g of the thiol compound (a2), 32.6 g of the modifier (b5), and 100 g of THF were added under a nitrogen atmosphere to produce a uniform solution at an internal temperature of 60°C. 0.77 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A5).

The weight average molecular weight (Mw) and the dispersibility (Mw/Mn) were obtained by GPC, and the results were as described below.
(A5):Mw = 1180, Mw/Mn = 1.02

### [Synthesis of compound (A6)]

5.0 g of the thiol compound (a1), 31.8 g of the modifier (b5), and 100 g of THF were added under a nitrogen atmosphere to produce a uniform solution at an internal temperature of 60°C. 0.75 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A6).

The weight average molecular weight (Mw) and the dispersibility (Mw/Mn) were obtained by GPC, and the results were as described below.
(A6):Mw = 1160, Mw/Mn = 1.02

### [Synthesis of compound (A7)]

5.0 g of the thiol compound (a3), 48.9 g of the modifier (b5), and 150 g of THF were added under a nitrogen atmosphere to produce a uniform solution at an internal temperature of 60°C. 1.15 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A7).

The weight average molecular weight (Mw) and the dispersibility (Mw/Mn) were obtained by GPC, and the results were as described below.
(A7):Mw = 2260, Mw/Mn = 1.03

### [Synthesis of compound (A8)]

5.0 g of the thiol compound (a4), 17.4 g of the modifier (b5), and 80 g of THF were added under a nitrogen atmosphere to produce a uniform solution at an internal temperature of 60°C. 0.41 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A8).

The weight average molecular weight (Mw) and the dispersibility (Mw/Mn) were obtained by GPC, and the results were as described below.
(A8):Mw = 1360, Mw/Mn = 1.03

### [Synthesis of compound (A9)]

5.0 g of the thiol compound (a5), 18.8 g of the modifier (b5), and 100 g of THF were added under a nitrogen atmosphere to produce a uniform solution at an internal temperature of 60°C. 0.44 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A9).

The weight average molecular weight (Mw) and the dispersibility (Mw/Mn) were obtained by GPC, and the results were as described below.
(A9):Mw = 3860, Mw/Mn = 1.05

### [Synthesis of compound (A10)]

5.0 g of the thiol compound (a1), 21.7 g of the modifier (b6), and 100 g of THF were added under a nitrogen atmosphere to produce a uniform solution at an internal temperature of 60°C. 0.75 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A10).

The weight average molecular weight (Mw) and the dispersibility (Mw/Mn) were obtained by GPC, and the results were as described below.
(A10):Mw = 860, Mw/Mn = 1.01

### [Synthesis of compound (A11)]

5.0 g of the thiol compound (a2), 38.1 g of the modifier (b7), and 150 g of THF were added under a nitrogen atmosphere to produce a uniform solution at an internal temperature of 60°C. 0.77 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A11).

The weight average molecular weight (Mw) and the dispersibility (Mw/Mn) were obtained by GPC, and the results were as described below.
(A11):Mw = 1310, Mw/Mn = 1.02

### [Synthesis of compound (A12)]

5.0 g of the thiol compound (a1), 37.2 g of the modifier (b7), and 150 g of THF were added under a nitrogen atmosphere to produce a uniform solution at an internal temperature of 60°C. 0.75 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A12).

The weight average molecular weight (Mw) and the dispersibility (Mw/Mn) were obtained by GPC, and the results were as described below.
(A12):Mw = 1330, Mw/Mn = 1.03

### [Synthesis of compound (A13)]

5.0 g of the thiol compound (a3), 57.3 g of the modifier (b7), and 200 g of THF were added under a nitrogen atmosphere to produce a uniform solution at an internal temperature of 60°C. 1.15 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A13).

The weight average molecular weight (Mw) and the dispersibility (Mw/Mn) were obtained by GPC, and the results were as described below.
(A13):Mw = 2580, Mw/Mn = 1.04

### [Synthesis of compound (A14)]

5.0 g of the thiol compound (a4), 20.3 g of the modifier (b7), and 80 g of THF were added under a nitrogen atmosphere to produce a uniform solution at an internal temperature of 60°C. 0.41 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A14).

The weight average molecular weight (Mw) and the dispersibility (Mw/Mn) were obtained by GPC, and the results were as described below.
(A14):Mw = 1410, Mw/Mn = 1.02

### [Synthesis of compound (A15)]

5.0 g of the thiol compound (a2), 46.2 g of the modifier (b8), and 80 g of THF were added under a nitrogen atmosphere to produce a uniform solution at an internal temperature of 60°C. 0.77 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A15).

The weight average molecular weight (Mw) and the dispersibility (Mw/Mn) were obtained by GPC, and the results were as described below.
(A15):Mw = 1540, Mw/Mn = 1.03

### [Synthesis of compound (A16)]

5.0 g of the thiol compound (a1), 45.0 g of the modifier (b8), and 150 g of THF were added under a nitrogen atmosphere to produce a uniform solution at an internal temperature of 60°C. 0.75 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A16).

The weight average molecular weight (Mw) and the dispersibility (Mw/Mn) were obtained by GPC, and the results were as described below.
(A16):Mw = 1560, Mw/Mn = 1.02

### [Synthesis of compound (A17)]

3.0 g of the thiol compound (a3), 41.6 g of the modifier (b8), and 150 g of THF were added under a nitrogen atmosphere to produce a uniform solution at an internal temperature of 60°C. 0.69 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A17).

The weight average molecular weight (Mw) and the dispersibility (Mw/Mn) were obtained by GPC, and the results were as described below.
(A17):Mw = 3240, Mw/Mn = 1.05

### [Synthesis of compound (A18)]

5.0 g of the thiol compound (a1), 45.0 g of the modifier (b9), and 150 g of THF were added under a nitrogen atmosphere to produce a uniform solution at an internal temperature of 60°C. 0.75 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A18).

The weight average molecular weight (Mw) and the dispersibility (Mw/Mn) were obtained by GPC, and the results were as described below.
(A18):Mw = 1620, Mw/Mn = 1.02

### [Synthesis of compound (A19)]

5.0 g of the thiol compound (a2), 46.1 g of the modifier (b10), and 150 g of THF were added under a nitrogen atmosphere to produce a uniform solution at an internal temperature of 60°C. 0.77 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A19).

The weight average molecular weight (Mw) and the dispersibility (Mw/Mn) were obtained by GPC, and the results were as described below.
(A19):Mw = 1520, Mw/Mn = 1.03

### [Synthesis of compound (A20)]

5.0 g of the thiol compound (a1), 44.9 g of the modifier (b10), and 150 g of THF were added under a nitrogen atmosphere to produce a uniform solution at an internal temperature of 60°C. 0.75 g of dimethyl 2,2'-azobis(isobutyrate) was slowly added thereto, and a reaction was performed under reflux for 24 hours. After the reaction, a low-boiling point component, such as a reaction solvent, was distilled away and dried under reduced pressure, thereby obtaining a compound (A20).

The weight average molecular weight (Mw) and the dispersibility (Mw/Mn) were obtained by GPC, and the results were as described below.
(A20):Mw = 1560, Mw/Mn = 1.03

### [Synthesis of compound (A21)]

5.0 g of the thiol compound (a1), 12.1 g of the modifier (b11), and 80 g of THF were added under a nitrogen atmosphere to produce a uniform solution at an internal temperature of 60°C. 1.31 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A21).

The weight average molecular weight (Mw) and the dispersibility (Mw/Mn) were obtained by GPC, and the results were as described below.
(A21):Mw = 560, Mw/Mn = 1.01

### [Synthesis of compound (A22)]

5.0 g of the thiol compound (a2), 15.4 g of the modifier (b12), and 80 g of THF were added under a nitrogen atmosphere to produce a uniform solution at an internal temperature of 60°C. 1.35 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A22).

The weight average molecular weight (Mw) and the dispersibility (Mw/Mn) were obtained by GPC, and the results were as described below.
(A22):Mw = 620, Mw/Mn = 1.01

### [Synthesis of compound (A23)]

5.0 g of the thiol compound (a1), 15.1 g of the modifier (b12), and 80 g of THF were added under a nitrogen atmosphere to produce a uniform solution at an internal temperature of 60°C. 1.31 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A23).

The weight average molecular weight (Mw) and the dispersibility (Mw/Mn) were obtained by GPC, and the results were as described below.
(A23):Mw = 620, Mw/Mn = 1.01

### [Synthesis of compound (A24)]

5.0 g of the thiol compound (a3), 23.2 g of the modifier (b12), and 100 g of THF were added under a nitrogen atmosphere to produce a uniform solution at an internal temperature of 60°C. 2.02 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A24).

The weight average molecular weight (Mw) and the dispersibility (Mw/Mn) were obtained by GPC, and the results were as described below.
(A24):Mw = 1160, Mw/Mn = 1.03

### [Synthesis of compound (A25)]

5.0 g of the thiol compound (a4), 8.2 g of the modifier (b12), and 80 g of THF were added under a nitrogen atmosphere to produce a uniform solution at an internal temperature of 60°C. 0.72 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A25).

The weight average molecular weight (Mw) and the dispersibility (Mw/Mn) were obtained by GPC, and the results were as described below.
(A25):Mw = 770, Mw/Mn = 1.02

### [Synthesis of compound (A26)]

5.0 g of the thiol compound (a5), 8.9 g of the modifier (b12), and 80 g of THF were added under a nitrogen atmosphere to produce a uniform solution at an internal temperature of 60°C. 0.78 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A26).

The weight average molecular weight (Mw) and the dispersibility (Mw/Mn) were obtained by GPC, and the results were as described below.
(A26):Mw = 2330, Mw/Mn = 1.05

### [Synthesis of compound (A27)]

5.0 g of the thiol compound (a2), 23.0 g of the modifier (b13), and 120 g of THF were added under a nitrogen atmosphere to produce a uniform solution at an internal temperature of 60°C. 1.35 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A27).

The weight average molecular weight (Mw) and the dispersibility (Mw/Mn) were obtained by GPC, and the results were as described below.
(A27):Mw = 880, Mw/Mn = 1.02

### [Synthesis of compound (A28)]

5.0 g of the thiol compound (a1), 22.5 g of the modifier (b13), and 120 g of THF were added under a nitrogen atmosphere to produce a uniform solution at an internal temperature of 60°C. 1.31 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A28).

The weight average molecular weight (Mw) and the dispersibility (Mw/Mn) were obtained by GPC, and the results were as described below.
(A28):Mw = 850, Mw/Mn = 1.01

### [Synthesis of compound (A29)]

5.0 g of the thiol compound (a2), 26.8 g of the modifier (b14), and 120 g of THF were added under a nitrogen atmosphere to produce a uniform solution at an internal temperature of 60°C. 1.35 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A29).

The weight average molecular weight (Mw) and the dispersibility (Mw/Mn) were obtained by GPC, and the results were as described below.
(A29):Mw = 1010, Mw/Mn = 1.03

### [Synthesis of compound (A30)]

5.0 g of the thiol compound (a1), 26.1 g of the modifier (b14), and 120 g of THF were added under a nitrogen atmosphere to produce a uniform solution at an internal temperature of 60°C. 1.31 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A30).

The weight average molecular weight (Mw) and the dispersibility (Mw/Mn) were obtained by GPC, and the results were as described below.
(A30):Mw = 1040, Mw/Mn = 1.02

### [Synthesis of compound (A31)]

3.0 g of the thiol compound (a3), 24.1 g of the modifier (b14), and 120 g of THF were added under a nitrogen atmosphere to produce a uniform solution at an internal temperature of 60°C. 1.21 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A31).

The weight average molecular weight (Mw) and the dispersibility (Mw/Mn) were obtained by GPC, and the results were as described below.
(A31):Mw = 1990, Mw/Mn = 1.04

### [Synthesis of compound (A32)]

5.0 g of the thiol compound (a4), 14.2 g of the modifier (b14), and 100 g of THF were added under a nitrogen atmosphere to produce a uniform solution at an internal temperature of 60°C. 0.72 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A32).

The weight average molecular weight (Mw) and the dispersibility (Mw/Mn) were obtained by GPC, and the results were as described below.
(A32):Mw = 1120, Mw/Mn = 1.03

### [Synthesis of compound (A33)]

5.0 g of the thiol compound (a5), 15.4 g of the modifier (b14), and 80 g of THF were added under a nitrogen atmosphere to produce a uniform solution at an internal temperature of 60°C. 0.78 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A33).

The weight average molecular weight (Mw) and the dispersibility (Mw/Mn) were obtained by GPC, and the results were as described below.
(A33):Mw = 3490, Mw/Mn = 1.06

### [Synthesis of compound (A34)]

5.0 g of the thiol compound (a1), 23.9 g of the modifier (b15), and 80 g of THF were added under a nitrogen atmosphere to produce a uniform solution at an internal temperature of 60°C. 1.31 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A34).

The weight average molecular weight (Mw) and the dispersibility (Mw/Mn) were obtained by GPC, and the results were as described below.
(A34):Mw = 890, Mw/Mn = 1.01

### [Synthesis of compound (A35)]

5.0 g of the thiol compound (a2), 18.4 g of the modifier (b16), and 80 g of THF were added under a nitrogen atmosphere to produce a uniform solution at an internal temperature of 60°C. 1.35 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A35).

The weight average molecular weight (Mw) and the dispersibility (Mw/Mn) were obtained by GPC, and the results were as described below.
(A35):Mw = 700, Mw/Mn = 1.01

### [Synthesis of compound (A36)]

5.0 g of the thiol compound (a1), 17.9 g of the modifier (b16), and 80 g of THF were added under a nitrogen atmosphere to produce a uniform solution at an internal temperature of 60°C. 1.31 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A36).

The weight average molecular weight (Mw) and the dispersibility (Mw/Mn) were obtained by GPC, and the results were as described below.
(A36):Mw = 720, Mw/Mn = 1.01

### [Synthesis of compound (A37)]

5.0 g of the thiol compound (a1), 19.7 g of the modifier (b17), and 100 g of THF were added under a nitrogen atmosphere to produce a uniform solution at an internal temperature of 60°C. 1.31 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A37).

The weight average molecular weight (Mw) and the dispersibility (Mw/Mn) were obtained by GPC, and the results were as described below.
(A37):Mw = 820, Mw/Mn = 1.02

### [Synthesis of compound (A38)]

3.0 g of the thiol compound (a2), 26.4 g of the modifier (b18), and 100 g of THF were added under a nitrogen atmosphere to produce a uniform solution at an internal temperature of 60°C. 0.81 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A38).

The weight average molecular weight (Mw) and the dispersibility (Mw/Mn) were obtained by GPC, and the results were as described below.
(A38):Mw = 1540, Mw/Mn = 1.03

### [Synthesis of compound (A39)]

3.0 g of the thiol compound (a1), 25.7 g of the modifier (b18), and 100 g of THF were added under a nitrogen atmosphere to produce a uniform solution at an internal temperature of 60°C. 0.79 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A39).

The weight average molecular weight (Mw) and the dispersibility (Mw/Mn) were obtained by GPC, and the results were as described below.
(A39):Mw = 1570, Mw/Mn = 1.02

### [Synthesis of compound (A40)]

2.0 g of the thiol compound (a3), 26.4 g of the modifier (b18), and 100 g of THF were added under a nitrogen atmosphere to produce a uniform solution at an internal temperature of 60°C. 0.81 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A40).

The weight average molecular weight (Mw) and the dispersibility (Mw/Mn) were obtained by GPC, and the results were as described below.
(A40):Mw = 3120, Mw/Mn = 1.05

### [Synthesis of compound (A41)]

5.0 g of the thiol compound (a4), 23.4 g of the modifier (b18), and 100 g of THF were added under a nitrogen atmosphere to produce a uniform solution at an internal temperature of 60°C. 0.72 g of triethylamine diluted with 5 ml of THF in advance was slowly added dropwise thereto, and a reaction was performed under reflux for 24 hours. After the reaction, a low-boiling point component, such as a reaction solvent or a catalyst, was distilled away and dried under reduced pressure, thereby obtaining a compound (A41).

The weight average molecular weight (Mw) and the dispersibility (Mw/Mn) were obtained by GPC, and the results were as described below.
(A41):Mw = 1700, Mw/Mn = 1.04

The structural formulae of the compounds (A1) to (A41) obtained in Synthesis Examples described above are shown below.

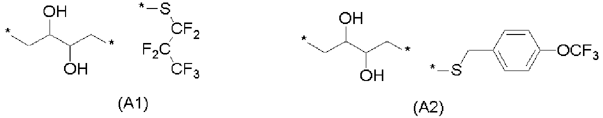

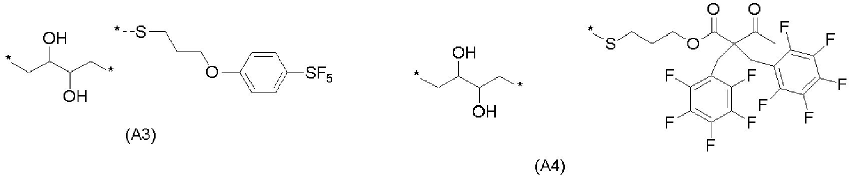

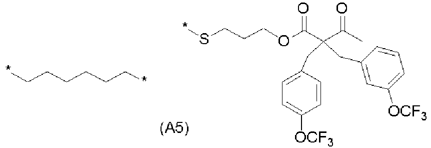

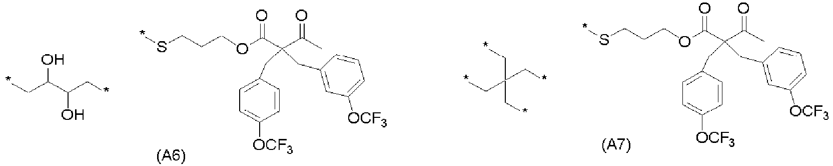

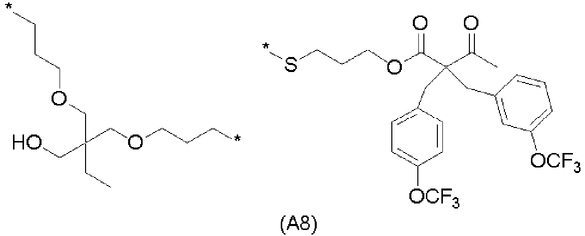

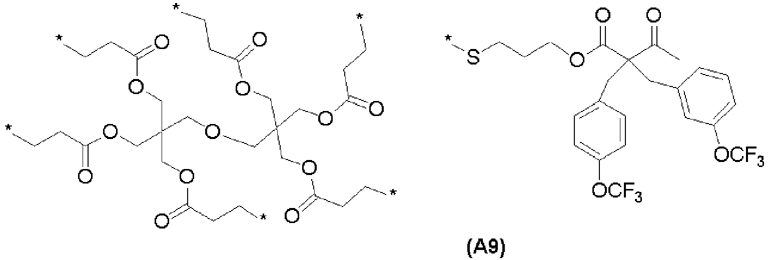

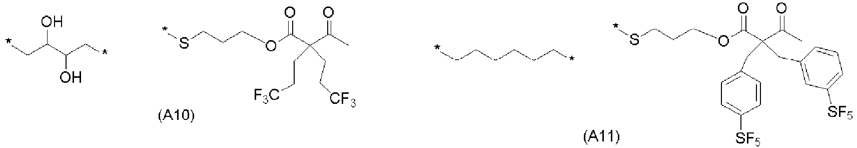

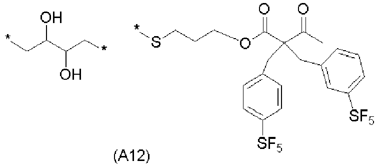

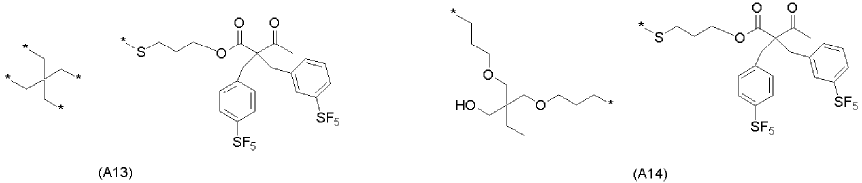

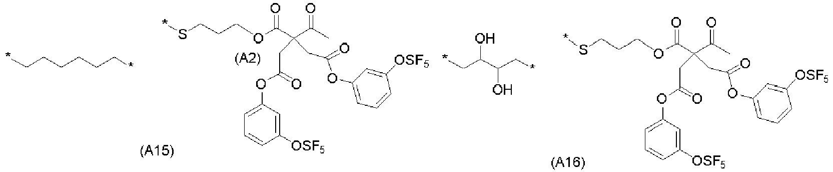

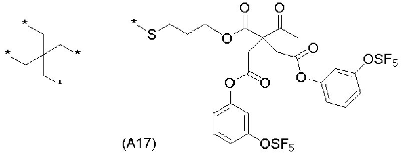

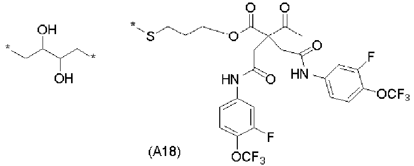

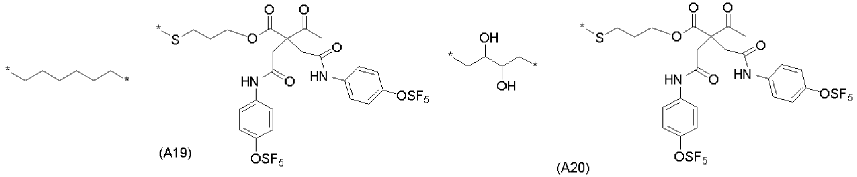

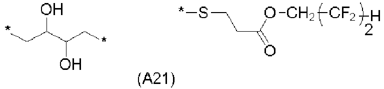

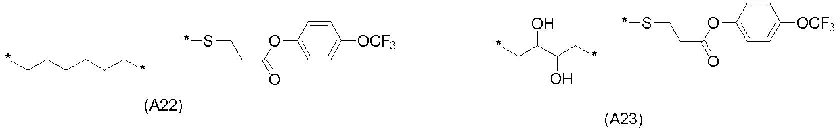

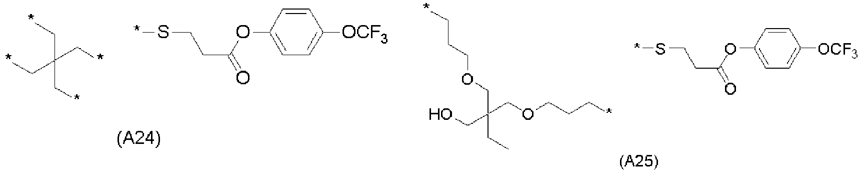

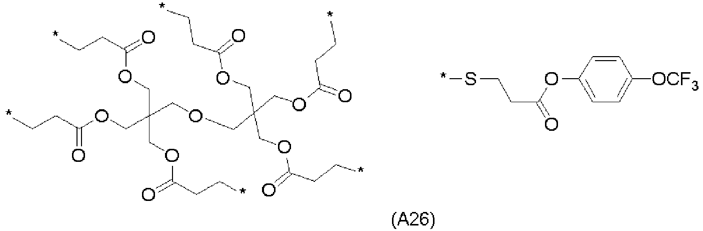

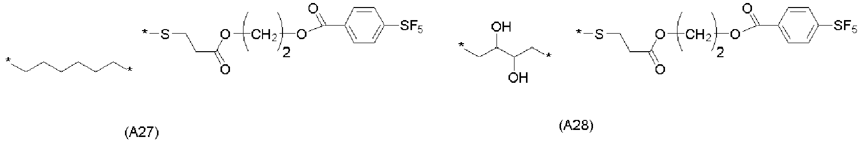

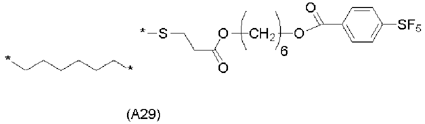

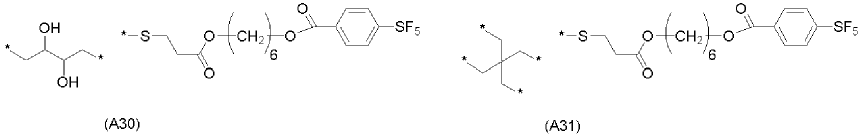

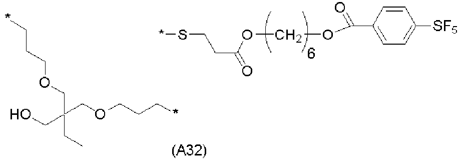

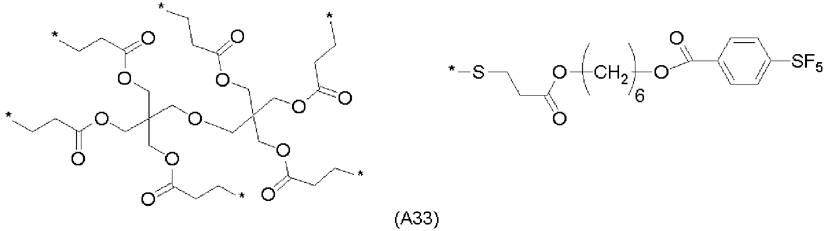

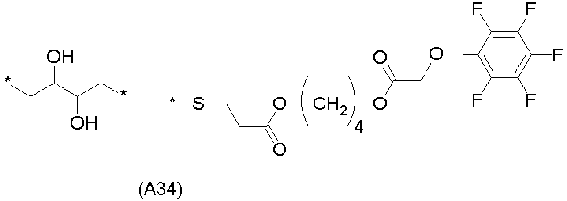

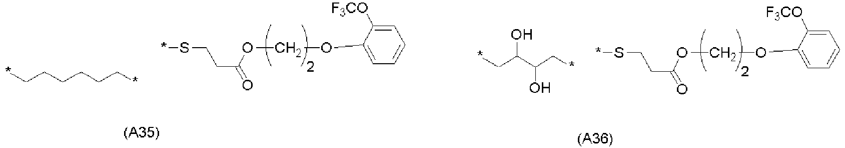

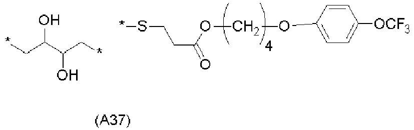

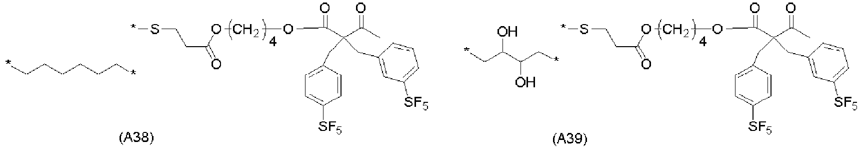

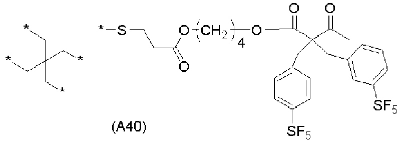

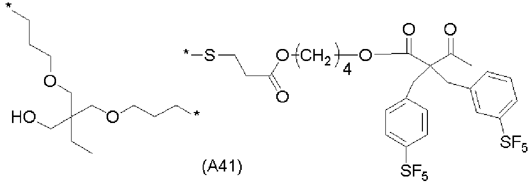

### [Syntheses of comparative polymers (R1) to (R5)]

For the syntheses of comparative polymers (R1) to (R5) used to prepare compositions for forming an organic film, the following monomers (c1) to (c6) were used.

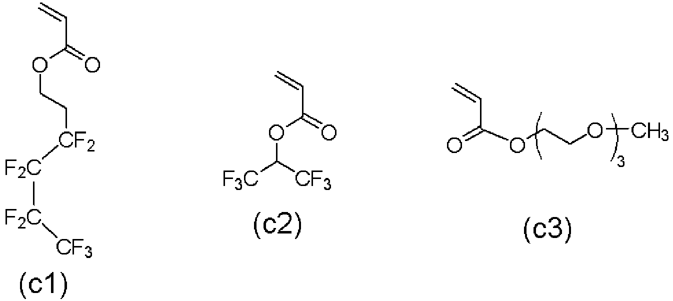

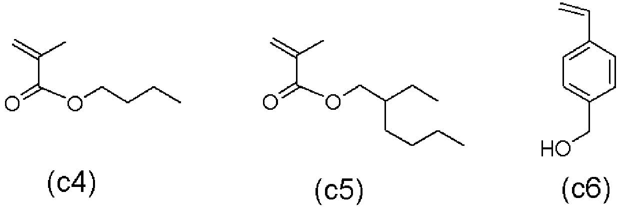

### [Comparative Synthesis Example 1] Synthesis of comparative polymer (R1)

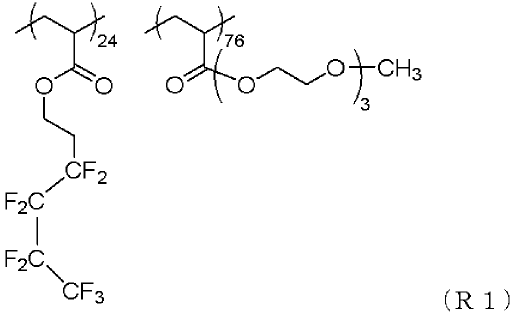

6.0 g of PGMEA was heated and stirred at 80°C under a nitrogen atmosphere. A mixture of 3.44 g (0.011 mol) of the monomer (c1), 7.46 g (0.034 mol) of the monomer (c3), 0.473 g of dimethyl 2,2-azobis(2-methylpropionate), and 34 g of PGMEA was added dropwise thereto for four hours. The components were further heated and stirred for 16 hours and then cooled to room temperature, thereby obtaining a PGMEA solution of an intended polymer (R1).

The weight average molecular weight (Mw) and the dispersibility (Mw/Mn) were obtained by GPC, and the results were as described below.
(R1):Mw = 9500, Mw/Mn = 1.20

### [Comparative Synthesis Example 2] Synthesis of comparative polymer (R2)

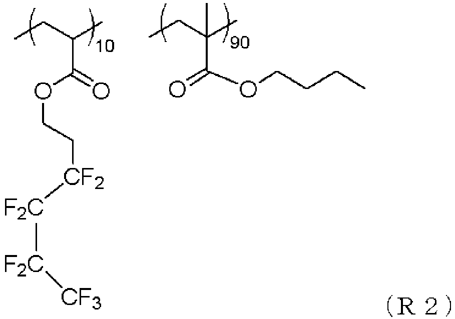

6.0 g of PGMEA was heated and stirred at 80°C under a nitrogen atmosphere. A mixture of 1.43 g (0.005 mol) of the monomer (c1), 5.76 g (0.041 mol) of the monomer (c4), 0.473 g of dimethyl 2,2-azobis(2-methylpropionate), and 34 g of PGMEA was added dropwise thereto for four hours. The components were further heated and stirred for 16 hours and then cooled to room temperature, thereby obtaining a PGMEA solution of an intended polymer (R2).

The weight average molecular weight (Mw) and the dispersibility (Mw/Mn) were obtained by GPC, and the results were as described below.
(R2):Mw = 5800, Mw/Mn = 1.42

### [Comparative Synthesis Example 3] Synthesis of comparative polymer (R3)

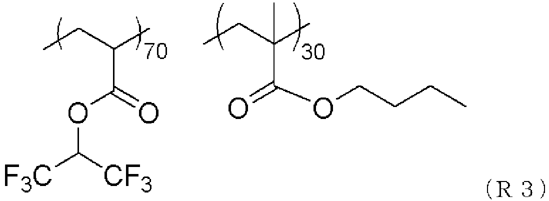

6.0 g of PGMEA was heated and stirred at 80°C under a nitrogen atmosphere. A mixture of 7.00 g (0.032 mol) of the monomer (c2), 1.92 g (0.014 mol) of the monomer (c4), 0.473 g of dimethyl 2,2-azobis(2-methylpropionate), and 34 g of PGMEA was added dropwise thereto for four hours. The components were further heated and stirred for 16 hours and then cooled to room temperature, thereby obtaining a PGMEA solution of an intended polymer (R3).

The weight average molecular weight (Mw) and the dispersibility (Mw/Mn) were obtained by GPC, and the results were as described below.
(R3):Mw = 6200, Mw/Mn = 1.33

### [Comparative Synthesis Example 4] Synthesis of comparative polymer (R4)

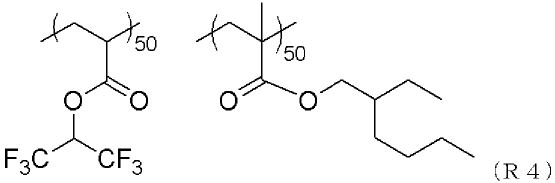

6.0 g of PGMEA was heated and stirred at 80°C under a nitrogen atmosphere. A mixture of 5.00 g (0.023 mol) of the monomer (c2), 3.20 g (0.023 mol) of the monomer (c5), 0.473 g of dimethyl 2,2-azobis(2-methylpropionate), and 34 g of PGMEA was added dropwise thereto for four hours. The components were further heated and stirred for 16 hours and then cooled to room temperature, thereby obtaining a PGMEA solution of an intended polymer (R4).

The weight average molecular weight (Mw) and the dispersibility (Mw/Mn) were obtained by GPC, and the results were as described below.
(R4):Mw = 8300, Mw/Mn = 1.33

### [Comparative Synthesis Example 5] Synthesis of comparative polymer (R5)

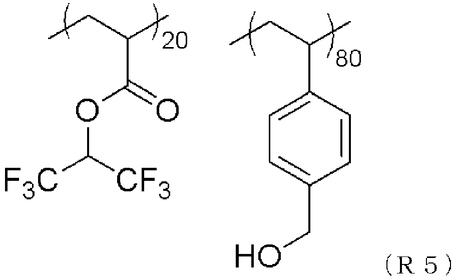

6.0 g of PGMEA was heated and stirred at 80°C under a nitrogen atmosphere. A mixture of 2.00 g (0.009 mol) of the monomer (c2), 5.41 g (0.036 mol) of the monomer (c6), 0.473 g of dimethyl 2,2-azobis(2-methylpropionate), and 34 g of PGMEA was added dropwise thereto for four hours. The components were further heated and stirred for 16 hours and then cooled to room temperature, thereby obtaining a PGMEA solution of an intended polymer (R5).

The weight average molecular weight (Mw) and the dispersibility (Mw/Mn) were obtained by GPC, and the results were as described below.
(R5):Mw = 3800, Mw/Mn = 1.44

### [Resins or compounds for forming organic film]

C1: Resin represented by the following formula (C1)
C2: Resin represented by the following formula (C2)
C3: Compound represented by the following formula (C3)
C4: Compound represented by the following formula (C4)
C5: Resin represented by the following formula (C5)
C6: Resin represented by the following formula (C6)

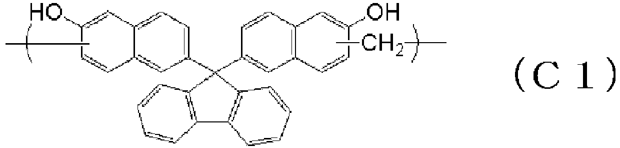

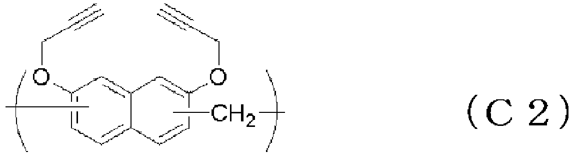

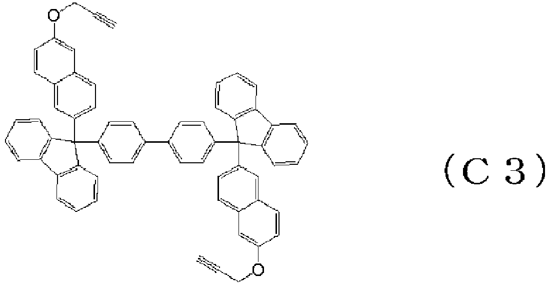

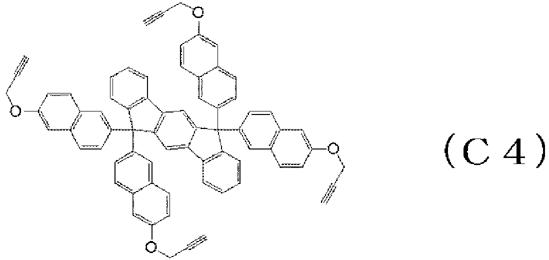

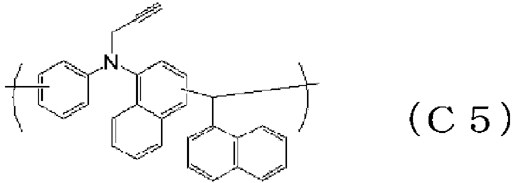

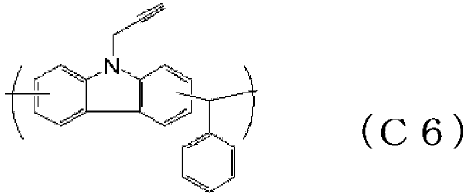

### [Solvents]

(D1): Propyleneglycol monomethylether acetate
(D2): Propyleneglycol monoethylether

### [Preparation of compositions for forming organic film (UDL-1 to 121 and comparative UDL-1 to 16)]

One of the polymers (A1) to (A41) and (R1) to (R5), one of the resins for forming an organic film (C1) to (C6), and one of the solvents were dissolved at fractions shown in Tables 1-1 to 1-4 and filtered with a 0.1 µm fluororesin filter, thereby preparing each of the compositions for forming an organic film (resist lower layer film materials: UDL-1 to 121 and comparative UDL-1 to 16).

**[Table 1-1]**

| Composition for forming organic film | Resin for forming organic film | | Compound | | Solvent 1 | | Solvent 2 | |
|---|---|---|---|---|---|---|---|---|
| | Type | Number of parts by mass | Type | Number of parts by mass | Type | Number of parts by mass | Type | Number of parts by mass |
| UDL-1 | (C1) | 10.00 | (A1) | 0.10 | (D1) | 89.90 | | |
| UDL-2 | (C1) | 10.00 | (A2) | 0.10 | (D1) | 89.90 | | |
| UDL-3 | (C1) | 10.00 | (A3) | 0.10 | (D1) | 89.90 | | |
| UDL-4 | (C1) | 10.00 | (A4) | 0.10 | (D1) | 89.90 | | |
| UDL-5 | (C1) | 10.00 | (A5) | 0.10 | (D1) | 89.90 | | |
| UDL-6 | (C1) | 10.00 | (A6) | 0.10 | (D1) | 89.90 | | |
| UDL-7 | (C1) | 10.00 | (A7) | 0.10 | (D1) | 89.90 | | |
| UDL-8 | (C1) | 10.00 | (A8) | 0.10 | (D1) | 89.90 | | |
| UDL-9 | (C1) | 10.00 | (A9) | 0.10 | (D1) | 89.90 | | |
| UDL-10 | (C1) | 10.00 | (A10) | 0.10 | (D1) | 89.90 | | |
| UDL-11 | (C1) | 10.00 | (A11) | 0.10 | (D1) | 89.90 | | |
| UDL-12 | (C1) | 10.00 | (A12) | 0.10 | (D1) | 89.90 | | |
| UDL-13 | (C1) | 10.00 | (A13) | 0.10 | (D1) | 89.90 | | |
| UDL-14 | (C1) | 10.00 | (A14) | 0.10 | (D1) | 89.90 | | |
| UDL-15 | (C1) | 10.00 | (A15) | 0.10 | (D1) | 89.90 | | |
| UDL-16 | (C1) | 10.00 | (A16) | 0.10 | (D1) | 89.90 | | |
| UDL-17 | (C1) | 10.00 | (A17) | 0.10 | (D1) | 89.90 | | |
| UDL-18 | (C1) | 10.00 | (A18) | 0.10 | (D1) | 89.90 | | |
| UDL-19 | (C1) | 10.00 | (A19) | 0.10 | (D1) | 89.90 | | |
| UDL-20 | (C1) | 10.00 | (A20) | 0.10 | (D1) | 89.90 | | |
| UDL-21 | (C1) | 10.00 | (A21) | 0.10 | (D1) | 89.90 | | |
| UDL-22 | (C1) | 10.00 | (A22) | 0.10 | (D1) | 89.90 | | |
| UDL-23 | (C1) | 10.00 | (A23) | 0.10 | (D1) | 89.90 | | |
| UDL-24 | (C1) | 10.00 | (A24) | 0.10 | (D1) | 89.90 | | |
| UDL-25 | (C1) | 10.00 | (A25) | 0.10 | (D1) | 89.90 | | |
| UDL-26 | (C1) | 10.00 | (A26) | 0.10 | (D1) | 89.90 | | |
| UDL-27 | (C1) | 10.00 | (A27) | 0.10 | (D1) | 89.90 | | |
| UDL-28 | (C1) | 10.00 | (A28) | 0.10 | (D1) | 89.90 | | |
| UDL-29 | (C1) | 10.00 | (A29) | 0.10 | (D1) | 89.90 | | |
| UDL-30 | (C1) | 10.00 | (A30) | 0.10 | (D1) | 89.90 | | |
| UDL-31 | (C1) | 10.00 | (A31) | 0.10 | (D1) | 89.90 | | |
| UDL-32 | (C1) | 10.00 | (A32) | 0.10 | (D1) | 89.90 | | |
| UDL-33 | (C1) | 10.00 | (A33) | 0.10 | (D1) | 89.90 | | |
| UDL-34 | (C1) | 10.00 | (A34) | 0.10 | (D1) | 89.90 | | |
| UDL-35 | (C1) | 10.00 | (A35) | 0.10 | (D1) | 89.90 | | |
| UDL-36 | (C1) | 10.00 | (A36) | 0.10 | (D1) | 89.90 | | |
| UDL-37 | (C1) | 10.00 | (A37) | 0.10 | (D1) | 89.90 | | |
| UDL-38 | (C1) | 10.00 | (A38) | 0.10 | (D1) | 89.90 | | |
| UDL-39 | (C1) | 10.00 | (A39) | 0.10 | (D1) | 89.90 | | |
| UDL-40 | (C1) | 10.00 | (A40) | 0.10 | (D1) | 89.90 | | |

**[Table 1-2]**

| Composition for forming organic film | Resin for forming organic film | | Compound | | Solvent 1 | | Solvent 2 | |
|---|---|---|---|---|---|---|---|---|
| | Type | Number of parts by mass | Type | Number of parts by mass | Type | Number of parts by mass | Type | Number of parts by mass |
| UDL-41 | (C1) | 10.00 | (A41) | 0.10 | (D1) | 89.90 | | |
| UDL-42 | (C1) | 10.00 | (A1) | 0.10 | (D1) | 62.93 | (D2) | 26.97 |
| UDL-43 | (C1) | 10.00 | (A6) | 0.10 | (D1) | 62.93 | (D2) | 26.97 |
| UDL-44 | (C1) | 10.00 | (A7) | 0.10 | (D1) | 62.93 | (D2) | 26.97 |
| UDL-45 | (C1) | 10.00 | (A26) | 0.10 | (D1) | 62.93 | (D2) | 26.97 |
| UDL-46 | (C1) | 10.00 | (A31) | 0.10 | (D1) | 62.93 | (D2) | 26.97 |
| UDL-47 | (C1) | 10.00 | (A40) | 0.10 | (D1) | 62.93 | (D2) | 26.97 |
| UDL-48 | (C1) | 10.00 | (A7) | 1.000 | (D1) | 89.00 | | |
| UDL-49 | (C1) | 10.00 | (A7) | 0.500 | (D1) | 89.50 | | |
| UDL-50 | (C1) | 10.00 | (A7) | 0.001 | (D1) | 90.00 | | |
| UDL-51 | (C2) | 10.00 | (A1) | 0.10 | (D1) | 89.90 | | |
| UDL-52 | (C2) | 10.00 | (A6) | 0.10 | (D1) | 89.90 | | |
| UDL-53 | (C2) | 10.00 | (A7) | 0.10 | (D1) | 89.90 | | |
| UDL-54 | (C2) | 10.00 | (A26) | 0.10 | (D1) | 89.90 | | |
| UDL-55 | (C2) | 10.00 | (A31) | 0.10 | (D1) | 89.90 | | |
| UDL-56 | (C2) | 10.00 | (A40) | 0.10 | (D1) | 89.90 | | |
| UDL-57 | (C3) | 10.00 | (A1) | 0.10 | (D1) | 89.90 | | |
| UDL-58 | (C3) | 10.00 | (A6) | 0.10 | (D1) | 89.90 | | |
| UDL-59 | (C3) | 10.00 | (A7) | 0.10 | (D1) | 89.90 | | |
| UDL-60 | (C3) | 10.00 | (A26) | 0.10 | (D1) | 89.90 | | |
| UDL-61 | (C3) | 10.00 | (A31) | 0.10 | (D1) | 89.90 | | |
| UDL-62 | (C3) | 10.00 | (A40) | 0.10 | (D1) | 89.90 | | |
| UDL-63 | (C4) | 10.00 | (A1) | 0.10 | (D1) | 89.90 | | |
| UDL-64 | (C4) | 10.00 | (A6) | 0.10 | (D1) | 89.90 | | |
| UDL-65 | (C4) | 10.00 | (A7) | 0.10 | (D1) | 89.90 | | |
| UDL-66 | (C4) | 10.00 | (A26) | 0.10 | (D1) | 89.90 | | |
| UDL-67 | (C4) | 10.00 | (A31) | 0.10 | (D1) | 89.90 | | |
| UDL-68 | (C4) | 10.00 | (A40) | 0.10 | (D1) | 89.90 | | |
| UDL-69 | (C5) | 10.00 | (A1) | 0.10 | (D1) | 89.90 | | |
| UDL-70 | (C5) | 10.00 | (A6) | 0.10 | (D1) | 89.90 | | |
| UDL-71 | (C5) | 10.00 | (A7) | 0.10 | (D1) | 89.90 | | |
| UDL-72 | (C5) | 10.00 | (A26) | 0.10 | (D1) | 89.90 | | |
| UDL-73 | (C5) | 10.00 | (A31) | 0.10 | (D1) | 89.90 | | |
| UDL-74 | (C5) | 10.00 | (A40) | 0.10 | (D1) | 89.90 | | |
| UDL-75 | (C6) | 10.00 | (A1) | 0.10 | (D1) | 89.90 | | |
| UDL-76 | (C6) | 10.00 | (A6) | 0.10 | (D1) | 89.90 | | |
| UDL-77 | (C6) | 10.00 | (A7) | 0.10 | (D1) | 89.90 | | |
| UDL-78 | (C6) | 10.00 | (A26) | 0.10 | (D1) | 89.90 | | |
| UDL-79 | (C6) | 10.00 | (A31) | 0.10 | (D1) | 89.90 | | |
| UDL-80 | (C6) | 10.00 | (A40) | 0.10 | (D1) | 89.90 | | |

**[Table 1-3]**

| Composition for forming organic film | Resin for forming organic film | | Compound | | Solvent 1 | | Solvent 2 | |
|---|---|---|---|---|---|---|---|---|
| | Type | Number of parts by mass | Type | Number of parts by mass | Type | Number of parts by mass | Type | Number of parts by mass |
| UDL-81 | (C1) | 25.00 | (A1) | 0.25 | (D1) | 74.75 | | |
| UDL-82 | (C1) | 25.00 | (A2) | 0.25 | (D1) | 74.75 | | |
| UDL-83 | (C1) | 25.00 | (A3) | 0.25 | (D1) | 74.75 | | |
| UDL-84 | (C1) | 25.00 | (A4) | 0.25 | (D1) | 74.75 | | |
| UDL-85 | (C1) | 25.00 | (A5) | 0.25 | (D1) | 74.75 | | |
| UDL-86 | (C1) | 25.00 | (A6) | 0.25 | (D1) | 74.75 | | |
| UDL-87 | (C1) | 25.00 | (A7) | 0.25 | (D1) | 74.75 | | |
| UDL-88 | (C1) | 25.00 | (A8) | 0.25 | (D1) | 74.75 | | |
| UDL-89 | (C1) | 25.00 | (A9) | 0.25 | (D1) | 74.75 | | |
| UDL-90 | (C1) | 25.00 | (A10) | 0.25 | (D1) | 74.75 | | |
| UDL-91 | (C1) | 25.00 | (A11) | 0.25 | (D1) | 74.75 | | |
| UDL-92 | (C1) | 25.00 | (A12) | 0.25 | (D1) | 74.75 | | |
| UDL-93 | (C1) | 25.00 | (A13) | 0.25 | (D1) | 74.75 | | |
| UDL-94 | (C1) | 25.00 | (A14) | 0.25 | (D1) | 74.75 | | |
| UDL-95 | (C1) | 25.00 | (A15) | 0.25 | (D1) | 74.75 | | |
| UDL-96 | (C1) | 25.00 | (A16) | 0.25 | (D1) | 74.75 | | |
| UDL-97 | (C1) | 25.00 | (A17) | 0.25 | (D1) | 74.75 | | |
| UDL-98 | (C1) | 25.00 | (A18) | 0.25 | (D1) | 74.75 | | |
| UDL-99 | (C1) | 25.00 | (A19) | 0.25 | (D1) | 74.75 | | |
| UDL-100 | (C1) | 25.00 | (A20) | 0.25 | (D1) | 74.75 | | |
| UDL-101 | (C1) | 25.00 | (A21) | 0.25 | (D1) | 74.75 | | |
| UDL-102 | (C1) | 25.00 | (A22) | 0.25 | (D1) | 74.75 | | |
| UDL-103 | (C1) | 25.00 | (A23) | 0.25 | (D1) | 74.75 | | |
| UDL-104 | (C1) | 25.00 | (A24) | 0.25 | (D1) | 74.75 | | |
| UDL-105 | (C1) | 25.00 | (A25) | 0.25 | (D1) | 74.75 | | |
| UDL-106 | (C1) | 25.00 | (A26) | 0.25 | (D1) | 74.75 | | |
| UDL-107 | (C1) | 25.00 | (A27) | 0.25 | (D1) | 74.75 | | |
| UDL-108 | (C1) | 25.00 | (A28) | 0.25 | (D1) | 74.75 | | |
| UDL-109 | (C1) | 25.00 | (A29) | 0.25 | (D1) | 74.75 | | |
| UDL-110 | (C1) | 25.00 | (A30) | 0.25 | (D1) | 74.75 | | |
| UDL-111 | (C1) | 25.00 | (A31) | 0.25 | (D1) | 74.75 | | |
| UDL-112 | (C1) | 25.00 | (A32) | 0.25 | (D1) | 74.75 | | |
| UDL-113 | (C1) | 25.00 | (A33) | 0.25 | (D1) | 74.75 | | |
| UDL-114 | (C1) | 25.00 | (A34) | 0.25 | (D1) | 74.75 | | |
| UDL-115 | (C1) | 25.00 | (A35) | 0.25 | (D1) | 74.75 | | |
| UDL-116 | (C1) | 25.00 | (A36) | 0.25 | (D1) | 74.75 | | |
| UDL-117 | (C1) | 25.00 | (A37) | 0.25 | (D1) | 74.75 | | |
| UDL-118 | (C1) | 25.00 | (A38) | 0.25 | (D1) | 74.75 | | |
| UDL-119 | (C1) | 25.00 | (A39) | 0.25 | (D1) | 74.75 | | |
| UDL-120 | (C1) | 25.00 | (A40) | 0.25 | (D1) | 74.75 | | |
| UDL-121 | (C1) | 25.00 | (A41) | 0.25 | (D1) | 74.75 | | |

**[Table 1-4]**

| Composition for forming organic film | Resin for forming organic film | | Compound | | Solvent 1 | | Solvent 2 | |
|---|---|---|---|---|---|---|---|---|
| | Type | Number of parts by mass | Type | Number of parts by mass | Type | Number of parts by mass | Type | Number of parts by mass |
| Comparative UDL-1 | (C1) | 10.00 | (R1) | 0.10 | (D1) | 89.90 | | |
| Comparative UDL-2 | (C2) | 10.00 | (R1) | 0.10 | (D1) | 89.90 | | |
| Comparative UDL-3 | (C3) | 10.00 | (R1) | 0.10 | (D1) | 89.90 | | |
| Comparative UDL-4 | (C4) | 10.00 | (R1) | 0.10 | (D1) | 89.90 | | |
| Comparative UDL-5 | (C5) | 10.00 | (R1) | 0.10 | (D1) | 89.90 | | |
| Comparative UDL-6 | (C6) | 10.00 | (R1) | 0.10 | (D1) | 89.90 | | |
| Comparative UDL-7 | (C1) | 10.00 | (R2) | 0.10 | (D1) | 89.90 | | |
| Comparative UDL-8 | (C1) | 10.00 | (R3) | 0.10 | (D1) | 89.90 | | |
| Comparative UDL-9 | (C1) | 10.00 | (R4) | 0.10 | (D1) | 89.90 | | |
| Comparative UDL-10 | (C1) | 10.00 | (R5) | 0.10 | (D1) | 89.90 | | |
| Comparative UDL-11 | (C1) | 10.00 | | | (D1) | 90.00 | | |
| Comparative UDL-12 | (C2) | 10.00 | | | (D1) | 90.00 | | |
| Comparative UDL-13 | (C3) | 10.00 | | | (D1) | 90.00 | | |
| Comparative UDL-14 | (C4) | 10.00 | | | (D1) | 90.00 | | |
| Comparative UDL-15 | (C5) | 10.00 | | | (D1) | 90.00 | | |
| Comparative UDL-16 | (C6) | 10.00 | | | (D1) | 90.00 | | |

### [Fabrication of silicon wafers on which organic cured film is formed using composition for forming organic film (UDL-1 to 121 and comparative UDL-1 to 16)]

2 ml of each of the compositions for forming an organic film prepared above (UDL-1 to 121 and comparative UDL-1 to 16) was ejected to the center of a 300 mm silicon wafer using a coater/developer "CLEAN TRACK LITHIUS Pro AP" from Tokyo Electron Limited, then, rotated at a rotation speed at which the average film thickness became as shown in Tables 2-1 to 2-4 after baking, and spread, thereby forming a film. While the silicon wafer was rotated at a speed of 1000 rpm, a removal liquid (a mixed liquid of propylene glycol monomethyl ether acetate and propylene glycol monomethyl ether (30:70, mass ratio)) was ejected from a removal liquid ejection nozzle in an ejection amount of 2 mL/s and moved from the outer peripheral portion of the silicon wafer toward the central portion up to a position of 3 mm at a speed of 5 mm/s, and the removal liquid was further ejected in an ejection amount of 2 mL/s for five seconds at the position. After that, the ejection of the removal liquid was stopped, and the silicon wafer was further rotated at a speed of 1000 rpm for 30 seconds. Next, the silicon wafer on which a film of the composition for forming an organic film had been formed was heated at 350°C for 60 seconds, thereby obtaining a silicon wafer having an organic cured film formed thereon.

### [Solvent resistance evaluation: Examples 1-1 to 1-121 and Comparative Examples 1-1 to 1-16]

A film of each of the compositions for forming an organic film (UDL-1 to 121 and comparative UDL-1 to 16) was formed on a silicon wafer by the above-described method, the film thickness was measured, a PGMEA solvent was dispensed thereon, left to stand for 30 seconds, spin-dried, and baked at 100°C for 60 seconds to evaporate PGMEA, and the film thickness was measured. The film thickness before the PGMEA solvent was dispensed was represented by X, the film thickness after the PGMEA solvent was dispensed was represented by X₁, and the absolute value of a value obtained by (X₁ - X)/X × 100 was regarded as the film thickness change rate (%). In a case where the film thickness change rate was less than 0.5%, the solvent resistance was evaluated as favorable, and in the case of 0.5% or more, the solvent resistance was evaluated as poor.

### [In-plane uniformity evaluation: Examples 1-1 to 1-121 and Comparative Examples 1-1 to 1-16]

A film of each of the compositions for forming an organic film (UDL-1 to 121 and comparative UDL-1 to 16) was formed on a silicon wafer by the above-described method, and the film thickness of an organic cured film was measured with an optical film thickness meter at 225 points on concentric circles within a range of a radius of 145 mm from the wafer center. The maximum value of the film thicknesses was represented by Xₘₐₓ, the minimum value was represented by Xₘᵢₙ, the average value was represented by X_{average}, and a value obtained by (Xₘₐₓ - Xₘᵢₙ)/X_{average} was regarded as in-plane uniformity (%). In a case where the value was less than 2%, the in-plane uniformity was evaluated as A (favorable), in the case of 2% or more and less than 3%, the in-plane uniformity was evaluated as B, and in the case of 3% or more, the in-plane uniformity was evaluated as C (poor).

**[Table 2-1]**

| | Composition for forming organic film | Average film thickness | Solvent resistance | In-plane uniformity |
|---|---|---|---|---|
| Example 1-1 | UDL-1 | 500 nm | Favorable | B |
| Example 1-2 | UDL-2 | 500 nm | Favorable | A |
| Example 1-3 | UDL-3 | 500 nm | Favorable | A |
| Example 1-4 | UDL-4 | 500 nm | Favorable | A |
| Example 1-5 | UDL-5 | 500 nm | Favorable | A |
| Example 1-6 | UDL-6 | 500 nm | Favorable | A |
| Example 1-7 | UDL-7 | 500 nm | Favorable | A |
| Example 1-8 | UDL-8 | 500 nm | Favorable | A |
| Example 1-9 | UDL-9 | 500 nm | Favorable | B |
| Example 1-10 | UDL-10 | 500 nm | Favorable | A |
| Example 1-11 | UDL-11 | 500 nm | Favorable | A |
| Example 1-12 | UDL-12 | 500 nm | Favorable | A |
| Example 1-13 | UDL-13 | 500 nm | Favorable | A |
| Example 1-14 | UDL-14 | 500 nm | Favorable | A |
| Example 1-15 | UDL-15 | 500 nm | Favorable | A |
| Example 1-16 | UDL-16 | 500 nm | Favorable | A |
| Example 1-17 | UDL-17 | 500 nm | Favorable | A |
| Example 1-18 | UDL-18 | 500 nm | Favorable | A |
| Example 1-19 | UDL-19 | 500 nm | Favorable | A |
| Example 1-20 | UDL-20 | 500 nm | Favorable | A |
| Example 1-21 | UDL-21 | 500 nm | Favorable | A |
| Example 1-22 | UDL-22 | 500 nm | Favorable | A |
| Example 1-23 | UDL-23 | 500 nm | Favorable | A |
| Example 1-24 | UDL-24 | 500 nm | Favorable | A |
| Example 1-25 | UDL-25 | 500 nm | Favorable | A |
| Example 1-26 | UDL-26 | 500 nm | Favorable | A |
| Example 1-27 | UDL-27 | 500 nm | Favorable | A |
| Example 1-28 | UDL-28 | 500 nm | Favorable | A |
| Example 1-29 | UDL-29 | 500 nm | Favorable | A |
| Example 1-30 | UDL-30 | 500 nm | Favorable | A |
| Example 1-31 | UDL-31 | 500 nm | Favorable | A |
| Example 1-32 | UDL-32 | 500 nm | Favorable | A |
| Example 1-33 | UDL-33 | 500 nm | Favorable | A |
| Example 1-34 | UDL-34 | 500 nm | Favorable | A |
| Example 1-35 | UDL-35 | 500 nm | Favorable | A |
| Example 1-36 | UDL-36 | 500 nm | Favorable | A |
| Example 1-37 | UDL-37 | 500 nm | Favorable | A |
| Example 1-38 | UDL-38 | 500 nm | Favorable | A |
| Example 1-39 | UDL-39 | 500 nm | Favorable | A |
| Example 1-40 | UDL-40 | 500 nm | Favorable | A |

**[Table 2-2]**

| | Composition for forming organic film | Average film thickness | Solvent resistance | In-plane uniformity |
|---|---|---|---|---|
| Example 1-41 | UDL-41 | 500 nm | Favorable | A |
| Example 1-42 | UDL-42 | 500 nm | Favorable | B |
| Example 1-43 | UDL-43 | 500 nm | Favorable | A |
| Example 1-44 | UDL-44 | 500 nm | Favorable | A |
| Example 1-45 | UDL-45 | 500 nm | Favorable | A |
| Example 1-46 | UDL-46 | 500 nm | Favorable | A |
| Example 1-47 | UDL-47 | 500 nm | Favorable | A |
| Example 1-48 | UDL-48 | 500 nm | Favorable | B |
| Example 1-49 | UDL-49 | 500 nm | Favorable | A |
| Example 1-50 | UDL-50 | 500 nm | Favorable | B |
| Example 1-51 | UDL-51 | 500 nm | Favorable | B |
| Example 1-52 | UDL-52 | 500 nm | Favorable | A |
| Example 1-53 | UDL-53 | 500 nm | Favorable | A |
| Example 1-54 | UDL-54 | 500 nm | Favorable | A |
| Example 1-55 | UDL-55 | 500 nm | Favorable | A |
| Example 1-56 | UDL-56 | 500 nm | Favorable | A |
| Example 1-57 | UDL-57 | 500 nm | Favorable | B |
| Example 1-58 | UDL-58 | 500 nm | Favorable | A |
| Example 1-59 | UDL-59 | 500 nm | Favorable | A |
| Example 1-60 | UDL-60 | 500 nm | Favorable | A |
| Example 1-61 | UDL-61 | 500 nm | Favorable | A |
| Example 1-62 | UDL-62 | 500 nm | Favorable | A |
| Example 1-63 | UDL-63 | 500 nm | Favorable | B |
| Example 1-64 | UDL-64 | 500 nm | Favorable | A |
| Example 1-65 | UDL-65 | 500 nm | Favorable | A |
| Example 1-66 | UDL-66 | 500 nm | Favorable | A |
| Example 1-67 | UDL-67 | 500 nm | Favorable | A |
| Example 1-68 | UDL-68 | 500 nm | Favorable | A |
| Example 1-69 | UDL-69 | 500 nm | Favorable | B |
| Example 1-70 | UDL-70 | 500 nm | Favorable | A |
| Example 1-71 | UDL-71 | 500 nm | Favorable | A |
| Example 1-72 | UDL-72 | 500 nm | Favorable | A |
| Example 1-73 | UDL-73 | 500 nm | Favorable | A |
| Example 1-74 | UDL-74 | 500 nm | Favorable | A |
| Example 1-75 | UDL-75 | 500 nm | Favorable | B |
| Example 1-76 | UDL-76 | 500 nm | Favorable | A |
| Example 1-77 | UDL-77 | 500 nm | Favorable | A |
| Example 1-78 | UDL-78 | 500 nm | Favorable | A |
| Example 1-79 | UDL-79 | 500 nm | Favorable | A |
| Example 1-80 | UDL-80 | 500 nm | Favorable | A |

**[Table 2-3]**

| | Composition for forming organic film | Average film thickness | Solvent resistance | In-plane uniformity |
|---|---|---|---|---|
| Example 1-81 | UDL-81 | 1000 nm | Favorable | B |
| Example 1-82 | UDL-82 | 1000 nm | Favorable | B |
| Example 1-83 | UDL-83 | 1000 nm | Favorable | B |
| Example 1-84 | UDL-84 | 1000 nm | Favorable | B |
| Example 1-85 | UDL-85 | 1000 nm | Favorable | B |
| Example 1-86 | UDL-86 | 1000 nm | Favorable | B |
| Example 1-87 | UDL-87 | 1000 nm | Favorable | A |
| Example 1-88 | UDL-88 | 1000 nm | Favorable | B |
| Example 1-89 | UDL-89 | 1000 nm | Favorable | B |
| Example 1-90 | UDL-90 | 1000 nm | Favorable | B |
| Example 1-91 | UDL-91 | 1000 nm | Favorable | B |
| Example 1-92 | UDL-92 | 1000 nm | Favorable | B |
| Example 1-93 | UDL-93 | 1000 nm | Favorable | A |
| Example 1-94 | UDL-94 | 1000 nm | Favorable | B |
| Example 1-95 | UDL-95 | 1000 nm | Favorable | B |
| Example 1-96 | UDL-96 | 1000 nm | Favorable | B |
| Example 1-97 | UDL-97 | 1000 nm | Favorable | A |
| Example 1-98 | UDL-98 | 1000 nm | Favorable | B |
| Example 1-99 | UDL-99 | 1000 nm | Favorable | B |
| Example 1-100 | UDL-100 | 1000 nm | Favorable | B |
| Example 1-101 | UDL-101 | 1000 nm | Favorable | B |
| Example 1-102 | UDL-102 | 1000 nm | Favorable | B |
| Example 1-103 | UDL-103 | 1000 nm | Favorable | B |
| Example 1-104 | UDL-104 | 1000 nm | Favorable | A |
| Example 1-105 | UDL-105 | 1000 nm | Favorable | B |
| Example 1-106 | UDL-106 | 1000 nm | Favorable | A |
| Example 1-107 | UDL-107 | 1000 nm | Favorable | B |
| Example 1-108 | UDL-108 | 1000 nm | Favorable | B |
| Example 1-109 | UDL-109 | 1000 nm | Favorable | B |
| Example 1-110 | UDL-110 | 1000 nm | Favorable | B |
| Example 1-111 | UDL-111 | 1000 nm | Favorable | A |
| Example 1-112 | UDL-112 | 1000 nm | Favorable | B |
| Example 1-113 | UDL-113 | 1000 nm | Favorable | A |
| Example 1-114 | UDL-114 | 1000 nm | Favorable | B |
| Example 1-115 | UDL-115 | 1000 nm | Favorable | B |
| Example 1-116 | UDL-116 | 1000 nm | Favorable | B |
| Example 1-117 | UDL-117 | 1000 nm | Favorable | B |
| Example 1-118 | UDL-118 | 1000 nm | Favorable | B |
| Example 1-119 | UDL-119 | 1000 nm | Favorable | B |
| Example 1-120 | UDL-120 | 1000 nm | Favorable | A |
| Example 1-121 | UDL-121 | 1000 nm | Favorable | B |

**[Table 2-4]**

| | Composition for forming organic film | Average film thickness | Solvent resistance | In-plane uniformity |
|---|---|---|---|---|
| Comparative Example 1-1 | Comparative UDL-1 | 500 nm | Favorable | A |
| Comparative Example 1-2 | Comparative UDL-2 | 500 nm | Favorable | A |
| Comparative Example 1-3 | Comparative UDL-3 | 500 nm | Favorable | A |
| Comparative Example 1-4 | Comparative UDL-4 | 500 nm | Favorable | A |
| Comparative Example 1-5 | Comparative UDL-5 | 500 nm | Favorable | A |
| Comparative Example 1-6 | Comparative UDL-6 | 500 nm | Favorable | A |
| Comparative Example 1-7 | Comparative UDL-7 | 500 nm | Favorable | A |
| Comparative Example 1-8 | Comparative UDL-8 | 500 nm | Favorable | A |
| Comparative Example 1-9 | Comparative UDL-9 | 500 nm | Favorable | A |
| Comparative Example 1-10 | Comparative UDL-10 | 500 nm | Favorable | A |
| Comparative Example 1-11 | Comparative UDL-11 | 500 nm | Favorable | C |
| Comparative Example 1-12 | Comparative UDL-12 | 500 nm | Favorable | C |
| Comparative Example 1-13 | Comparative UDL-13 | 500 nm | Favorable | C |
| Comparative Example 1-14 | Comparative UDL-14 | 500 nm | Favorable | C |
| Comparative Example 1-15 | Comparative UDL-15 | 500 nm | Favorable | C |
| Comparative Example 1-16 | Comparative UDL-16 | 500 nm | Favorable | C |

### [Hump suppression properties evaluation: Examples 2-1 to 2-121 and Comparative Examples 2-1 to 2-10]

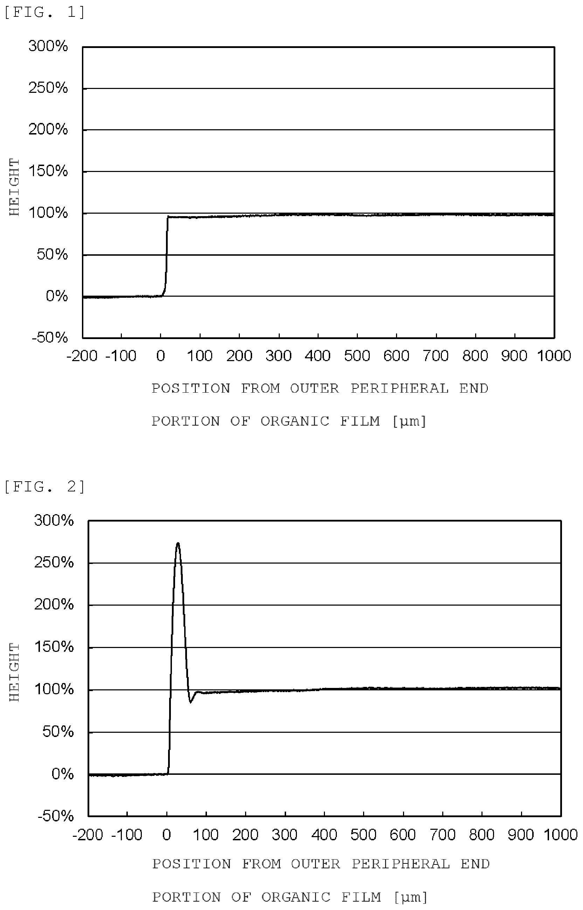

A film of each of the compositions for forming an organic film (UDL-1 to 121 and comparative UDL-1 to 10) was formed on a silicon wafer by the above-described method, and the height change from the outer peripheral end portion of an organic film up to the position of 1000 µm toward the silicon wafer center was measured using Alpha-Step D-600 (contact-type profiler) manufactured by KLA-TENCOR Corporation. When the height of the silicon wafer was set to zero, in a case where the maximum height was less than 110% of the film thickness as shown in FIG. 1, the hump suppression properties were evaluated as favorable, and in a case where a region with the height being 150% or more was generated as shown in FIG. 2, the hump suppression properties were evaluated as poor.

### [Embedding characteristics evaluation-1: Examples 2-1 to 2-121 and Comparative Examples 2-1 to 2-10]

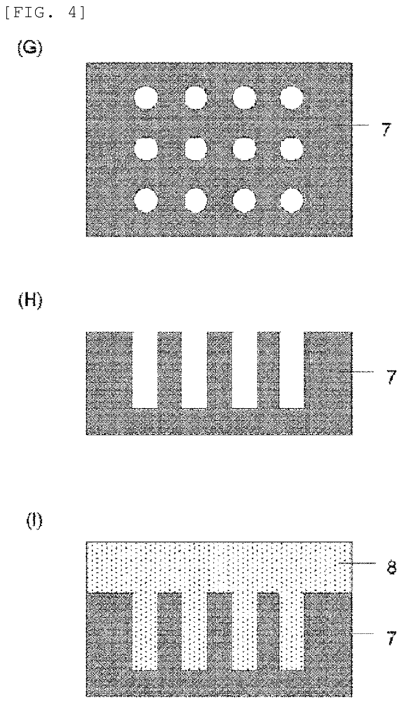

As shown in FIG. 4, a film of each of the compositions for forming an organic film (UDL-1 to 121 and comparative UDL-1 to 10) was formed on a SiO₂ wafer substrate having a dense hole pattern (hole diameter: 0.2 µm, hole depth: 1.0 µm, distance between the centers of two adjacent holes: 0.4 µm) by the above-described method, and a resist lower layer film 8 was formed. The substrate used was a base substrate (SiO₂ wafer substrate) 7 having a dense hole pattern as shown in FIG. 4(G) (bird's-eye view) and FIG. 4(H) (cross-sectional view). The cross-sectional shape of each wafer substrate obtained was observed using a scanning electron microscope (SEM), and whether or not the insides of the holes were filled with the resist lower layer film with no voids was confirmed. In the case of using a resist lower layer film material having poor embedding characteristics, voids are generated in the insides of the holes. In the case of using a resist lower layer film material having favorable embedding characteristics, in the present evaluation, the insides of the holes were filled with the resist lower layer film with no voids as shown in FIG. 4(I). In a case where no voids were generated, the embedding characteristics were evaluated as favorable, and in a case where voids were generated, the embedding characteristics were evaluated as poor.

### [Embedding characteristics evaluation-2: Examples 2-81 to 2-121]

As shown in FIG. 4, a film of each of the compositions for forming an organic film (UDL-81 to 121) was formed on a SiO₂ wafer substrate having a dense hole pattern (hole diameter: 0.2 µm, hole depth: 2.0 µm, distance between the centers of two adjacent holes: 0.4 µm) by the above-described method, and a resist lower layer film 8 was formed. The substrate used was a base substrate (SiO₂ wafer substrate) 7 having a dense hole pattern as shown in FIG. 4(G) (bird's-eye view) and FIG. 4(H) (cross-sectional view). The cross-sectional shape of each wafer substrate obtained was observed using a scanning electron microscope (SEM), and whether or not the insides of the holes were filled with the resist lower layer film with no voids was confirmed. In the case of using a resist lower layer film material having poor embedding characteristics, voids are generated in the insides of the holes. In the case of using a resist lower layer film material having favorable embedding characteristics, in the present evaluation, the insides of the holes were filled with the resist lower layer film with no voids as shown in FIG. 4(I). In a case where no voids were generated, the embedding characteristics were evaluated as favorable, and in a case where voids were generated, the embedding characteristics were evaluated as poor. In the present evaluation, in order to evaluate the superiority or inferiority of the embedding performance, the evaluation conditions were so strict that voids were more easily generated than in the embedding characteristics evaluation-1.

**[Table 3-1]**

| | Composition for forming organic film | Hump suppression properties evaluation | Embedding characteristics evaluation-1 | Embedding characteristics evaluation-2 |
|---|---|---|---|---|
| Example 2-1 | UDL-1 | Favorable | Favorable | - |
| Example 2-2 | UDL-2 | Favorable | Favorable | - |
| Example 2-3 | UDL-3 | Favorable | Favorable | - |
| Example 2-4 | UDL-4 | Favorable | Favorable | - |
| Example 2-5 | UDL-5 | Favorable | Favorable | - |
| Example 2-6 | UDL-6 | Favorable | Favorable | - |
| Example 2-7 | UDL-7 | Favorable | Favorable | - |
| Example 2-8 | UDL-8 | Favorable | Favorable | - |
| Example 2-9 | UDL-9 | Favorable | Favorable | - |
| Example 2-10 | UDL-10 | Favorable | Favorable | - |
| Example 2-11 | UDL-11 | Favorable | Favorable | - |
| Example 2-12 | UDL-12 | Favorable | Favorable | - |
| Example 2-13 | UDL-13 | Favorable | Favorable | - |
| Example 2-14 | UDL-14 | Favorable | Favorable | - |
| Example 2-15 | UDL-15 | Favorable | Favorable | - |
| Example 2-16 | UDL-16 | Favorable | Favorable | - |
| Example 2-17 | UDL-17 | Favorable | Favorable | - |
| Example 2-18 | UDL-18 | Favorable | Favorable | - |
| Example 2-19 | UDL-19 | Favorable | Favorable | - |
| Example 2-20 | UDL-20 | Favorable | Favorable | - |
| Example 2-21 | UDL-21 | Favorable | Favorable | - |
| Example 2-22 | UDL-22 | Favorable | Favorable | - |
| Example 2-23 | UDL-23 | Favorable | Favorable | - |
| Example 2-24 | UDL-24 | Favorable | Favorable | - |
| Example 2-25 | UDL-25 | Favorable | Favorable | - |
| Example 2-26 | UDL-26 | Favorable | Favorable | - |
| Example 2-27 | UDL-27 | Favorable | Favorable | - |
| Example 2-28 | UDL-28 | Favorable | Favorable | - |
| Example 2-29 | UDL-29 | Favorable | Favorable | - |
| Example 2-30 | UDL-30 | Favorable | Favorable | - |
| Example 2-31 | UDL-31 | Favorable | Favorable | - |
| Example 2-32 | UDL-32 | Favorable | Favorable | - |
| Example 2-33 | UDL-33 | Favorable | Favorable | - |
| Example 2-34 | UDL-34 | Favorable | Favorable | - |
| Example 2-35 | UDL-35 | Favorable | Favorable | - |
| Example 2-36 | UDL-36 | Favorable | Favorable | - |
| Example 2-37 | UDL-37 | Favorable | Favorable | - |
| Example 2-38 | UDL-38 | Favorable | Favorable | - |
| Example 2-39 | UDL-39 | Favorable | Favorable | - |
| Example 2-40 | UDL-40 | Favorable | Favorable | - |

**[Table 3-2]**

| | Composition for forming organic film | Hump suppression properties evaluation | Embedding characteristics evaluation-1 | Embedding characteristics evaluation-2 |
|---|---|---|---|---|
| Example 2-41 | UDL-41 | Favorable | Favorable | - |
| Example 2-42 | UDL-42 | Favorable | Favorable | - |
| Example 2-43 | UDL-43 | Favorable | Favorable | - |
| Example 2-44 | UDL-44 | Favorable | Favorable | - |
| Example 2-45 | UDL-45 | Favorable | Favorable | - |
| Example 2-46 | UDL-46 | Favorable | Favorable | - |
| Example 2-47 | UDL-47 | Favorable | Favorable | - |
| Example 2-48 | UDL-48 | Favorable | Favorable | - |
| Example 2-49 | UDL-49 | Favorable | Favorable | - |
| Example 2-50 | UDL-50 | Favorable | Favorable | - |
| Example 2-51 | UDL-51 | Favorable | Favorable | - |
| Example 2-52 | UDL-52 | Favorable | Favorable | - |
| Example 2-53 | UDL-53 | Favorable | Favorable | - |
| Example 2-54 | UDL-54 | Favorable | Favorable | - |
| Example 2-55 | UDL-55 | Favorable | Favorable | - |
| Example 2-56 | UDL-56 | Favorable | Favorable | - |
| Example 2-57 | UDL-57 | Favorable | Favorable | - |
| Example 2-58 | UDL-58 | Favorable | Favorable | - |
| Example 2-59 | UDL-59 | Favorable | Favorable | - |
| Example 2-60 | UDL-60 | Favorable | Favorable | - |
| Example 2-61 | UDL-61 | Favorable | Favorable | - |
| Example 2-62 | UDL-62 | Favorable | Favorable | - |
| Example 2-63 | UDL-63 | Favorable | Favorable | - |
| Example 2-64 | UDL-64 | Favorable | Favorable | - |
| Example 2-65 | UDL-65 | Favorable | Favorable | - |
| Example 2-66 | UDL-66 | Favorable | Favorable | - |
| Example 2-67 | UDL-67 | Favorable | Favorable | - |
| Example 2-68 | UDL-68 | Favorable | Favorable | - |
| Example 2-69 | UDL-69 | Favorable | Favorable | - |
| Example 2-70 | UDL-70 | Favorable | Favorable | - |
| Example 2-71 | UDL-71 | Favorable | Favorable | - |
| Example 2-72 | UDL-72 | Favorable | Favorable | - |
| Example 2-73 | UDL-73 | Favorable | Favorable | - |
| Example 2-74 | UDL-74 | Favorable | Favorable | - |
| Example 2-75 | UDL-75 | Favorable | Favorable | - |
| Example 2-76 | UDL-76 | Favorable | Favorable | - |
| Example 2-77 | UDL-77 | Favorable | Favorable | - |
| Example 2-78 | UDL-78 | Favorable | Favorable | - |
| Example 2-79 | UDL-79 | Favorable | Favorable | - |
| Example 2-80 | UDL-80 | Favorable | Favorable | - |

**[Table 3-3]**

| | Composition for forming organic film | Hump suppression properties evaluation | Embedding characteristics evaluation-1 | Embedding characteristics evaluation-2 |
|---|---|---|---|---|
| Example 2-81 | UDL-81 | Favorable | Favorable | Poor |
| Example 2-82 | UDL-82 | Favorable | Favorable | Poor |
| Example 2-83 | UDL-83 | Favorable | Favorable | Poor |
| Example 2-84 | UDL-84 | Favorable | Favorable | Poor |
| Example 2-85 | UDL-85 | Favorable | Favorable | Poor |
| Example 2-86 | UDL-86 | Favorable | Favorable | Poor |
| Example 2-87 | UDL-87 | Favorable | Favorable | Favorable |
| Example 2-88 | UDL-88 | Favorable | Favorable | Poor |
| Example 2-89 | UDL-89 | Favorable | Favorable | Poor |
| Example 2-90 | UDL-90 | Favorable | Favorable | Poor |
| Example 2-91 | UDL-91 | Favorable | Favorable | Poor |
| Example 2-92 | UDL-92 | Favorable | Favorable | Poor |
| Example 2-93 | UDL-93 | Favorable | Favorable | Favorable |
| Example 2-94 | UDL-94 | Favorable | Favorable | Poor |
| Example 2-95 | UDL-95 | Favorable | Favorable | Favorable |
| Example 2-96 | UDL-96 | Favorable | Favorable | Favorable |
| Example 2-97 | UDL-97 | Favorable | Favorable | Favorable |
| Example 2-98 | UDL-98 | Favorable | Favorable | Poor |
| Example 2-99 | UDL-99 | Favorable | Favorable | Favorable |
| Example 2-100 | UDL-100 | Favorable | Favorable | Favorable |
| Example 2-101 | UDL-101 | Favorable | Favorable | Poor |
| Example 2-102 | UDL-102 | Favorable | Favorable | Poor |
| Example 2-103 | UDL-103 | Favorable | Favorable | Poor |
| Example 2-104 | UDL-104 | Favorable | Favorable | Poor |
| Example 2-105 | UDL-105 | Favorable | Favorable | Poor |
| Example 2-106 | UDL-106 | Favorable | Favorable | Favorable |
| Example 2-107 | UDL-107 | Favorable | Favorable | Poor |
| Example 2-108 | UDL-108 | Favorable | Favorable | Poor |
| Example 2-109 | UDL-109 | Favorable | Favorable | Poor |
| Example 2-110 | UDL-110 | Favorable | Favorable | Poor |
| Example 2-111 | UDL-111 | Favorable | Favorable | Favorable |
| Example 2-112 | UDL-112 | Favorable | Favorable | Poor |
| Example 2-113 | UDL-113 | Favorable | Favorable | Favorable |
| Example 2-114 | UDL-114 | Favorable | Favorable | Poor |
| Example 2-115 | UDL-115 | Favorable | Favorable | Poor |
| Example 2-116 | UDL-116 | Favorable | Favorable | Poor |
| Example 2-117 | UDL-117 | Favorable | Favorable | Poor |
| Example 2-118 | UDL-118 | Favorable | Favorable | Poor |
| Example 2-119 | UDL-119 | Favorable | Favorable | Poor |
| Example 2-120 | UDL-120 | Favorable | Favorable | Favorable |
| Example 2-121 | UDL-121 | Favorable | Favorable | Favorable |

**[Table 3-4]**

| | Composition for forming organic film | Hump suppression properties evaluation | Embedding characteristics evaluation-1 | Embedding characteristics evaluation-2 |
|---|---|---|---|---|
| Comparative Example 2-1 | Comparative UDL-1 | Poor | Poor | - |
| Comparative Example 2-2 | Comparative UDL-2 | Poor | Poor | - |
| Comparative Example 2-3 | Comparative UDL-3 | Poor | Poor | - |
| Comparative Example 2-4 | Comparative UDL-4 | Poor | Poor | - |
| Comparative Example 2-5 | Comparative UDL-5 | Poor | Poor | - |
| Comparative Example 2-6 | Comparative UDL-6 | Poor | Poor | - |
| Comparative Example 2-7 | Comparative UDL-7 | Poor | Poor | - |
| Comparative Example 2-8 | Comparative UDL-8 | Poor | Poor | - |
| Comparative Example 2-9 | Comparative UDL-9 | Poor | Poor | - |
| Comparative Example 2-10 | Comparative UDL-10 | Favorable | Poor | - |

As shown in Tables 2-1 to 2-4 and Tables 3-1 to 3-4, it was confirmed that the compositions for forming an organic film (UDL-1 to 121) of the present invention were excellent in terms of solvent resistance, in-plane uniformity, hump suppression properties, and embedding characteristics.

### [Patterning test: Examples 3-1 to 3-80]

An organic cured film was formed on a SiO₂ wafer substrate using each of the compositions for forming an organic film (UDL-1 to 80) by the above-described method, the following silicon-containing resist middle layer film material (SOG1) was applied thereon and baked at 200°C for 60 seconds to form a silicon-containing resist middle layer film having a film thickness of 35 nm. The following single-layer resist for ArF was applied thereon as a resist upper layer film material and baked at 105°C for 60 seconds to form a photoresist film having a film thickness of 100 nm. The following immersion protective film material (TC-1) was applied on the photoresist film and baked at 90°C for 60 seconds to form a protective film having a film thickness of 50 nm.

The silicon-containing resist middle layer film material (SOG1) was prepared by dissolving a polymer (SP1), a crosslinking catalyst, and an acid in an organic solvent and water in fractions in Table 4 and filtering the solution with a 0.1 µm fluororesin filter.

**[Table 4]**

| | Polymer (Number of parts by mass) | Crosslinking catalyst (Number of parts by mass) | Acid (Number of parts by mass) | Organic solvent (Number of parts by mass) | Water (Number of parts by mass) |
|---|---|---|---|---|---|
| SOG1 | SP1 (100) | TMPANO₃ (1) | Maleic acid (1) | PGEE (4410) | Water (490) |

The polymer (SP1) is shown below. 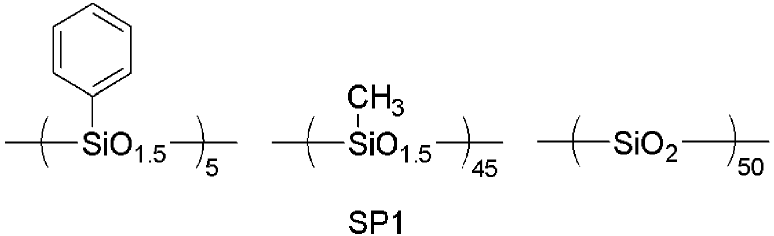
TMPANO₃: Trimethylphenylammonium nitrate
PGEE: Propylene glycol ethyl ether

The resist upper layer film material (single-layer resist for ArF) was prepared by dissolving a polymer (RP1), an acid generator (PAG-A), and a basic compound (Amine1) in a solvent containing 0.1 mass% of FC-430 (manufactured by Sumitomo 3M Limited) in fractions in Table 5 and filtering the solution with a 0.1 µm fluororesin filter.

**[Table 5]**

| | Polymer (Parts by mass) | Acid generator (Parts by mass) | Basic compound (Parts by mass) | Solvent (Parts by mass) |
|---|---|---|---|---|
| Single-layer resist for ArF | RP1 (100) | PAG-A (6.6) | Aminel (0.8) | PGMEA (2500) |

The polymer (RP1), the acid generator (PAG-A), and the basic compound (Amine1) are shown below.

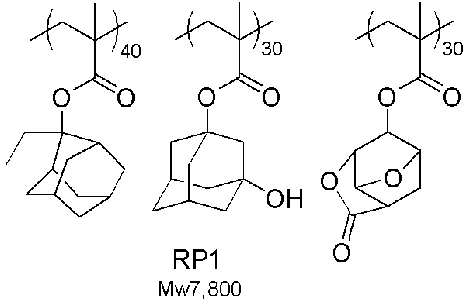

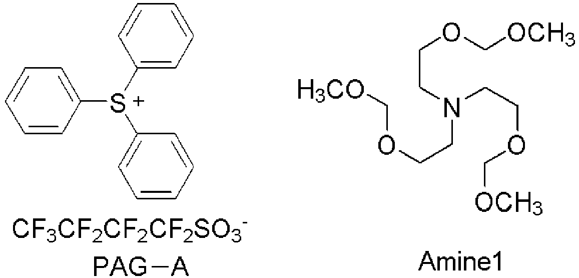

The immersion protective film material (TC-1) was prepared by dissolving a polymer (PP1) in an organic solvent in a fraction in Table 6 and filtering the solution with a 0.1 µm fluororesin filter.

**[Table 6]**

| | Polymer (Parts by mass) | Organic solvent (Parts by mass) |
|---|---|---|
| TC-1 | PP1 (100) | Diisoamyl ether (2700) |
| | | 2-Methyl-1-butanol (270) |

The polymer (PP1) is shown below.

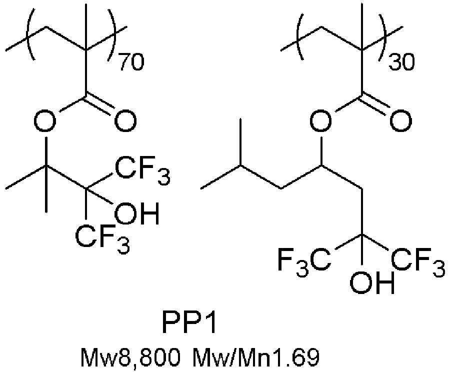

Next, the single-layer resist for ArF was exposed with an ArF immersion lithography device (Nikon Corporation; NSR-S610C, NA 1.30, σ 0.98/0.65, 35-degree dipole s polarized lighting, 6% halftone phase shift mask), baked (PEB) at 100°C for 60 seconds, and developed with a 2.38 mass% tetramethylammonium hydroxide (TMAH) aqueous solution for 30 seconds to obtain a 55 nm 1:1 positive type line and space pattern (resist upper layer film pattern).

Next, the silicon-containing resist middle layer film was dry-etched (pattern transfer) using an etching device Telius manufactured by Tokyo Electron Limited using the resist upper layer film pattern as a mask, the organic film was dry-etched (pattern transfer) using the obtained silicon-containing resist middle layer film pattern as a mask, and the SiO₂ wafer substrate (SiO₂ film) was dry-etched (pattern transfer) using the obtained organic film pattern as a mask. The etching conditions are as described below.

### (Transfer conditions of resist upper layer film pattern to silicon-containing resist middle layer film)

| | |
|---|---|
| Chamber pressure | 10.0 Pa |
| RF power | 1,500 W |
| CF₄ gas flow rate | 75 mL/min |
| O₂ gas flow rate | 15 mL/min |
| Time | 15 sec |

### (Transfer conditions of silicon-containing resist middle layer film pattern to organic film)

| | |
|---|---|
| Chamber pressure | 2.0 Pa |
| RF power | 500 W |
| Ar gas flow rate | 75 mL/min |
| O₂ gas flow rate | 45 mL/min |
| Time | 120 sec |

### (Transfer conditions of organic film pattern to SiO₂ wafer substrate)

| | |
|---|---|
| Chamber pressure | 2.0 Pa |
| RF power | 2,200 W |
| C₅F₁₂ gas flow rate | 20 mL/min |
| C₂F₆ gas flow rate | 10 mL/min |
| Ar gas flow rate | 300 mL/min |
| O₂ gas flow rate | 60 mL/min |
| Time | 90 sec |

The results of observing the cross sections of the obtained patterns with an electron microscope manufactured by Hitachi, Ltd. (S-4700) are shown in Tables 7-1 and 7-2.

**[Table 7-1]**

| | Composition for forming organic film | Shape after substrate transfer etching |
|---|---|---|
| Example 3-1 | UDL-1 | Favorable |
| Example 3-2 | UDL-2 | Favorable |
| Example 3-3 | UDL-3 | Favorable |
| Example 3-4 | UDL-4 | Favorable |
| Example 3-5 | UDL-5 | Favorable |
| Example 3-6 | UDL-6 | Favorable |
| Example 3-7 | UDL-7 | Favorable |
| Example 3-8 | UDL-8 | Favorable |
| Example 3-9 | UDL-9 | Favorable |
| Example 3-10 | UDL-10 | Favorable |
| Example 3-11 | UDL-11 | Favorable |
| Example 3-12 | UDL-12 | Favorable |
| Example 3-13 | UDL-13 | Favorable |
| Example 3-14 | UDL-14 | Favorable |
| Example 3-15 | UDL-15 | Favorable |
| Example 3-16 | UDL-16 | Favorable |
| Example 3-17 | UDL-17 | Favorable |
| Example 3-18 | UDL-18 | Favorable |
| Example 3-19 | UDL-19 | Favorable |
| Example 3-20 | UDL-20 | Favorable |
| Example 3-21 | UDL-21 | Favorable |
| Example 3-22 | UDL-22 | Favorable |
| Example 3-23 | UDL-23 | Favorable |
| Example 3-24 | UDL-24 | Favorable |
| Example 3-25 | UDL-25 | Favorable |
| Example 3-26 | UDL-26 | Favorable |
| Example 3-27 | UDL-27 | Favorable |
| Example 3-28 | UDL-28 | Favorable |
| Example 3-29 | UDL-29 | Favorable |
| Example 3-30 | UDL-30 | Favorable |
| Example 3-31 | UDL-31 | Favorable |
| Example 3-32 | UDL-32 | Favorable |
| Example 3-33 | UDL-33 | Favorable |
| Example 3-34 | UDL-34 | Favorable |
| Example 3-35 | UDL-35 | Favorable |
| Example 3-36 | UDL-36 | Favorable |
| Example 3-37 | UDL-37 | Favorable |
| Example 3-38 | UDL-38 | Favorable |
| Example 3-39 | UDL-39 | Favorable |
| Example 3-40 | UDL-40 | Favorable |

**[Table 7-2]**

| | Composition for forming organic film | Shape after substrate transfer etching |
|---|---|---|
| Example 3-41 | UDL-41 | Favorable |
| Example 3-42 | UDL-42 | Favorable |
| Example 3-43 | UDL-43 | Favorable |
| Example 3-44 | UDL-44 | Favorable |
| Example 3-45 | UDL-45 | Favorable |
| Example 3-46 | UDL-46 | Favorable |
| Example 3-47 | UDL-47 | Favorable |
| Example 3-48 | UDL-48 | Favorable |
| Example 3-49 | UDL-49 | Favorable |
| Example 3-50 | UDL-50 | Favorable |
| Example 3-51 | UDL-51 | Favorable |
| Example 3-52 | UDL-52 | Favorable |
| Example 3-53 | UDL-53 | Favorable |
| Example 3-54 | UDL-54 | Favorable |
| Example 3-55 | UDL-55 | Favorable |
| Example 3-56 | UDL-56 | Favorable |
| Example 3-57 | UDL-57 | Favorable |
| Example 3-58 | UDL-58 | Favorable |
| Example 3-59 | UDL-59 | Favorable |
| Example 3-60 | UDL-60 | Favorable |
| Example 3-61 | UDL-61 | Favorable |
| Example 3-62 | UDL-62 | Favorable |
| Example 3-63 | UDL-63 | Favorable |
| Example 3-64 | UDL-64 | Favorable |
| Example 3-65 | UDL-65 | Favorable |
| Example 3-66 | UDL-66 | Favorable |
| Example 3-67 | UDL-67 | Favorable |
| Example 3-68 | UDL-68 | Favorable |
| Example 3-69 | UDL-69 | Favorable |
| Example 3-70 | UDL-70 | Favorable |
| Example 3-71 | UDL-71 | Favorable |
| Example 3-72 | UDL-72 | Favorable |
| Example 3-73 | UDL-73 | Favorable |
| Example 3-74 | UDL-74 | Favorable |
| Example 3-75 | UDL-75 | Favorable |
| Example 3-76 | UDL-76 | Favorable |
| Example 3-77 | UDL-77 | Favorable |
| Example 3-78 | UDL-78 | Favorable |
| Example 3-79 | UDL-79 | Favorable |
| Example 3-80 | UDL-80 | Favorable |

As shown in Tables 7-1 to 7-2, in all of Examples 3-1 to 3-80 where the compositions for forming an organic film of the present invention (UDL-1 to 80) were used, the resist upper layer film pattern was favorably transferred up to the SiO₂ wafer substrate in the end, and it was confirmed that the composition for forming an organic film of the present invention can be suitably used in microfabrication by a multilayer resist method.

### [Preparation of resist upper layer film materials (PR1 to 41 and comparative PR)]

As resist upper layer film materials, resist upper layer film materials (PR1 to 41 and comparative PR) were each prepared by dissolving a polymer (P-1), an acid generator (PAG-1), a quencher (Q-1), and a compound (A1 to A41) in a solvent in fractions shown in Tables 8-1 and 8-2, and filtering the solution with a 0.1 µm fluororesin filter.

**[Table 8-1]**

| Resist upper layer film material | Polymer (Number of parts by mass) | Additive (Number of parts by mass) | Compound (Number of parts by mass) | Solvent (Number of parts by mass) |
|---|---|---|---|---|
| PR1 | P-1 (100) | PAG-1(25.0) | (A1) | PGMEA(1800) |
| | | Q-1(6.6) | (0.1) | EL(1200) |
| PR2 | P-1 (100) | PAG-1(25.0) | (A2) | PGMEA(1800) |
| | | Q-1(6.6) | (0.1) | EL(1200) |
| PR3 | P-1 (100) | PAG-1(25.0) | (A3) | PGMEA(1800) |
| | | Q-1(6.6) | (0.1) | EL(1200) |
| PR4 | P-1 (100) | PAG-1(25.0) | (A4) | PGMEA(1800) |
| | | Q-1(6.6) | (0.1) | EL(1200) |
| PR5 | P-1 (100) | PAG-1(25.0) | (A5) | PGMEA(1800) |
| | | Q-1(6.6) | (0.1) | EL(1200) |
| PR6 | P-1 (100) | PAG-1(25.0) | (A6) | PGMEA(1800) |
| | | Q-1(6.6) | (0.1) | EL(1200) |
| PR7 | P-1 (100) | PAG-1(25.0) | (A7) | PGMEA(1800) |
| | | Q-1(6.6) | (0.1) | EL(1200) |
| PR8 | P-1 (100) | PAG-1(25.0) | (A8) | PGMEA(1800) |
| | | Q-1(6.6) | (0.1) | EL(1200) |
| PR9 | P-1 (100) | PAG-1(25.0) | (A9) | PGMEA(1800) |
| | | Q-1(6.6) | (0.1) | EL(1200) |
| PR10 | P-1 (100) | PAG-1(25.0) | (A10) | PGMEA(1800) |
| | | Q-1(6.6) | (0.1) | EL(1200) |
| PR11 | P-1 (100) | PAG-1(25.0) | (A11) | PGMEA(1800) |
| | | Q-1(6.6) | (0.1) | EL(1200) |
| PR12 | P-1 (100) | PAG-1(25.0) | (A12) | PGMEA(1800) |
| | | Q-1(6.6) | (0.1) | EL(1200) |
| PR13 | P-1 (100) | PAG-1(25.0) | (A13) | PGMEA(1800) |
| | | Q-1(6.6) | (0.1) | EL(1200) |
| PR14 | P-1 (100) | PAG-1(25.0) | (A14) | PGMEA(1800) |
| | | Q-1(6.6) | (0.1) | EL(1200) |
| PR15 | P-1 (100) | PAG-1(25.0) | (A15) | PGMEA(1800) |
| | | Q-1(6.6) | (0.1) | EL(1200) |
| PR16 | P-1 (100) | PAG-1(25.0) | (A16) | PGMEA(1800) |
| | | Q-1(6.6) | (0.1) | EL(1200) |
| PR17 | P-1 (100) | PAG-1(25.0) | (A17) | PGMEA(1800) |
| | | Q-1(6.6) | (0.1) | EL(1200) |
| PR18 | P-1 (100) | PAG-1(25.0) | (A18) | PGMEA(1800) |
| | | Q-1(6.6) | (0.1) | EL(1200) |
| PR19 | P-1 (100) | PAG-1(25.0) | (A19) | PGMEA(1800) |
| | | Q-1(6.6) | (0.1) | EL(1200) |
| PR20 | P-1 (100) | PAG-1(25.0) | (A20) | PGMEA(1800) |
| | | Q-1(6.6) | (0.1) | EL(1200) |

**[Table 8-2]**

| Resist upper layer film material | Polymer (Number of parts by mass) | Additive (Number of parts by mass) | Compound (Number of parts by mass) | Solvent (Number of parts by mass) |
|---|---|---|---|---|
| PR21 | P-1 (100) | PAG-1(25.0) | (A21) | PGMEA(1800) |
| | | Q-1(6.6) | (0.1) | EL(1200) |
| PR22 | P-1 (100) | PAG-1(25.0) | (A22) | PGMEA(1800) |
| | | Q-1(6.6) | (0.1) | EL(1200) |
| PR23 | P-1 (100) | PAG-1(25.0) | (A23) | PGMEA(1800) |
| | | Q-1(6.6) | (0.1) | EL(1200) |
| PR24 | P-1 (100) | PAG-1(25.0) | (A24) | PGMEA(1800) |
| | | Q-1(6.6) | (0.1) | EL(1200) |
| PR25 | P-1 (100) | PAG-1(25.0) | (A25) | PGMEA(1800) |
| | | Q-1(6.6) | (0.1) | EL(1200) |
| PR26 | P-1 (100) | PAG-1(25.0) | (A26) | PGMEA(1800) |
| | | Q-1(6.6) | (0.1) | EL(1200) |
| PR27 | P-1 (100) | PAG-1(25.0) | (A27) | PGMEA(1800) |
| | | Q-1(6.6) | (0.1) | EL(1200) |
| PR28 | P-1 (100) | PAG-1(25.0) | (A28) | PGMEA(1800) |
| | | Q-1(6.6) | (0.1) | EL(1200) |
| PR29 | P-1 (100) | PAG-1(25.0) | (A29) | PGMEA(1800) |
| | | Q-1(6.6) | (0.1) | EL(1200) |
| PR30 | P-1 (100) | PAG-1(25.0) | (A30) | PGMEA(1800) |
| | | Q-1(6.6) | (0.1) | EL(1200) |
| PR31 | P-1 (100) | PAG-1(25.0) | (A31) | PGMEA(1800) |
| | | Q-1(6.6) | (0.1) | EL(1200) |
| PR32 | P-1 (100) | PAG-1(25.0) | (A32) | PGMEA(1800) |
| | | Q-1(6.6) | (0.1) | EL(1200) |
| PR33 | P-1 (100) | PAG-1(25.0) | (A33) | PGMEA(1800) |
| | | Q-1(6.6) | (0.1) | EL(1200) |
| PR34 | P-1 (100) | PAG-1(25.0) | (A34) | PGMEA(1800) |
| | | Q-1(6.6) | (0.1) | EL(1200) |
| PR35 | P-1 (100) | PAG-1(25.0) | (A35) | PGMEA(1800) |
| | | Q-1(6.6) | (0.1) | EL(1200) |
| PR36 | P-1 (100) | PAG-1(25.0) | (A36) | PGMEA(1800) |
| | | Q-1(6.6) | (0.1) | EL(1200) |
| PR37 | P-1 (100) | PAG-1(25.0) | (A37) | PGMEA(1800) |
| | | Q-1(6.6) | (0.1) | EL(1200) |
| PR38 | P-1 (100) | PAG-1(25.0) | (A38) | PGMEA(1800) |
| | | Q-1(6.6) | (0.1) | EL(1200) |
| PR39 | P-1 (100) | PAG-1(25.0) | (A39) | PGMEA(1800) |
| | | Q-1(6.6) | (0.1) | EL(1200) |
| PR40 | P-1 (100) | PAG-1(25.0) | (A40) | PGMEA(1800) |
| | | Q-1(6.6) | (0.1) | EL(1200) |
| PR41 | P-1 (100) | PAG-1(25.0) | (A41) | PGMEA(1800) |
| | | Q-1(6.6) | (0.1) | EL(1200) |
| Comparative PR | P-1 (100) | PAG-1(25.0) | - | PGMEA(1800) |
| | | Q-1(6.6) | | EL(1200) |

The polymer (P-1), the acid generator (PAG-1), the quencher (Q-1), and the solvent are shown below. 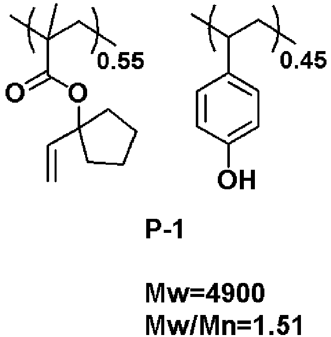 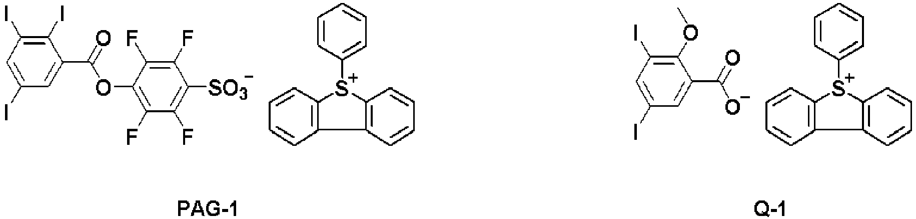
PGMEA: Propylene glycol monomethyl ether acetate
EL: Ethyl lactate

### [Fabrication of silicon wafers on which resist upper layer film is formed using resist upper layer film material (PR1 to 41 and comparative PR)]

2 ml of each of the resist upper layer film materials prepared above (PR1 to 41 and comparative PR) was ejected to the center of the silicon wafer using the coater/developer "CLEAN TRACK LITHIOUS Pro AP" from Tokyo Electron Limited, then, rotated at a rotation speed at which the average film thickness reached 45 nm after baking, and spread. Next, the silicon wafer coated with the resist upper layer film was heated at 110 degrees for 30 seconds, thereby obtaining a silicon wafer having a resist upper layer film thereon.

### [In-plane uniformity evaluation of resist upper layer films: Examples 4-1 to 4-41 and Comparative Example 4-1]

The film thickness of each of the resist upper layer films (PR1 to 41 and comparative PR) formed on the silicon wafer by the above-described method was measured with an optical film thickness meter at 225 points on concentric circles within a range of a radius of 145 mm from the wafer center. The maximum value of the film thickness was represented by Xₘₐₓ, the minimum value was represented by Xₘᵢₙ, the average value was represented by X_{average}, and a value obtained by (Xₘₐₓ - Xₘᵢₙ)/X_{average} was regarded as in-plane uniformity (%). In a case where the value was less than 3%, the in-plane uniformity was evaluated as favorable, and in the case of 3% or more, the in-plane uniformity was evaluated as poor. The results are shown in Table 9.

**[Table 9]**

| | Resist upper layer film material | In-plane uniformity |
|---|---|---|
| Example 4-1 | PR1 | Favorable |
| Example 4-2 | PR2 | Favorable |
| Example 4-3 | PR3 | Favorable |
| Example 4-4 | PR4 | Favorable |
| Example 4-5 | PR5 | Favorable |
| Example 4-6 | PR6 | Favorable |
| Example 4-7 | PR7 | Favorable |
| Example 4-8 | PR8 | Favorable |
| Example 4-9 | PR9 | Favorable |
| Example 4-10 | PR10 | Favorable |
| Example 4-11 | PR11 | Favorable |
| Example 4-12 | PR12 | Favorable |
| Example 4-13 | PR13 | Favorable |
| Example 4-14 | PR14 | Favorable |
| Example 4-15 | PR15 | Favorable |
| Example 4-16 | PR16 | Favorable |
| Example 4-17 | PR17 | Favorable |
| Example 4-18 | PR18 | Favorable |
| Example 4-19 | PR19 | Favorable |
| Example 4-20 | PR20 | Favorable |
| Example 4-21 | PR21 | Favorable |
| Example 4-22 | PR22 | Favorable |
| Example 4-23 | PR23 | Favorable |
| Example 4-24 | PR24 | Favorable |
| Example 4-25 | PR25 | Favorable |
| Example 4-26 | PR26 | Favorable |
| Example 4-27 | PR27 | Favorable |
| Example 4-28 | PR28 | Favorable |
| Example 4-29 | PR29 | Favorable |
| Example 4-30 | PR30 | Favorable |
| Example 4-31 | PR31 | Favorable |
| Example 4-32 | PR32 | Favorable |
| Example 4-33 | PR33 | Favorable |
| Example 4-34 | PR34 | Favorable |
| Example 4-35 | PR35 | Favorable |
| Example 4-36 | PR36 | Favorable |
| Example 4-37 | PR37 | Favorable |
| Example 4-38 | PR38 | Favorable |
| Example 4-39 | PR39 | Favorable |
| Example 4-40 | PR40 | Favorable |
| Example 4-41 | PR41 | Favorable |
| Comparative Example 4-1 | Comparative PR | Poor |

As shown in Table 9, since the resist upper layer film materials (PR1 to 41) of the present invention have excellent in-plane uniformity, it is found that the compound of the present invention functions as a surfactant that imparts excellent leveling performance and can be used in a variety of compositions for forming an organic film regardless of the type of resin to be combined.

### [Preparation of silicon-containing resist middle layer film materials (SOG2 to 42 and comparative SOG1)]

As silicon-containing resist middle layer film materials, silicon-containing resist middle layer film materials (SOG2 to 42 and comparative SOG1) were each prepared by dissolving the polymer (SP1), a crosslinking catalyst, and an acid in an organic solvent and water in fractions shown in Tables 10-1 and 10-2, and filtering the solution with a 0.1 µm fluororesin filter.

**[Table 10-1]**

| Material for forming silicon-containing resist middle layer film | Polymer (Number of parts by mass) | Crosslinking catalyst (Number of parts by mass) | Acid (Number of parts by mass) | Compound (Number of parts by mass) | Organic solvent (Number of parts by mass) | Water (Number of parts by mass) |
|---|---|---|---|---|---|---|
| SOG2 | SP1 (100) | TMPANO3 (1.0) | Maleic acid (1.0) | (A1) | PGEE (44100) | Water (4900) |
| | | | | (1.0) | | |
| SOG3 | SP1 (100) | TMPANO3 (1.0) | Maleic acid (1.0) | (A2) | PGEE (44100) | Water (4900) |
| | | | | (1.0) | | |
| SOG4 | SP1 (100) | TMPANO3 (1.0) | Maleic acid (1.0) | (A3) | PGEE (44100) | Water (4900) |
| | | | | (1.0) | | |
| SOG5 | SP1 (100) | TMPANO3 (1.0) | Maleic acid (1.0) | (A4) | PGEE (44100) | Water (4900) |
| | | | | (1.0) | | |
| SOG6 | SP1 (100) | TMPANO3 (1.0) | Maleic acid (1.0) | (A5) | PGEE (44100) | Water (4900) |
| | | | | (1.0) | | |
| SOG7 | SP1 (100) | TMPANO3 (1.0) | Maleic acid (1.0) | (A6) | PGEE (44100) | Water (4900) |
| | | | | (1.0) | | |
| SOG8 | SP1 (100) | TMPANO3 (1.0) | Maleic acid (1.0) | (A7) | PGEE (44100) | Water (4900) |
| | | | | (1.0) | | |
| SOG9 | SP1 (100) | TMPANO3 (1.0) | Maleic acid (1.0) | (A8) | PGEE (44100) | Water (4900) |
| | | | | (1.0) | | |
| SOG10 | SP1 (100) | TMPANO3 (1.0) | Maleic acid (1.0) | (A9) | PGEE (44100) | Water (4900) |
| | | | | (1.0) | | |
| SOG11 | SP1 (100) | TMPANO3 (1.0) | Maleic acid (1.0) | (A10) | PGEE (44100) | Water (4900) |
| | | | | (1.0) | | |
| SOG12 | SP1 (100) | TMPANO3 (1.0) | Maleic acid (1.0) | (A11) | PGEE (44100) | Water (4900) |
| | | | | (1.0) | | |
| SOG13 | SP1 (100) | TMPANO3 (1.0) | Maleic acid (1.0) | (A12) | PGEE (44100) | Water (4900) |
| | | | | (1.0) | | |
| SOG14 | SP1 (100) | TMPANO3 (1.0) | Maleic acid (1.0) | (A13) | PGEE (44100) | Water (4900) |
| | | | | (1.0) | | |
| SOG15 | SP1 (100) | TMPANO3 (1.0) | Maleic acid (1.0) | (A14) | PGEE (44100) | Water (4900) |
| | | | | (1.0) | | |
| SOG16 | SP1 (100) | TMPANO3 (1.0) | Maleic acid (1.0) | (A15) | PGEE (44100) | Water (4900) |
| | | | | (1.0) | | |
| SOG17 | SP1 (100) | TMPANO3 (1.0) | Maleic acid (1.0) | (A16) | PGEE (44100) | Water (4900) |
| | | | | (1.0) | | |
| SOG18 | SP1 (100) | TMPANO3 (1.0) | Maleic acid (1.0) | (A17) | PGEE (44100) | Water (4900) |
| | | | | (1.0) | | |
| SOG19 | SP1 (100) | TMPANO3 (1.0) | Maleic acid (1.0) | (A18) | PGEE (44100) | Water (4900) |
| | | | | (1.0) | | |
| SOG20 | SP1 (100) | TMPANO3 (1.0) | Maleic acid (1.0) | (A19) | PGEE (44100) | Water (4900) |
| | | | | (1.0) | | |
| SOG21 | SP1 (100) | TMPANO3 (1.0) | Maleic acid (1.0) | (A20) | PGEE (44100) | Water (4900) |
| | | | | (1.0) | | |
| SOG22 | SP1 (100) | TMPANO3 (1.0) | Maleic acid (1.0) | (A21) | PGEE (44100) | Water (4900) |
| | | | | (1.0) | | |
| SOG23 | SP1 (100) | TMPANO3 (1.0) | Maleic acid (1.0) | (A22) | PGEE (44100) | Water (4900) |
| | | | | (1.0) | | |
| SOG24 | SP1 (100) | TMPANO3 (1.0) | Maleic acid (1.0) | (A23) | PGEE (44100) | Water (4900) |
| | | | | (1.0) | | |
| SOG25 | SP1 (100) | TMPANO3 (1.0) | Maleic acid (1.0) | (A24) | PGEE | Water (4900) |
| | | | | (1.0) | (44100) | |
| SOG26 | SP1 (100) | TMPANO3 (1.0) | Maleic acid (1.0) | (A25) | PGEE (44100) | Water (4900) |
| | | | | (1.0) | | |

**[Table 10-2]**

| Material for forming silicon-containing resist middle layer film | Polymer (Number of parts by mass) | Crosslinking catalyst (Number of parts by mass) | Acid (Number of parts by mass) | Compound (Number of parts by mass) | Organic solvent (Number of parts by mass) | Water (Number of parts by mass) |
|---|---|---|---|---|---|---|
| SOG27 | SP1 (100) | TMPANO3 (1.0) | Maleic acid (1.0) | (A26) | PGEE (44100) | Water (4900) |
| | | | | (1.0) | | |
| SOG28 | SP1 (100) | TMPANO3 (1.0) | Maleic acid (1.0) | (A27) | PGEE (44100) | Water (4900) |
| | | | | (1.0) | | |
| SOG29 | SP1 (100) | TMPANO3 (1.0) | Maleic acid (1.0) | (A28) | PGEE (44100) | Water (4900) |
| | | | | (1.0) | | |
| SOG30 | SP1 (100) | TMPANO3 (1.0) | Maleic acid (1.0) | (A29) | PGEE (44100) | Water (4900) |
| | | | | (1.0) | | |
| SOG31 | SP1 (100) | TMPANO3 (1.0) | Maleic acid (1.0) | (A30) | PGEE (44100) | Water (4900) |
| | | | | (1.0) | | |
| SOG32 | SP1 (100) | TMPANO3 (1.0) | Maleic acid (1.0) | (A31) | PGEE (44100) | Water (4900) |
| | | | | (1.0) | | |
| SOG33 | SP1 (100) | TMPANO3 (1.0) | Maleic acid (1.0) | (A32) | PGEE (44100) | Water (4900) |
| | | | | (1.0) | | |
| SOG34 | SP1 (100) | TMPANO3 (1.0) | Maleic acid (1.0) | (A33) | PGEE (44100) | Water (4900) |
| | | | | (1.0) | | |
| SOG35 | SP1 (100) | TMPANO3 (1.0) | Maleic acid (1.0) | (A34) | PGEE (44100) | Water (4900) |
| | | | | (1.0) | | |
| SOG36 | SP1 (100) | TMPANO3 (1.0) | Maleic acid (1.0) | (A35) | PGEE (44100) | Water (4900) |
| | | | | (1.0) | | |
| SOG37 | SP1 (100) | TMPANO3 (1.0) | Maleic acid (1.0) | (A36) | PGEE (44100) | Water (4900) |
| | | | | (1.0) | | |
| SOG38 | SP1 (100) | TMPANO3 (1.0) | Maleic acid (1.0) | (A37) | PGEE (44100) | Water (4900) |
| | | | | (1.0) | | |
| SOG39 | SP1 (100) | TMPANO3 (1.0) | Maleic acid (1.0) | (A38) | PGEE (44100) | Water (4900) |
| | | | | (1.0) | | |
| SOG40 | SP1 (100) | TMPANO3 (1.0) | Maleic acid (1.0) | (A39) | PGEE (44100) | Water (4900) |
| | | | | (1.0) | | |
| SOG41 | SP1 (100) | TMPANO3 (1.0) | Maleic acid (1.0) | (A40) | PGEE (44100) | Water (4900) |
| | | | | (1.0) | | |
| SOG42 | SP1 (100) | TMPANO3 (1.0) | Maleic acid (1.0) | (A41) | PGEE (44100) | Water (4900) |
| | | | | (1.0) | | |
| Comparative SOG1 | SP1 (100) | TMPANO3 (1.0) | Maleic acid (1.0) | - | PGEE (44100) | Water (4900) |

| | | | | | | |
|---|---|---|---|---|---|---|
| PGEE: Propylene glycol monoethyl ether | | | | | | |

### [Silicon-containing resist middle layer film coatability evaluation: Examples 5-1 to 5-41 and Comparative Example 5-1]

An organic cured film was formed on a silicon wafer substrate using the composition for forming an organic film (comparative UDL-1) by the above-described method, and each of the following silicon-containing resist middle layer film materials (SOG2 to 42 and comparative SOG1) was applied thereon and baked at 200°C for 60 seconds to form a silicon-containing resist middle layer film. In addition, the state of the coating film of the silicon-containing resist middle layer film was visually observed and evaluated.

In a case where the state of the coating film was favorable, the coatability was evaluated as favorable, and in a case where a pinhole was generated, the coatability was evaluated as poor.

In the present evaluation, in order to evaluate the superiority or inferiority of the coatability of the silicon-containing resist middle layer film, the evaluation conditions were so particularly strict that the film thickness of the silicon-containing resist middle layer film was 5 nm.

**[Table 11]**

| | Material for forming silicon-containing resist middle layer film | Evaluation of coatability of silicon-containing resist middle layer film |
|---|---|---|
| Example 5-1 | SOG2 | Favorable |
| Example 5-2 | SOG3 | Favorable |
| Example 5-3 | SOG4 | Favorable |
| Example 5-4 | SOG5 | Favorable |
| Example 5-5 | SOG6 | Favorable |
| Example 5-6 | SOG7 | Favorable |
| Example 5-7 | SOG8 | Favorable |
| Example 5-8 | SOG9 | Favorable |
| Example 5-9 | SOG10 | Favorable |
| Example 5-10 | SOG11 | Favorable |
| Example 5-11 | SOG12 | Favorable |
| Example 5-12 | SOG13 | Favorable |
| Example 5-13 | SOG14 | Favorable |
| Example 5-14 | SOG15 | Favorable |
| Example 5-15 | SOG16 | Favorable |
| Example 5-16 | SOG17 | Favorable |
| Example 5-17 | SOG18 | Favorable |
| Example 5-18 | SOG19 | Favorable |
| Example 5-19 | SOG20 | Favorable |
| Example 5-20 | SOG21 | Favorable |
| Example 5-21 | SOG22 | Favorable |
| Example 5-22 | SOG23 | Favorable |
| Example 5-23 | SOG24 | Favorable |
| Example 5-24 | SOG25 | Favorable |
| Example 5-25 | SOG26 | Favorable |
| Example 5-26 | SOG27 | Favorable |
| Example 5-27 | SOG28 | Favorable |
| Example 5-28 | SOG29 | Favorable |
| Example 5-29 | SOG30 | Favorable |
| Example 5-30 | SOG31 | Favorable |
| Example 5-31 | SOG32 | Favorable |
| Example 5-32 | SOG33 | Favorable |
| Example 5-33 | SOG34 | Favorable |
| Example 5-34 | SOG35 | Favorable |
| Example 5-35 | SOG36 | Favorable |
| Example 5-36 | SOG37 | Favorable |
| Example 5-37 | SOG38 | Favorable |
| Example 5-38 | SOG39 | Favorable |
| Example 5-39 | SOG40 | Favorable |
| Example 5-40 | SOG41 | Favorable |
| Example 5-41 | SOG42 | Favorable |
| Comparative Example 5-1 | Comparative SOG1 | Poor |

As shown in Table 11, the silicon-containing resist middle layer film materials (SOG2 to 42) do not generate pinholes and have excellent film formability. From this fact, it is found that the compound of the present invention can be applied and used as a surfactant that develops high film formability not only in organic films but also in a variety of compositions for forming a film.

What has been described above shows that the composition for forming an organic film of the present invention has excellent film formability, high-level embedding characteristics, and hump suppression properties, and is thus extremely useful as an organic film material for use in a multilayer resist process, in addition, the patterning process of the present invention using this composition is capable of embedding a hole or trench having an extremely high aspect ratio with no voids, is also capable of highly accurately forming a fine pattern, is capable of forming an organic film in which a hump is suppressed, and is thus capable of efficiently manufacturing a semiconductor element and the like.

In addition, the compound of the present invention develops high film formability or the like and can be thus widely used as a surfactant.

The present description includes the following inventions.
[1]: A composition for forming an organic film comprising: (A) a resin or compound for forming an organic film; (B) a compound represented by the following general formula (1); and (C) a solvent: 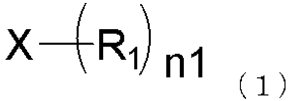
   wherein R₁ is a terminal group represented by the following general formula (2), X is an n1-valent organic group having 2 to 50 carbon atoms, and n1 represents an integer of 2 to 8: 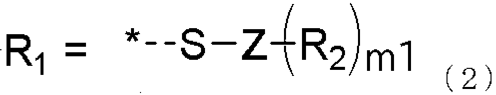
   wherein R₂ is a monovalent organic group, wherein the monovalent organic group contains at least any of structures having fluorine atoms represented by the following general formula (3), m1 is 1 or 2, wherein when m1 is 1, Z represents a single bond or a divalent organic group optionally containing a heteroatom, and when m1 is 2, Z represents a trivalent organic group optionally containing a heteroatom, and * represents an attachment point, 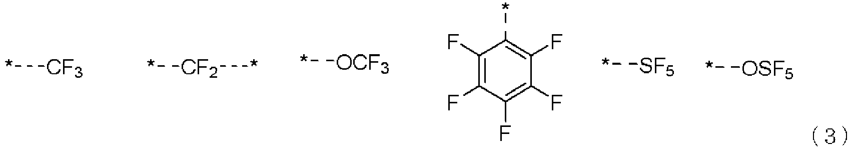
   wherein * represents an attachment point.
[2]: The composition for forming an organic film according to [1], wherein the terminal group represented by the general formula (2) in the (B) compound is any one or more of the following general formulae (4) to (8): 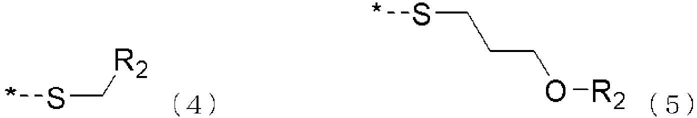 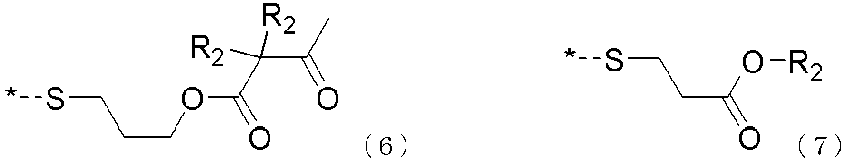 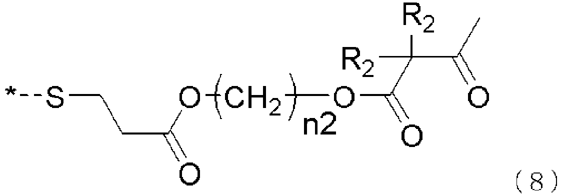 wherein R₂ is as defined above, and n2 represents an integer of 2 to 6.
[3]: The composition for forming an organic film according to [1] or [2], wherein R₂ in the (B) compound is any of groups represented by the following general formula (9): 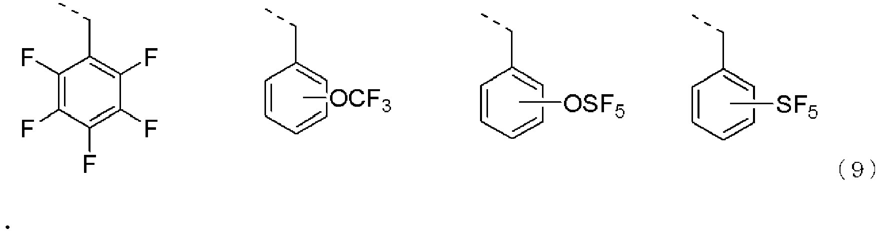
[4]: The composition for forming an organic film according to any one of [1] to [3], wherein the (B) compound has a weight-average molecular weight of 300 to 4000.
[5]: The composition for forming an organic film according to any one of [1] to [4], wherein a ratio Mw/Mn of a weight-average molecular weight Mw to a number-average molecular weight Mn of the (B) compound in terms of polystyrene by gel permeation chromatography is 1.00 ≤ Mw/Mn ≤ 1.20.
[6]: The composition for forming an organic film according to any one of [1] to [5], wherein a content of the (B) compound is 0.01 parts by mass to 5 parts by mass based on 100 parts by mass of the (A) resin or compound for forming an organic film.
[7]: A method for forming an organic film for use in a process of manufacturing a semiconductor device, the method comprising: performing spin coating of the composition for forming an organic film according to any one of [1] to [6] on a substrate to be processed; and performing a heat treatment on the substrate coated with the composition for forming an organic film at a temperature of 100°C or higher and 600°C or lower for a range of 10 to 600 seconds, thereby forming a cured film.
[8]: A patterning process comprising: forming an organic film on a body to be processed using the composition for forming an organic film according to any one of [1] to [6]; forming a resist middle layer film on the organic film using a resist middle layer film material containing a silicon atom; forming a resist upper layer film on the resist middle layer film using a resist upper layer film material composed of a photoresist composition; forming a circuit pattern in the resist upper layer film; transferring the pattern to the resist middle layer film by etching using the resist upper layer film in which the circuit pattern has been formed as a mask; transferring the pattern to the organic film by etching using the resist middle layer film to which the pattern has been transferred as a mask; and further transferring the pattern to the body to be processed by etching using the organic film to which the pattern has been transferred as a mask.
[9]: A patterning process comprising: forming an organic film on a body to be processed using the composition for forming an organic film according to any one of [1] to [6]; forming a resist middle layer film on the organic film using a resist middle layer film material containing a silicon atom; forming an organic antireflective film or an adhesive film on the resist middle layer film, forming a resist upper layer film on the organic antireflective film or adhesive film using a resist upper layer film material composed of a photoresist composition; forming a circuit pattern in the resist upper layer film; transferring the pattern to the organic antireflective film or adhesive film and the resist intermediate film by etching using the resist upper layer film in which the circuit pattern has been formed as a mask; transferring the pattern to the organic film by etching using the resist middle layer film to which the pattern has been transferred as a mask; and further transferring the pattern to the body to be processed by etching using the organic film to which the pattern has been transferred as a mask.
[10]: A patterning process comprising: forming an organic film on a body to be processed using the composition for forming an organic film according to any one of [1] to [6]; forming an inorganic hard mask selected from a silicon oxide film, a silicon nitride film, and a silicon oxynitride film on the organic film; forming a resist upper layer film on the inorganic hard mask using a resist upper layer film material composed of a photoresist composition; forming a circuit pattern in the resist upper layer film; transferring the pattern to the inorganic hard mask by etching using the resist upper layer film in which the circuit pattern has been formed as a mask; transferring the pattern to the organic film by etching using the inorganic hard mask to which the pattern has been transferred as a mask; and further transferring the pattern to the body to be processed by etching using the organic film to which the pattern has been transferred as a mask.
[11]: A patterning process comprising: forming an organic film on a body to be processed using the composition for forming an organic film according to any one of [1] to [6]; forming an inorganic hard mask selected from a silicon oxide film, a silicon nitride film, and a silicon oxynitride film on the organic film; forming an organic antireflective film or an adhesive film on the inorganic hard mask; forming a resist upper layer film on the organic antireflective film or adhesive film using a resist upper layer film material composed of a photoresist composition; forming a circuit pattern in the resist upper layer film; transferring the pattern to the organic antireflective film or adhesive film and the inorganic hard mask by etching using the resist upper layer film in which the circuit pattern has been formed as a mask; transferring the pattern to the organic film by etching using the inorganic hard mask to which the pattern has been transferred as a mask; and further transferring the pattern to the body to be processed by etching using the organic film to which the pattern has been transferred as a mask.
[12]: The patterning process according to [10] or [11], wherein the inorganic hard mask is formed by a CVD method or an ALD method.
[13]: The patterning process according to any one of [8] to [12], wherein in formation of the circuit pattern, the circuit pattern is formed by lithography using light having a wavelength of 10 nm or longer and 300 nm or shorter, direct writing using an electron beam, nanoimprinting, or a combination thereof.
[14]: The patterning process according to any one of [8] to [13], wherein in formation of the circuit pattern, the circuit pattern is developed by alkali development or with an organic solvent.
[15]: The patterning process according to any one of [8] to [14], wherein the body to be processed is a semiconductor device substrate or the semiconductor device substrate having any one of a metal film, a metal carbide film, a metal oxide film, a metal nitride film, a metal oxycarbide film, and a metal oxynitride film formed thereon.
[16]: The patterning process according to [15], wherein a metal that constitutes the body to be processed is silicon, titanium, tungsten, hafnium, zirconium, chromium, germanium, copper, silver, gold, aluminum, indium, gallium, arsenic, palladium, iron, tantalum, iridium, molybdenum, or an alloy thereof.
[17]: A compound represented by the following general formula (1): 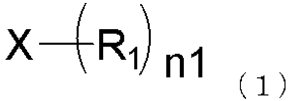
   wherein R₁ is a terminal group represented by the following general formula (2), X is an n1-valent organic group having 2 to 50 carbon atoms, and n1 represents an integer of 2 to 8: 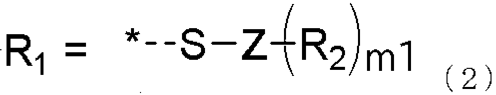
   wherein R₂ is a monovalent organic group, wherein the monovalent organic group contains at least any of structures having fluorine atoms represented by the following general formula (3), m1 is 1 or 2, wherein when m1 is 1, Z represents a single bond or a divalent organic group optionally containing a heteroatom, and when m1 is 2, Z represents a trivalent organic group optionally containing a heteroatom, and * represents an attachment point, 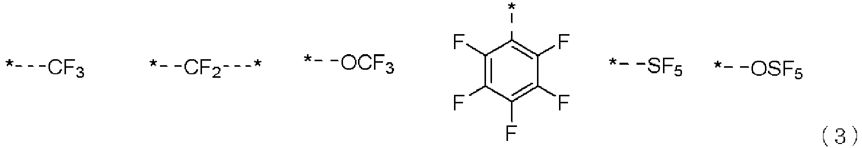
   wherein * represents an attachment point.
[18]: The compound according to [17], wherein the terminal group represented by the general formula (2) is any one or more of the following general formulae (4) to (8): 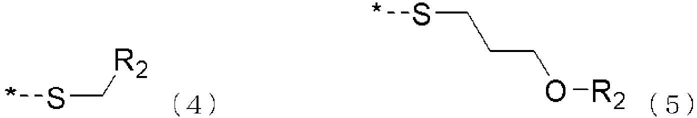 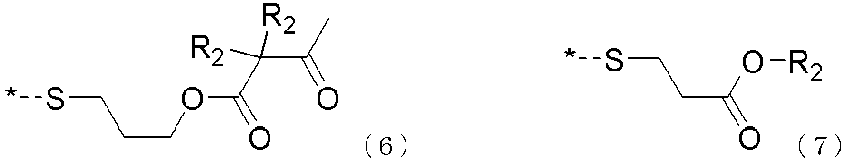 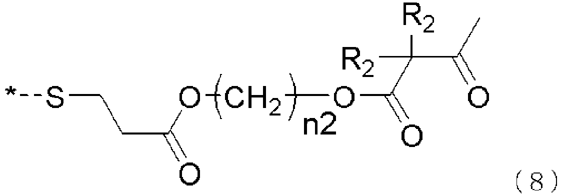 wherein R₂ is as defined above, and n2 represents an integer of 2 to 6.
[19]: The compound according to [17] or [18], wherein R₂ is any of groups represented by the following general formula (9):

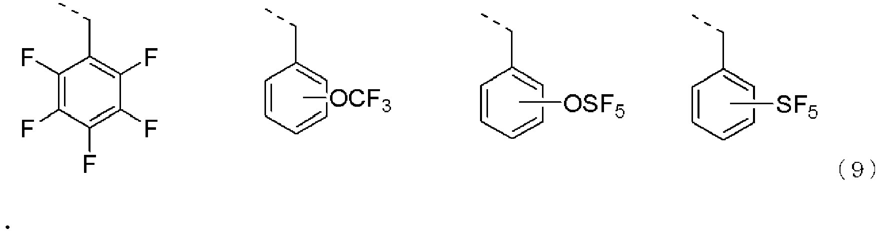

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A compound represented by the following general formula (1): 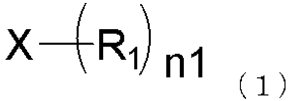
wherein R₁ is a terminal group represented by any one or more of the following general formulae (5) to (8), X is an n1-valent organic group having 2 to 50 carbon atoms, and n1 represents an integer of 2 to 8: 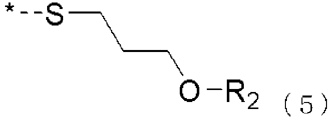 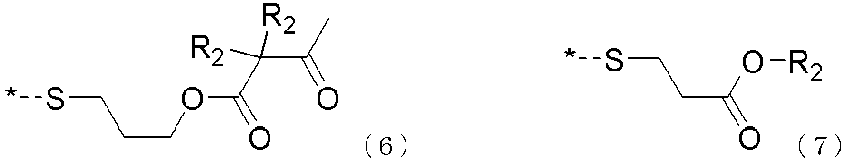 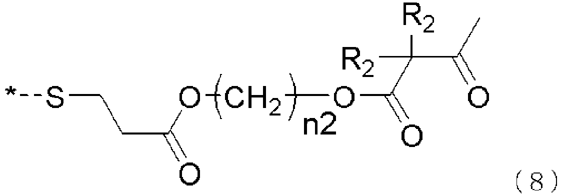
wherein R₂ is a monovalent organic group, wherein the monovalent organic group contains at least any of structures having fluorine atoms represented by the following general formula (3), n2 represents an integer of 2 to 6 and * represents an attachment point, 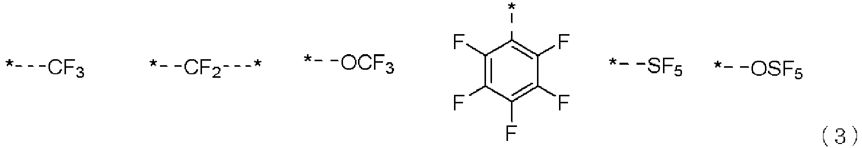
wherein * represents an attachment point.

2. The compound according to claim 1, wherein R₂ is any of groups represented by the following general formula (9):
